# EUROPEAN PATENT SPECIFICATION

(11) **EP 1 440 084 B1**
(45) Date of publication and mention of the grant of the patent: **06.01.2010**
(21) Application number: 02801948.7
(22) Date of filing: 18.10.2002
(51) Int. Cl.: C07K 14/00

(54) **SPERM FACTOR SEQUENCES**
SPERM FAKTOREN SEQUENZEN
SEQUENCE DE FACTEUR SPERMATIQUE

(30) Priority: 24.10.2001 GB 0125498; 28.06.2002 GB 0214945
(43) Date of publication of application: 28.07.2004
(73) Proprietor: University College Cardiff Consultants Ltd., Cardiff, Wales CF24 0DE (GB)
(72) Inventor: LAI, Tony, University of Wales College of Medicine, Cardiff CF14 4XN (GB)
(74) Representative: Fyles, Julie Marie
(86) International application number: PCT/GB2002/004739
(87) International publication number: WO 2003/035678

(56) References cited:
- WO-A-02/06302
- WO-A-96/25495
- SAUNDERS C.M. ET AL: "PLC zeta: a sperm specific trigger of Ca2+ oscillation in eggs and embryo development" DEVELOPMENT, vol. 129, 2002, pages 3533-3544, XP002238166
- WU H. ET AL.: "sperm factor induces intracellular free calcium oscillations by stimulating the phosphoinositide pathway" BIOLOGY OF REPRODUCTION, vol. 64, 2001, pages 1338-1349, XP002238167
- DATABASE EMBL [Online] retrieved from EMBL Database accession no. AY035866 XP002238168
- DATABASE EMBL [Online] retrieved from EMBL Database accession no. AK006672 XP002238169

## Description

This invention relates to the regulation and control of intracellular free calcium ion concentrations and more particularly to the control of cytoplasmic calcium oscillations (CCOs) in mammalian oocytes (eggs). In particular, it relates to phospho-inositide-specific phospholipase C proteins that trigger CCOs that are indistinguishable from those occurring at oocyte fertilization and nucleic acid sequences encoding such proteins, and the use thereof in biotechnology, diagnosis or medicine.

Transient changes in the intracellular calcium (Ca²⁺⁾ concentration are known to be responsible for activating numerous physiological processes, including memory formation, muscle contraction, hormone secretion, fertilization, gene transcription and apoptosis. One striking phenomenon observed in numerous cell types, including cardiac myocytes, endothelial cells and eggs, is the generation of a series of regular calcium transients or oscillations in response to cellular stimuli. The best-studied example of this phenomenon is during mammalian fertilization, where calcium levels in the egg begin to oscillate in a regular fashion following fusion with the sperm. These calcium oscillations occurring at fertilisation, sometimes referred to as the "calcium waves", are believed to be the trigger for egg activation and consequent embryo development. Studies carried out over many years have attempted to discover and isolate the causative agent of this phenomenon with a view to using it for research and for a variety of practical applications, including diagnosis.

This striking Ca²⁺ signalling phenomenon in fertilized mammalian eggs arises from increases in inositol 1,4,5-trisphosphate (IP₃) levels, which activates IP₃ receptor- mediated Ca²⁺ release from intracellular stores in the egg. However, the basic mechanism involved in stimulation of phospho-inositide metabolism following sperm-egg interaction has not been determined in any species.

The 'sperm factor hypothesis' of signalling at fertilization proposes that spermatozoa contain a soluble Ca²⁺⁻ releasing factor that enters the egg after the gamete membranes fuse together and generates Ca²⁺ oscillations. This is consistent with the finding that cytoplasmic fusion of sperm and egg is a prelude to Ca²⁺ release. Direct support for this hypothesis comes from experiments where micro-injection into eggs of either single spermatozoa or soluble sperm extracts triggers Ca²⁺ oscillations similar to those at fertilization in mammalian - and some non-mammalian - eggs. The mammalian sperm factor that generates Ca²⁺ oscillations is protein-based; acts across species; and can cause Ca²⁺ release in somatic cells as well as in cell-free systems, such as sea urchin egg homogenates. Sperm specifically express a Ca²⁺ oscillation- inducing protein, because micro-injecting messenger RNA (mRNA) isolated from spermatogenic cells, but not mRNA from other tissues, elicits fertilization-like Ca²⁺ oscillations in mouse eggs.

In intact eggs and egg homogenates, mammalian sperm extracts trigger Ca²⁺ release *via* stimulating IP₃ production, indicating involvement of a phospho-inositide-specific phospholipase C (*ie* PI-PLC, usually referred to in short as PLC) in the signal transduction mechanism. The high level of PLC enzyme activity measured biochemically in sperm extracts has led some researchers to suggest that the sperm factor may itself be a PLC. However, the PLC-beta, gamma and delta (β, γ and δ) isoforms that exist in sperm are not detected in the chromatographic fractions of sperm extract that specifically cause Ca²⁺ oscillations. Also, when purified, recombinant PLCβ2, γ1 or δ1 proteins are added to egg homogenates, they fail to cause Ca²⁺ release. A PLCδ4 splice variant expressed in sperm has been shown to be involved in the acrosome reaction, rather than Ca²⁺ release in eggs at fertilization. Previous research in this field has been described in international patent specification no. WO 96/25495, to which reference should be made for a full understanding of, and as background to, the present application. The contents of WO 96/25495 are therefore incorporated herein by reference.

Patent specification no. WO 96/25945 assigned the cause of the above-mentioned calcium oscillations to a substance (a sperm factor) present in the equatorial segment of sperm, which was believed to diffuse into the egg after fusion therewith. This substance was identified as a 33kD (approx.) protein of specified amino acid sequence. The nucleic acid coding for this protein was also specified. However, after cloning the gene and undertaking subsequent expression studies, it was concluded that this sperm factor candidate was unable to reconstitute calcium oscillations. A truncate form of the *c-kit* receptor, has also previously been a sperm factor candidate. However, neither these two, nor any other sperm proteins, have been shown to generate Ca²⁺ oscillations in eggs, the single-most distinctive feature of mammalian fertilization.

These observations have led some workers in the field to conclude, "sperm-derived PLC is not responsible for initiating Ca²⁺ release at fertilization" (Mehlmann et al in Dev Biol 236 492-501 (2001)), whilst others have stated: "the identification of this protein remains a problem for the next century of fertilization research" (Runft et al in Dev Biol 245 237-54 (2002)).

On the contrary, these observations led us to investigate the possible existence of a distinct, uncharacterised sperm PLC isoform. The present invention relates to the presence of a new PLC isoform specifically expressed in mammalian sperm (hereinafter called PLC-zeta; PLCζ), which uniquely possesses all the essential properties of the sperm factor. The results of our studies are consistent with sperm PLC being the physiological trigger of egg activation, and thus an essential protein for mammalian fertilization and embryo development

The amino acid sequences of both the human and mouse proteins are given hereinafter as SEQ ID NOS: 1 and 2, respectively, and their nucleic acid coding sequences as SEQ ID NOS: 3 and 4, respectively. Also given is the rat protein as SEQ ID NO: 11, and its nucleic acid coding sequence SEQ ID NO: 10.

Recently, the Genbank database disclosed various nucleic acid sequences of human and mouse testes, without attributing any function thereto and predicting an open reading frame (ORF; protein or polypeptide sequence) having a start position corresponding to a position being at least 100 amino acids from the start position of the SEQ ID NOS: 3 and 4. In particular, Genbank Accession No AK006672 (deposited 05-JUL-2001) comprises 2227 base pairs of mouse testis sequence but predicts an ORF encoding 537 amino acids with a start position corresponding to position aa 111 (MEIDH) of the mouse sequence [SEQ ID NO: 4] (*ie* missing the first 110aa (amino acids).);

Genbank Accession No XM029802 (deposited 16-OCT-2001) comprises 2113 base pairs of human testis sequence, not identical to and predictive of an ORF encoding 504 amino acids with a start position corresponding to position aa 105 (MSKAI) of the human sequence [SEQ ID NO: 3] (*ie* missing the first 104aa);

Genbank Accession No NM033123 (deposited 21-AUG-2001) comprises 2132 base pairs of human testis sequence in database, but predicts an ORF encoding 504 amino acids with a start position corresponding to position aa 105 (MSKAI) of the human sequence [SEQ ID NO: 3] (*ie* missing the first 104aa); and

Genbank Accession No AY035866 (deposited 22-JUN-2001) comprises 2132 base pairs of human testis sequence in database, but predicts an ORF encoding 504 amino acids with a start position corresponding to position aa 105 (MSKAI) of the human sequence [SEQ ID NO: 3] (*ie* missing the first 104aa).

Genbank Accession No. AB070108 (deposited 16-AUG-2001) comprises 2219 base pairs of monkey testis sequence with an ORF of 1923 base pairs (nucleotides 220-2142) encoding 641 amino acids, without attributing any function thereto or connection with a putative sperm factor. [SEQ ID NOs: 6 and 7, respectively].

Similarly, Genbank Accession No. AB070109 (deposited 16-AUG-2001) comprises 2218 base pairs of monkey testis sequence with an ORF of 1920 base pairs (nucleotides 220-2139) encoding 640 amino acids, without attributing any function thereto or connection with a putative sperm factor. [SEQ ID NOs: 8 and 9, respectively].

The differences in the protein sequence between AB070108 and AB070109 are shown below:

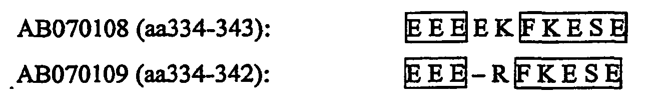

Accordingly, the present invention provides a PLC-zeta protein, characterised by exhibiting one or more of the following properties:
(a) An amino acid sequence comprising in the range of from 600 to 720, preferably 600 to 699, more preferably 600 to 650, amino acid residues;
(b) A domain sequence comprising the EF hand, X, Y, and C2 domains but absent the PH domain; and
(c) At least five consecutive amino acid residues from a conserved region, which region is selected from:
   (i) QDDFRGGKI (11-19);
   (ii) LLEKLD (27-32); and
   (iii) QGRIT (52-56) in the EF1 domain;
   (iv) ENRKIL (82-87); and
   (v) FLTQEQY (95-101) in the EF2 domain;
   (vi) YQQFNE (403-408) in the Y domain; and
   (vii) TLTIR (516-520);
   (viii) ISGIQLP (522-528); and
   (ix) LCMNKGYRR (609-617) in the C2 domain,
      wherein the residues are denoted by their conventional single letter codes and the numbers in parentheses refer to the sequence co-ordinates within the 641 amino acid ORF of the monkey AB070108 (monkey A) sequence.

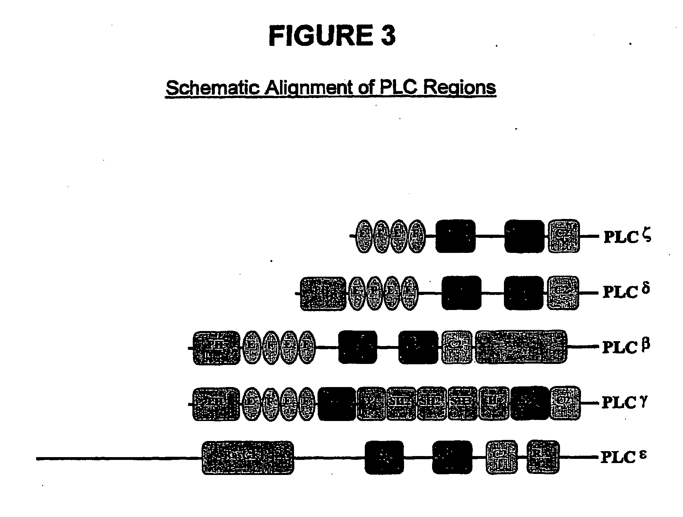

Below is a table (Table 1) showing a comparison between lengths of various PLCs (criterion (a)); Figure 3 shows the domain comparison between the various PLCs (criterion (b)); and [SEQ ID NO: 12] illustrates the conserved regions of PLC-zeta cross-species, compared to other PLCs (criterion (c)). Comparison between the PLC sequence types was made using the Clustal W analysis program, available at http://www.clustalw.genome.ad.jp, using the default settings.

**Table 1: PLCs - Sequence Lengths**

| **PLC Types** | **Species** | **No. of amino acid residues** |
|---|---|---|
| zeta | human | 608 |
| zeta | monkeyA | 641 |
| zeta | mouse | 647 |
| zeta | rat | 646 |
| | | |
| beta 1 | human | 1211 |
| beta 2 | human | 1181 |
| beta 3 | mouse | 1234 |
| beta 4 | mouse | 1175 |
| gamma 1 | human | 1290 |
| gamma 2 | human | 1252 |
| delta 1 | human | 756 |
| delta 2 | bovine | 764 |
| delta 3 | human | 736 |
| delta 4 | rat | 772 |
| epsilon | human | 2302 |
| 1 | potato | 596 |
| 2 | potato | 565 |
| 3 | potato | 585 |

Accordingly, the present invention provides an isolated, purified or recombinant nucleic acid molecule comprising:
a nucleic acid molecule encoding a PLC-zeta; PLCζ, polypeptide, capable of triggering
calcium oscillations in oocytes.

The nucleic acid molecule of the invention is identified by the virtue of the sequences disclosed herein and further includes sequences substantially homologous thereto or sequences that hybridize thereto under stringent conditions.

In a further aspect of the invention there is provided at least one oligonucleotide specific for a part of the aforementioned sequences. Preferably, said oligonucleotide includes the primers described herein and more specifically the following:
Forward human primer: 5' CAG CGA GCT CTT ATC TGA CGT ACC AAA C 3' (28mer).
Reverse TriplEx primer: 5' CTC GGG AAG CGC GCC ATT GTG TTG GT 3' (26mer).
Forward mouse primer: 5' GCT AAC GCG TCA GTT ACA TGC GTC ACT C 3' (28mer)
Reverse T7 primer: 5' GTA ATA CGA CTC ACT ATA GGG C 3' (22mer)
Forward human primer: 5' CAG CGA GCT CTT ATC TGA CGT ACC AAA C 3' (28mer)
Reverse human primer: 5' ATG AAA CTA TGG AAA TGA GAT GGT 3' (24mer)
Forward mouse primer: 5' GCT AAC GCG TCA GTT ACA TGC GTC ACT C 3' (28mer)
Reverse mouse primer: 5' ATC ATG GAA AGC CAA CTT C 3' (19mer)

By "substantially homologous" herein is meant that the nucleic acid sequence has at least 70% identity of its nucleotide bases with those of sequence (a), in matching positions in the sequence. A further 10% of its nucleotide bases may comprise conservative substitutions (with similar bases), and therefore the sequence has at least 80% overall homology. More preferred are sequences having at least 80% identity with the sequence (a) and about 90% overall homology. Such homologous sequences encode a protein having substantially the same biological activity as the proteins of the invention.

Oligonucleotides "specific for" any of these nucleic acid sequences (a) to (c) above are useful for identifying and isolating the biologically active peptides of this invention, and comprise a unique sequence encoding a unique fragment of the amino acid sequence of the peptide.

In particular, the present invention provides a nucleic acid sequence as defined above,
wherein the sequence is a DNA or RNA sequence, such as cDNA, cRNA or mRNA. More particularly, the present invention provides:
a DNA sequence identified herein by [SEQ ID NO: 3], which sequence (being the human PLC-zeta; PLCζ nucleotide sequence, 1827 nucleotides) corresponds with the polypeptide identified herein as [SEQ ID NO: 1];
a DNA sequence identified herein by [SEQ ID NO: 4], which sequence (being the mouse PLC-zeta; PLCζ nucleotide sequence, 1944 nucleotides) corresponds with the polypeptide identified herein as [SEQ ID NO: 2]; and
a DNA sequence identified herein by [SEQ ID NO: 10], which sequence (being the rat PLC-zeta; PLCζ nucleotide sequence, 1938 nucleotides) corresponds with the polypeptide identified herein as [SEQ ID NO: 11].

The mouse sequence has been deposited under Genbank Accession No AF 435950, which comprises 1941 nucleotides of the protein-coding region plus the stop codon (3 nucleotides) (these, together, consist of the [SEQ ID NO: 4]) plus the untranslated region (totalling 2187 nucleotides) identified herein as [SEQ ID NO: 5].

Therefore, the present invention further provides a polypeptide of
[SEQ ID NO: 1], being the human PLC-zeta; PLCζ amino acid sequence, 608 residues;
[SEQ ID NO: 2], being the mouse PLC-zeta; PLCζ amino acid sequence, total 647 residues; and
[SEQ ID NO: 11], being the rat PLC-zeta; PLCζ amino acid sequence, total 646 residues, in which amino acids are represented by their conventional single letter codes.

Furthermore, the invention provides for the use of certain known sequences to which a function has not previously been assigned as a PLC-zeta, PLCζ or sperm factor. In particular, the invention provides for such use of the monkey proteins [SEQ ID NOs: 7 and 9].

The deduced human and mouse proteins of SEQ ID NOS: 1 and 2 differ by 39 amino acids in length and their cDNA sequences differ correspondingly. It will be appreciated that similarly active proteins and corresponding nucleic acid sequences encoding them will be present in the sperm of other mammalian species, including species of farm animals *eg* sheep and pigs, and other animal species *eg* fish. All such proteins and nucleic acid sequences have a high degree of sequence homology with one another, and can be readily isolated using the newly discovered DNA sequences or parts thereof to probe the appropriate cDNA libraries of other species. It is expected that the molecular weight of the proteins will be in the range of from 65 to 80 kD, preferably in the range of from 70 to 75 kD, especially about 70kD, as determined by mass spectrometry.

Derivatives of the proteins disclosed herein (*ie* of [SEQ ID NOS: 1, 2 and 11], and homologous sequences) having substantially similar biological activity are also encompassed. For example, one or more of which derivatives may comprise post-translational modifications, such as glycosylation at asparagine, serine or threonine; and/or sulphato- or phospho- groups on tyrosine, such as are commonly found in polypeptides; polymorphisms, such as single nucleotide polymorphisms (SNPs); and those further comprising a leader/signal sequence.

The invention further provides a tagged derivative of a PLC-zeta, such as a tagged derivative of any polypeptide sequence specifically identified herein, including [SEQ ID NOs: 1, 2, 7, 9 and 11], for use in identifying the PLC-zeta in diagnostic tests, other assays or otherwise as a research or clinical tool. Suitably, the PLC-zeta is tagged with c-Myc as described in Example 6 hereinbelow, antibodies to which are commercially available (*eg* from Santa Cruz Biotechnology).

A polypeptide encompassed by this invention can also be prepared by providing or culturing a host, transformed with an expression vector comprising a DNA sequence encoding the polypeptide under such conditions that the polypeptide is expressed therein, and optionally isolating the polypeptide thus obtained. This approach is typically based on obtaining a nucleotide sequence encoding the polypeptide it is wished to express, and expressing the polypeptide in a recombinant organism. The cultivation of the genetically modified organism leads to the production of the desired product displaying full biological activity. The present invention therefore also comprises a polypeptide produced by a recombinant DNA technique, which polypeptide is one encompassed above. The invention further comprises a synthetic, or protein-engineered, polypeptide encompassed above.

The present invention therefore further provides: a recombinant construct comprising any nucleic acid sequence according to the invention; a vector comprising such a construct; and a host transformed or transfected by such a vector.

The present invention therefore still further provides a cultured or non-human cell, plasmid, virus, non human live organism or other vehicle that has been genetically- or protein-engineered to produce a polypeptide according to the present invention, said cell, plasmid, virus, non human live organism or other vehicle having incorporated expressibly therein a sequence as disclosed herein. Such cells may include animal, such as mammal, for example human or humanised cells, for use in gene therapy to treat or prevent conditions such as those mentioned herein. Such cells particularly include stem cells derived by cell nuclear transfer in accordance with the present invention. The present invention therefore also further provides animal clones derived from nuclear transfer techniques enhanced by using the PLC-zeta of this invention.

Therefore, the present invention further provides a method for the preparation of a polypeptide according to the present invention, which method comprises:
(a) isolation and/or purification from mammalian sperm; or
(b) expression of a nucleic acid sequence encoding the polypeptide and, optionally, isolation and/or purification of the resulting polypeptide.

The present invention therefore comprises *inter alia* the human, mouse, rat or other mammalian protein PLC-zeta, or non-mammalian (*eg* fish) PLC-zeta, the nucleic acid sequence coding therefor, cells transfected with the nucleic acid sequence, and a process for producing PLC-zeta by cultivation of the transfected cells and recovery of the expressed product.

The recombinant proteins, especially the mouse (including the c-Myc-tagged mouse), monkey (both AB 070108 and AB070109) and human PLC-zeta, have been shown to generate cytoplasmic calcium oscillations (CCOs) when introduced into mammalian cells. Furthermore, the injection of complementary RNA (cRNA) encoding PLC-zeta into mouse eggs also generates identical CCOs to those observed when they are fertilized by sperm. It has also been found that PLC-zeta is capable of producing embryo development to the blastocyst stage (*ie* the stage at which stem cells are found).

Accordingly, the invention also provides a variety of applications and/or uses of the proteins and nucleic acid sequences of this invention, including the following:

### 1. Treatment of mammalian infertility:

The human PLC-zeta; PLCζ protein we have identified may be used in treating human male infertility. This PLC-zeta; PLCζ protein triggers calcium changes upon sperm fusion with egg, the physiological process which results in egg activation and consequent embryo development. Absence or significant reduction of the level of active PLC-zeta; PLCζ in sperm would be expected to result in infertile males. That the PLC-zeta; PLCζ protein is highly expressed in mammalian testis is supported by the following:
(a) the cDNA has been isolated from testis cDNA libraries (human testis and mouse spermatid); and
(b) search of the EST database using our PLC-zeta; PLCζ sequences human and mouse reveals sequence matches found in testis-derived cDNA libraries.

Assay of the PLC-zeta; PLCζ protein in human sperm samples may therefore be used to identify males who have less than normal levels of the active protein (*ie* protein having the ability to cause cell calcium oscillations) and are infertile for this reason. This assay may be achieved by the use of antibodies to the protein prepared by methods well known to those skilled in the art.

To correct such deficiencies, the addition of active PLC-zeta; PLCζ to sperm lacking an active PLC-zeta; PLCζ can be carried out in conjunction with the clinical IVF (*in vitro* fertilization) technique of intra-cytoplasmic sperm injection, ICSI (Intra-Cytoplasmic Sperm Injection, comprising introduction of a single sperm directly into the egg). The ICSI procedure has been successfully used by major IVF clinics to produce thousands of live human births.

### 2. Improvement in stem cell production:

The ethical use of 'spare' embryo-derived stem cells in therapy of human degenerative diseases has been of great public debate recently. The generation of stem cells directly from a patient would remove the need for use of donated embryos. Cloning of cells, tissues and animals (*eg* 'Dolly', the sheep) have been achieved by fusing a somatic cell with an enucleated egg. Activation of the fused egg to trigger development of the hybrid cell to form a blastocyst, from which stem cells can be harvested, is a very low efficiency process with <1% success rate. Thus, the application of a native protein with a physiologically relevant activity, that is, to trigger egg activation, following the fusion process occurring between the somatic cell and the egg cell, would enhance the success rate of fused cells in proceeding to develop further.

Stem cells derived from nuclear transfer techniques enhanced by using PLC-zeta have potential application to a variety of human diseases and conditions, including Parkinsonism, Alzheimer's disease, heart failure and diabetes, to which stem cell therapy could be applied

### 3. Animal cloning:

An extension of the application 2., above, is to implant the successfully developing blastocyst into a pregnant female host to produce full development to term and live birth of clones derived from a single adult animal cell. This process is currently being developed for the production of biomedicines in transgenic animals, e.g. sheep and pigs, as well as for the potential use of animal cells and organs for transplantation into humans but the current success rate for this procedure, as mentioned above is very low, <1%, due to the difficulties in achieving viable hybrid cells upon fusion.

In another aspect, the present invention provides a method for the treatment or prevention of a condition or disorder mentioned herein, wherein the polypeptide is administered by means of being expressed in the cells of the patient, which cells have incorporated expressibly therein a nucleic acid sequence coding for the polypeptide. Alternative to gene therapy, the polypeptides of the invention may be administered as a pharmaceutical formulation.

Accordingly, the present invention provides the use of a polypeptide described herein or a nucleic acid sequence coding for the polypeptide in medicine, including gene therapy; and also the use of such a polypeptide in the manufacture of a medicament.

Therefore, according to a further aspect of the present invention, there is provided a pharmaceutical formulation comprising a polypeptide according to the invention (as described above) and a pharmaceutically acceptable carrier therefor. The term "pharmaceutically acceptable carrier" as used herein should be taken to mean any inert, non-toxic, solid or liquid filler, diluent or encapsulating material, or other excipient, which does not react adversely with the active ingredient(s) or with a patient.

Such formulations and carriers are well known in the art and include pharmaceutical formulations that may be, for example, administered to a patient systemically, such as parenterally, or orally or topically.

The term 'parenteral' as used here includes subcutaneous, intravenous, intramuscular, intra-arterial and intra-tracheal injection, and infusion techniques. Parenteral formulations are preferably administered intravenously, either in bolus form or as a continuous infusion, or subcutaneously, according to known procedures. Preferred liquid carriers, which are well known for parenteral use, include sterile water, saline, aqueous dextrose, sugar solutions, ethanol, glycols and oils.

Tablets and capsules for oral administration may contain conventional excipients such as binding agents, fillers, lubricants, wetting agents, and the like. Oral liquid preparations may be in the form of aqueous or oily suspensions, solutions, emulsions, syrups, elixirs or the like, or may be presented as a dry product for reconstitution with water or other suitable vehicle for use. Such liquid preparations may contain conventional additives, such as suspending agents, emulsifying agents, non-aqueous vehicles and preservatives.

Formulations suitable for topical application may be in the form of aqueous or oily suspensions, solutions, emulsions, gels or, preferably, emulsion-based ointments.

Unit doses of the pharmaceutical formulations according to the invention may contain daily-required amounts of the polypeptides, or sub-multiples thereof to make a desired dose. The optimum therapeutically-acceptable dosage and dose rate for a given patient (which may be a mammal, such as a human) depend on a variety of factors, such as the potency of the active ingredient(s); the age, body weight, general health, sex and diet of the patient; the time and route of administration; rate of clearance; the object of the treatment (for example, treatment or prophylaxis); and the nature of the disease to be treated.

It is expected that systemic doses in the range of from 0.005 to 50 mg/kg body weight, preferably of from 0.005 to 10 mg/kg and more preferably 0.01 to 1 mg/kg, will be effective. According to the nature of the disease being treated, one single dose may comprise in the range of from 0.005 to 10 mg/kg body weight active ingredient, whether applied systemically or topically.

The present invention therefore further provides:
(a) the use of a polypeptide of this invention in therapy;
(b) the use of a polypeptide of this invention in the preparation of a medicament;
(c) a method for the treatment or prevention of a condition in a patient, which condition involves suppression, inhibition or inactivation of the generation of CCOs, which method comprises administration to said patient of a non-toxic, inhibitory amount of a polypeptide of the invention;
(d) the use of a polypeptide of this invention in the generation of CCOs in mammalian cells;
(e) a method of treating male infertility in a mammal, which method comprises adding the polypeptide of this invention to the sperm of the mammal; and
(f) a method of improving oocyte-somatic cell nuclear transfer efficiency in cell cloning, which method comprises adding a polypeptide according to this invention, or nucleic acid encoding the polypeptide, to an oocyte before or after fusion with the contents of a somatic cell.

Furthermore, the protein or nucleic acid sequence coding therefor according to this invention may be used in a diagnostic method to determine the state of fertility (*eg* whether fertile or infertile) of a respective mammal, such as a human.

Accordingly, the present invention further provides a diagnostic method for determining the fertility status of a mammal, which method comprises determining the amount of a protein according to this invention, or nucleic acid sequence coding therefore, present or absent in a test sample obtained from the mammal, which amount is indicative of the level of fertility of the mammal.

A further diagnostic or screening method comprises:
(a) obtaining a test sample comprising a nucleotide sequence of the mammalian PLCζ gene from the individual; and
(b) comparing a region of the sequence obtained from the test sample with the corresponding region of a wild type mammalian PLCζ sequence, such as [SEQ ID NO: 3, 4, 5, 6, 8 or 10] whereby a variation in the sample sequence relative to the predetermined sequence is indicative of a condition, such as lowered fertility or infertility, associated with disruption in calcium oscillation patterns that are a prerequisite to normal biological function absent the condition.

Preferably, the test sample comprises genomic DNA. -

A particularly preferred screening method is one for screening an individual suspected of a fertility problem, which screening method comprises the steps of:
(a) obtaining a test sample comprising a nucleotide sequence of the human PLCζ gene or an amino acid sequence encoded thereby from the individual; and
(b) analysing the test sample for the presence of a variant of the human PLCζ gene or an amino acid sequence encoded thereby or for the presence of one or more surrogate markers that are indicative of or correlated to the presence of a variant of the human PLCζ gene or an amino acid sequence encoded thereby,
wherein the variant of the human PLCζ gene or an amino acid sequence encoded thereby exhibits at least one variation when compared to the wild type PLCζ sequence.

It will be evident to the person skilled in the art that the above methods apply equally to other mammals than humans and to other animals than mammals.

The analysis step (b) may be selected from one or more of: conventional protein sequencing methods (such as mass spectroscopy, micro-array analysis, pyrosequencing, etc), and/or antibody-based methods of detection (*eg* ELISA). In any of the methods according to the invention, antibodies to the protein may be raised.

Therefore, in a method of testing for male infertility, which method comprises assaying the protein PLCζ in a sperm sample, the method could be carried out using an antibody to the protein, in particular, a monoclonal antibody to the protein PLCζ. Alternatively, the PLC-zeta gene sequence may be determined in a sample comprising genomic DNA, using methods known to those skilled in the art, such as PCR amplification, restriction enzyme analysis and DNA sequencing.

Accordingly, the present invention still further provides an antibody raised to a polypeptide according to the invention, particularly a monoclonal antibody thereto.

The screening method may comprise the use of simultaneous screens for multiple, known variations or for all possible variations by hybridization of a labelled sample of DNA (cDNA or genomic DNA derived from the individual) to micro-arrays of variation-specific oligonucleotide probes immobilised on a solid support. For example, chip technology may be used, wherein the chip is a miniature parallel analytical device.

The methods of the invention may be carried out using a kit, which kit may comprise:
(a) an oligonucleotide comprising a nucleic acid sequence corresponding to a region of a PLCζ variant, which region incorporates at least one variation from the corresponding wild-type PLCζ gene sequence; and/or
(b) an oligonucleotide comprising a nucleic acid sequence corresponding to the wild-type PLCζ gene sequence in the region specified in (a); and/or
(c) an oligonucleotide comprising a nucleic acid sequence corresponding to a specific region of the wild-type PLCζ gene sequence, which specific region comprises a sequence not otherwise present in the genomic DNA of the mammal; and/or
(d) antibodies, such as monoclonal antibodies, raised to any peptide sequence corresponding to an oligonucleotide specific to any one of (a) to (c) above; and, optionally,
(e) one or more reagent(s) suitable for amplifying (*eg* by carrying out PCR) desired regions of the individual's DNA.

Preferably, any of kit components (a) to (c) comprise(s) a plurality of said oligonucleotides immobilised on a solid support.

In a further aspect, the present invention provides an inhibitor or antagonist of PLC-zeta for use in reducing, suppressing or preventing cytoplasmic calcium oscillations in oocytes and/or for reducing or inhibiting fertility. Such PLC-zeta inhibitors or antagonists may comprise known chemical compounds, biological material or other agents, or may comprise new active agents. Accordingly, the invention further provides an active agent suitable for reducing, suppressing or preventing cytoplasmic calcium oscillations in oocytes and/or for reducing or inhibiting fertility, which active agent is an inhibitor or antagonist of PLC-zeta. Such active agents may be provided in the form of a pharmaceutical formulation in association with a pharmaceutically acceptable carrier therefore, as described above, and may be suitable for use as a male contraceptive.

The invention will now be further described in the following, non-limiting, Examples, with reference to the accompanying Figures 1 to 5, in which:
Figure 1: is a plot of calcium concentration (nM; ordinate) with time (secs; abscissa), showing expression of mouse PLC-zeta plasmid DNA by transfection in CHO cells;
Figure 2: is a plot of calcium concentration (nM; ordinate) with time (secs; abscissa), showing expression of mouse PLC-zeta complementary RNA by micro-injection into mouse eggs;
Figure 3: is a schematic alignment of PLC regions, showing similarities and differences between PLC-zeta and other PLCs;
Figure 4a: is a graph of the percentage of mouse eggs reaching 2-cell stage after 24 hours and morula/blastocyst stage after 96 hours, following micro-injection with PLC-zeta cRNA (0.02 mg/ml) or pathogenically activated with strontium (5 mM, 4 hours) or fertilised with sperm in vivo and placing in a 5% CO2 incubator at 37C;
Figure 4b: comprises two micrographs illustrating mouse embryos at the 2-cell stage and blastocyst stage, respectively, following the treatment illustrated in Figure 4a;
Figure 5: is a micrograph illustrating mouse egg 24 hours following micro-injection with ^{D210R}PLC-zeta, illustrating lack of development to 2-cell stage.
Figure 6a: shows dose-dependent calcium oscillations in fura-red loaded mouse eggs, triggered by micro-injection of cRNA encoding mouse sperm PLC-zeta (2 and 0.002 mg/ml, top and middle travces, respectively) and after pre-incubation with 10 uM cycloheximide (0.02 mg/ml, bottom trace); and
Figure 6b: illustrates the mean interspike interval of calcium oscillations in eggs, following micro-injection of various PLC-zeta cRNA concentrations. Compared with the interval observed upon *in vitro* fertilisation (IVF). * indicates statistically significant (Student's unpaired t-test) from IVF at the 5% level.

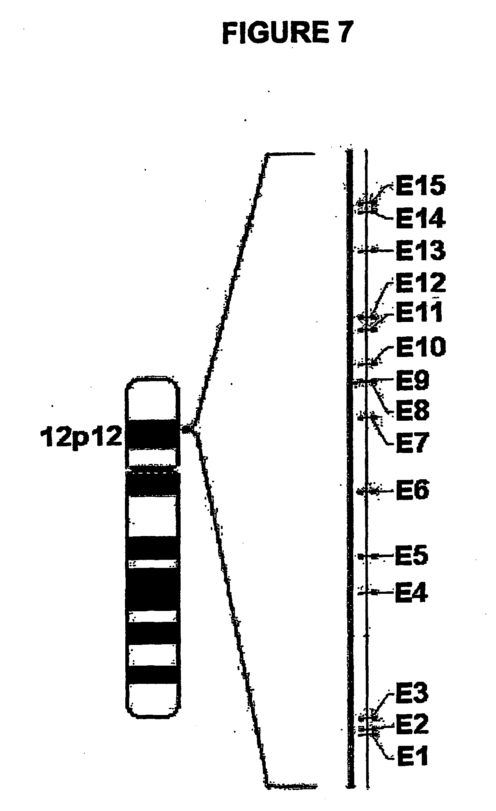

Figure 7: Structure of the human *plc-zeta* gene.
   The genomic organisation of the fifteen *plc-zeta* exons identified within the 179456 bp contig (Accession number AC023940) are shown aligned to a 54.8 kb region of chromosome 12 (12p12.3). Exons are labelled E1 to E15. The start and stop codons for hPLCæ are located within E2 and E 15, respectively. Solid line between exons represent the introns (see Table 2).

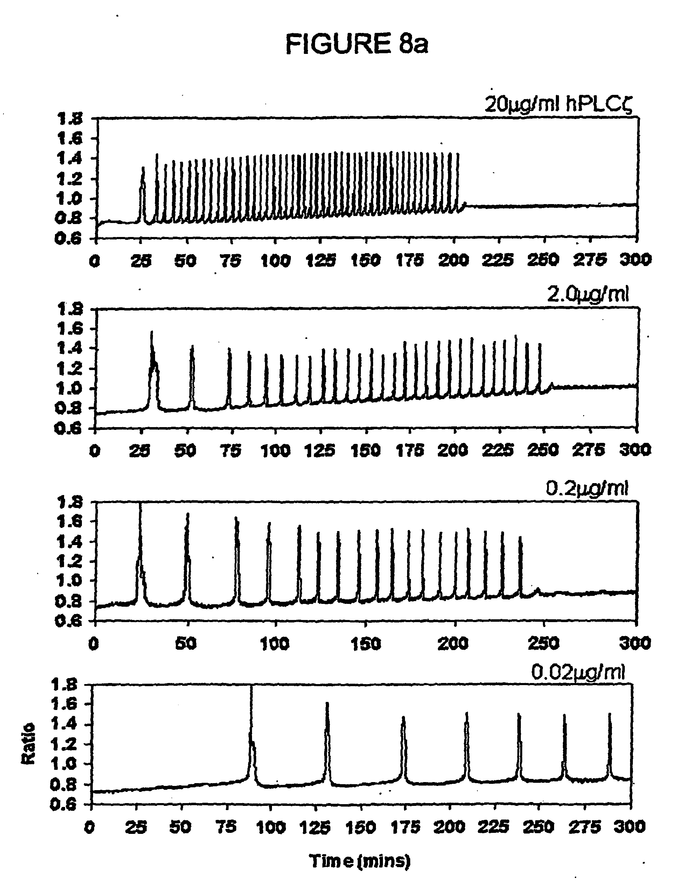

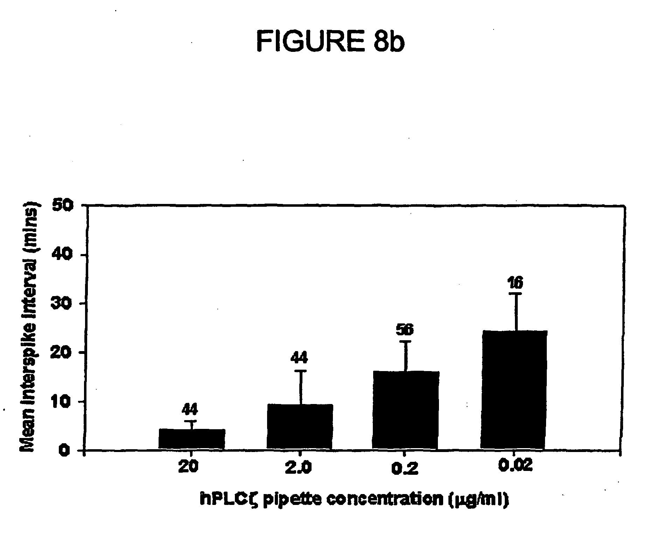

Figure 8: Ca2+ oscillations in mouse oocytes microinjected with human PLC-zeta cRNA. A. Dose-dependent Ca2+ oscillations in MII-arrested mouse oocytes after microinjection of hPLC-zeta cRNA. The four traces show the cytoplasmic Ca2+ oscillations observed upon microinjection with cRNA at the indicated pipette concentration, from 20 to 0.02 µg/ml. B. Mean interspike interval of Ca2+ oscillations in mouse oocytes triggered by the various hPLC-zeta cRNA concentrations. The number of microinjected oocytes is shown above each dose. The mean interspike interval at each dose is statistically different from each other using a students paired t-test, p = <0.0001 (20 µg/ml, 4.21 ± 1.79; 2.0 µg/ml, 9.26 ± 7.14;0.2 µg/ml, 16.0 ± 6.40; 0.02 µg/ml, 24.34 ± 7.68).

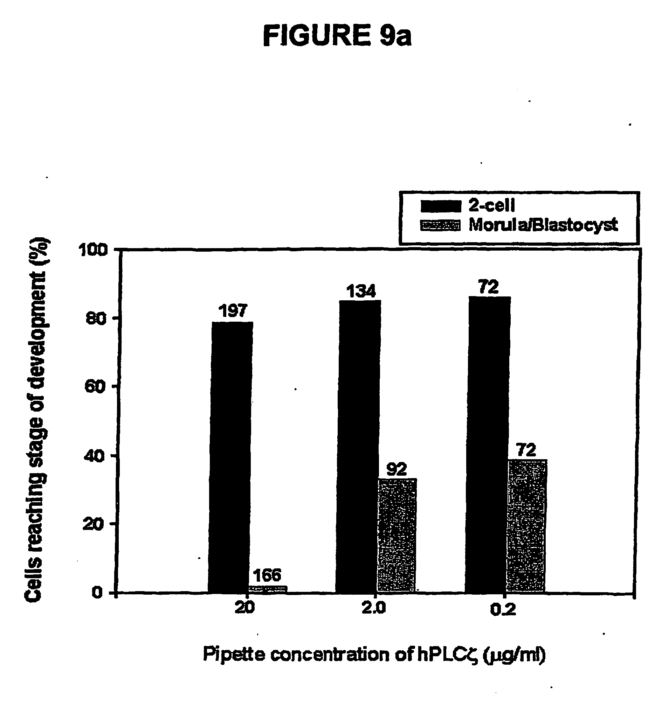

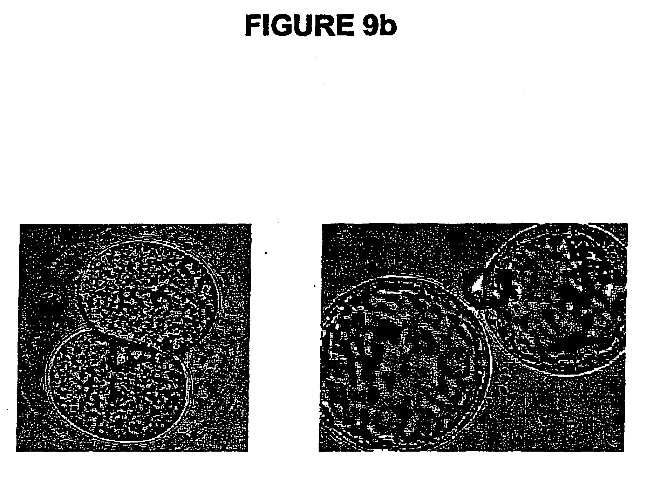

Figure 9: Embryonic development of mouse oocytes microinjected with human PLC-zeta cRNA.
   A. Mouse oocytes were microinjected with different hPLC-zeta cRNA concentrations (20-0.2 µg/ml). The percentage of oocytes reaching the 2-cell stage after 24 hours and morula/blastocyst after 96 hours were recorded.
   B. Micrographs showing development of mouse embryos at the 2-cell stage (left) and blastocyst stage (right) following microinjection of unfertilized oocytes with hPLC-zeta cRNA (0.2 µg/ml).

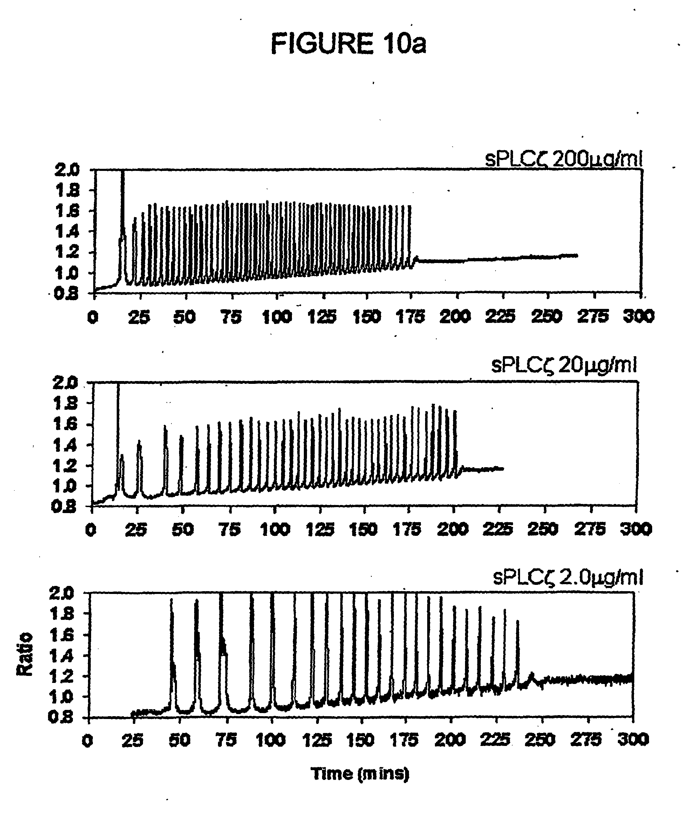

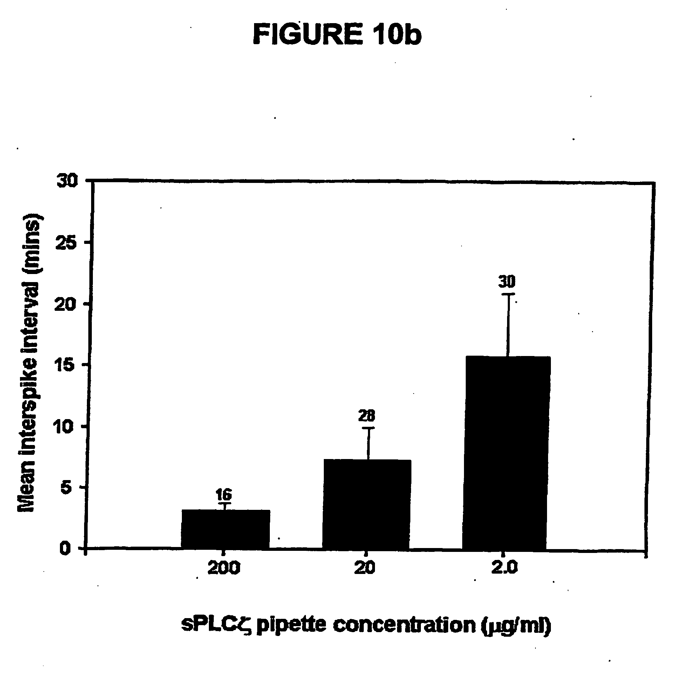

Figure 10: Ca2+ oscillations in mouse oocytes with simian PLC-zeta cRNA.
   A. Dose-dependent Ca2+ oscillations in MII-arrested mouse oocytes after microinjection of sPLC-zeta cRNA. The three traces show the cytoplasmic Ca2+ oscillations observed upon microinjection with cRNA at the indicated pipette concentration, from 200 to 2 µg/ml.
   B. Mean interspike interval of Ca2+ oscillations in mouse oocytes triggered by the various sPLC-zeta cRNA concentrations. The number of microinjected oocytes is shown above each dose. The mean interspike interval at each dose is statistically different from each other using a students paired t-test, p = <0.0001 (200 µg/ml, 3.18 ± 0.55; 20 µg/ml, 7.35 ± 2.69; 2.0 µg/ml, 15.77 ± 5.20).

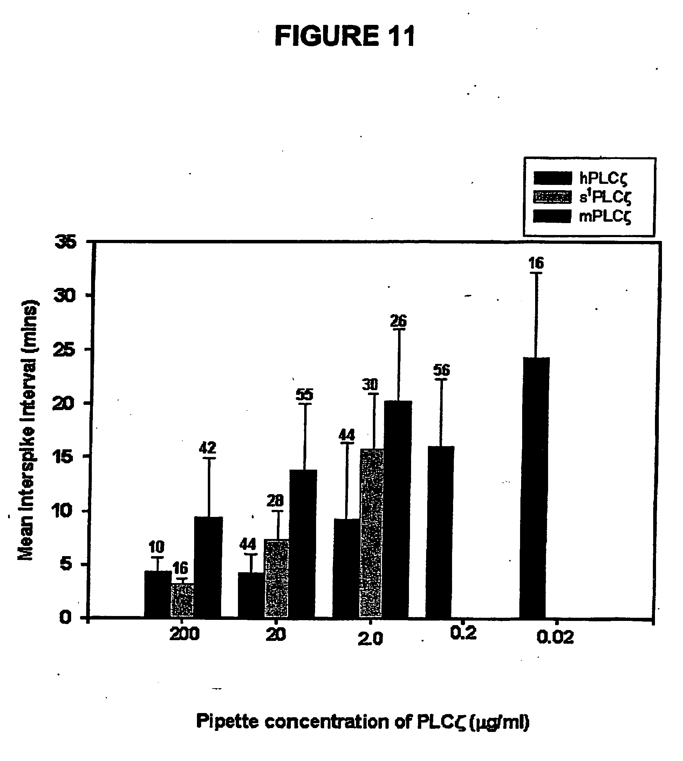

Figure 11: Mean interpike intervals observed with human, simian and mouse PLC-zeta cRNA.

Comparison of the mean interspike interval of Ca2+ oscillations in mouse oocytes triggered by the three species of PLC-zeta cRNA. Human, simian and mouse PLC-zeta cRNAs each triggered Ca2+ oscillations within 2 hours of microinjection of 200-2.0 µg/ml PLC-zeta cRNA. Only hPLC-zeta was effective at the lower doses of 0.2 and 0.02 µg/ml. The number of oocytes microinjected is shown above each dose. The mean interspike interval at each dose for human, simian and mouse is statistically different from each other using a students paired t-test (p = <0.005).

Table 2 shows the genomic organization of the human PLC-zeta gene. The gene is localized to chromosome 12p12.3

### EXAMPLE 1: Isolation of the nucleic acid/protein by PCR cloning (Human)

The human expressed sequence tag (EST) database at NCBI (National Center for Biotechnology Information, National Library of Medicine, National Institutes of Health, Bethesda, MD 20891, U.S.A.) was searched using the BLAST algorithm (http://www.ncbi.nlm.nih.gov/BLAST/) for phospho-inositide-specific phospholipase C-related sequences using the published sequence of the rat phospholipase C delta 4 isoform (NCBI accession number U16655 -). Of the numerous positive 'hits' that were obtained, a class of novel ESTs was observed to be derived from human testis cDNA (*eg* accession numbers AI217888; AA707583; AA861064; AA609626).

Using the same approach for database searching as above, the mouse EST database at NCBIgave a related class of novel ESTs derived from mouse testis cDNA (*eg* accession numbers AV257260, AV277909, AV273316, and AV277562).

All these ESTs represent partial testis cDNA sequences (comprising fewer than 400 base pairs), as a complete open reading frame (ORF) was not identified in any of them.

Using polymerase chain reaction (PCR) cloning techniques with specific oligonucleotides designed to amplify sequences related to those ESTs described above, the complete protein coding sequence of the human and mouse phospholipase C-zeta; PLCζ were obtained as follows:
The primers used for PCR from a human testis cDNA library (Clontech Laboratories 1020 East Meadow Circle, Palo Alto, CA94303-4230, U.S.A. #HL5503u) were:
   Forward human primer: 5' CAG CGA GCT CTT ATC TGA CGT ACC AAA C 3' (28mer)
   Reverse TriplEx primer: 5' CTC GGG AAG CGC GCC ATT GTG TTG GT 3' (26mer)

The forward primer was derived from the human EST sequences and included the predicted stop codon TGA, underlined. The reverse primer encoded the Clontech lambda TriplEx2 vector sequence. PCR was performed in a 50uL reaction volume with initial denaturation at 96 ° C for 3 minutes, followed by 30 cycles of 94°C for 30 seconds, 60 ° C for 30 seconds and 72 ° C for 3 minutes, and a final extension at 72 °C for 5 minutes. The single ∼2 kilobase product amplified using these primers with Pfu DNA polymerase, according to manufacturer's instructions (Promega Corporation catalogue # M7745, Promega UK Ltd, Delta House, Chilworth Research Centre, Southampton SO16 7NS, U.K.), was cloned into the commercial vector pTOPO-Blunt and plasmids transformed into competent *E. coli* for plasmid DNA preparation according to manufacturer's instructions (Invitrogen Inc. catalogue no. K2800-20, Invitrogen BV, PO Box 2312, 9704 CH Groningen, The Netherlands). Plasmid DNA was isolated from *E. coli* cultures using Qiagen miniprep purification columns according to manufacturer's instructions (Qiagen cat. no. 12125, QIAGEN Ltd. - UK, Boundary Court, Gatwick Road, Crawley, West Sussex, RH10 9AX, U.K.).

The primers used for PCR from a mouse spermatid cDNA library (made using the lambda ZAP II vector (available from Stratagene Inc. 11011 North Torrey Pines Road, La Jolla, CA 92037, U.S.A.), provided by Dr. Paul Burgoyne, National Institute for Medical Research, London) were:
Forward mouse primer: 5' GCT AAC GCG TCA GTT ACA TGC GTC ACT C 3' (28mer)
Reverse T7 primer: 5' GTA ATA CGA CTC ACT ATA GGG C 3' (22mer)

The forward primer was derived from the mouse EST sequences and included the predicted stop codon TCA, underlined. The reverse primer encoded Stratagene lambda ZAP II vector sequence (T7 sequence). PCR was performed in a 50uL reaction volume with initial denaturation at 96°C for 3 minutes followed by 30 cycles of 94 °C for 30 seconds, 60°C for 30 seconds and 72 ° C for 3 minutes, and a final extension at 72°C for 5 minutes. The single ∼2 kilobase product amplified using these primers with Pfu DNA polymerase, according to manufacturer's instructions (Promega Corp.), was cloned into the commercial vector pTOPO-Blunt and plasmids transformed into competent *E.coli* for plasmid DNA preparation according to manufacturer's instructions (Invitrogen Inc.). Plasmid DNA was isolated from *E.coli* cultures using Qiagen Miniprep™ purification columns according to manufacturer's instructions (Qiagen).

Nucleotide sequence analysis of the amplified and cloned human and mouse DNAs was determined by standard dideoxy sequencing performed on an Applied Biosystems ABI377 automated DNA sequencer using the dRhodamine dye terminator kit (PE Applied Biosystems, Kelvin Close, Birchwood Science Park North, Warrington, WA3 7PB, U.K.). Open reading frame (ORF) analysis of the complete human and mouse nucleotide sequences using MacVector sequence analysis software (Oxford Molecular, The Medawar Centre, Oxford Science Park, Oxford, OX4 4GA, U.K.) revealed the complete protein coding sequence of the human and mouse PLC-zeta; PLC proteins. The human sequence revealed an ORF of 1824 base pairs encoding a 608 amino acid sequence (SEQ ID NO: 1). The mouse sequence revealed an ORF of 1941 base pairs encoding a 647 amino acid sequence (SEQ ID NO: 2).

### Identification and cloning of simian PLC-zeta

A cynomolgus monkey cDNA library was prepared from size-selected, adult *Macaca fascicularis* testes cDNAs of >1.5 kb, and a number of novel, full-length insert DNA sequences were determined. Blast searching with the hPLC-zeta sequence revealed two homologous simian sequences derived from the adult *M. fascicularis* testis cDNA library (Accession numbers, AB070108 and AB070109). The ORF within these two cynomolgusmonkey cDNA clones were amplified by PCR with *Pfu* DNA polymerase, as described above, cloned into pcDNA3.1-V5-His-TOPO (Invitrogen) (pcDNA-zeta) and the insert DNA sequenced along both strands, as described above. Homology sequence analysis and alignment was performed using ClustalW (www.clustalw.genome.ad.jp) and domain structure by RPS-Blast (www.ncbi.nlm.nih.gov/structure/cdd).

### EXAMPLE 2: Preparation of recombinant vectors for expression in mammalian cells

The complete ORF of both human and mouse PLC-zeta; PLCζ sequences were subcloned into the mammalian expression vector, pTargeT (Promega, Delta House, Chilworth Research Centre, Southampton SO16 7NS, U.K.). The full-length sequences were amplified by PCR with Pfu polymerase (Promega) as described above, using specific oligonucleotides designed to the start and stop codons as follows:
The human primers used were:
   Forward human primer: 5' CAG CGA GCT CTT ATC TGA CGT ACC AAA C 3' (28mer)
   Reverse human primer: 5' ATG AAA CTA TGG AAA TGA GAT GGT 3' (24mer)

The reverse human primer included the start codon, ATG, underlined, and the forward human primer included the stop codon as used in the orginal PCR cloning steps described above. PCR was performed as described above. The -1.8 kilobase product was cloned into pTOPO-Blunt and the DNA insert was sequenced as described above. The ∼1.8 kilobase human DNA insert was excised from the pTOPO-Blunt vector by digestion with the restriction enzyme *EcoR1*, the restricted fragment was separated by agarose ge1 electrophoresis, purified using the Qiagen DNA ge1 extraction kit and ligated into the *EcoR1* pre-digested mammalian vector, pTarget. Ligation was performed at 12 °C overnight in the presence of 10 units of T4 DNA ligase (Promega), and ligated plasmid was transformed into competent E.coli XL-1 Blue (Stratagene), and plasmid DNA purified using Qiagen columns as described above. Restriction enzyme digestion of plasmid DNA revealed the clones containing the correct orientation of the human PLC-zeta; PLC insert.

The mouse primers used were:
Forward mouse primer: 5' GCT VAC GCG TCA GTT ACA TGC GTC ACT C 3' (28mer)
Reverse mouse primer: 5' ATC ATG GAA AGC CAA CTT C 3' (19mer)

The reverse mouse primer included the start codon, ATG, underlined, and the forward mouse primer included the stop codon as used in the orginal PCR cloning steps described above. PCR was performed as described above. The ∼1.9 kilobase product was cloned into pTOPO-Blunt and the DNA insert was sequenced as described above. The ∼1.9 kilobase mouse DNA insert was excised from the pTOPO-Blunt vector by digestion with the restriction enzyme *EcoR1*, the restricted fragment was separated by agarose gel electrophoresis, purified using the Qiagen DNA gel extraction kit and ligated into the *EcoR1* pre-digested mammalian vector, pTargeT. Ligation was performed at 12°C overnight in the presence of 10 units of T4 DNA ligase (Promega), and ligated plasmid was transformed into competent E.coli XL-1 Blue (Stratagene), and plasmid DNA purified using Qiagen columns as described above. Restriction enzyme digestion of plasmid DNA revealed the clones containing the correct orientation of the mouse PLC-zeta; PLCζ insert.

### EXAMPLE 3: Transfection of human and mouse expression plasmids into CHO cells

The human and mouse pTargeT/ PLC expression plasmid DNAs prepared as described in Example 2 were separately introduced, by a lipid-mediated transfection procedure, into the Chinese hamster ovary (CHO) cell line grown in tissue culture. CHO cells cultured in serum-containing media, DMEM, (Dulbecco's Modified Eagle Medium) to a density of 500,000 cells per culture dish, were transfected with 40µg plasmid DNA plus 40uL of Lipofectamine2000 (Life Technologies Ltd, 3 Fountain Drive, Inchinnan Business Park, Paisley, U.K.) in serum-free DMEM. After 15 hours, the CHO cells were returned to serum-containing DMEM.

In parallel, control experiments, identical CHO cells were treated in the same way with Lipofectamine but in the absence of plasmid DNA.

### EXAMPLE 4: Demonstration of effectiveness - PLC in CHO Cells

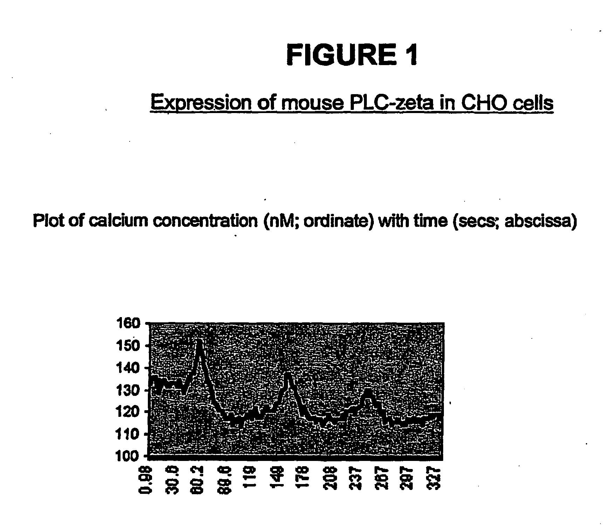

Transfected cells prepared according to Example 3 were washed with culture medium 30 minutes after transfection, then incubated with the calcium-sensitive fluorescent indicator, fura-2-AM for 60 minutes. After further washing with medium, the cells were then placed on a microscope stage and the changes in cell calcium levels, as detected by the fluorescence of the fura-2, were monitored. Only in cells transfected with the PLCζ expression plasmid, the cell calcium level was observed to change periodically. This specific temporal behaviour of cell calcium, *ie* to produce calcium oscillations, is the same as that observed in eggs when fused with sperm at fertilization, and when soluble sperm proteins are injected directly into eggs. Figure 1 demonstrates this with respect to mouse PLCζ. This indicates that the novel PLCζ proteins we have identified in human and mouse testis may be used to specifically control cell calcium levels in mammalian cells.

### EXAMPLE 5: Demonstration of effectiveness - PLC in Oocytes

The open reading frame of human and mouse PLCζ cloned into the pTargeT vector as described in Example 2, were linearised by restriction, and complementary RNA (cRNA) encoding PLCζ was synthesized with a Ribomax RNA synthesis kit (Promega) then re-suspended in 120mM KCl, 20mM HEPES, pH 7.4. Mouse oocytes arrested at MII stage were harvested from female mice and loaded with fura 2-AM for 10 minutes, washed in H-KSOM and placed on a Nikon Diaphot stage. cRNA was micro-injected to 3-5% of egg volume and calcium was monitored as described by Swann, K in Development 110 1295-1302 (1990).

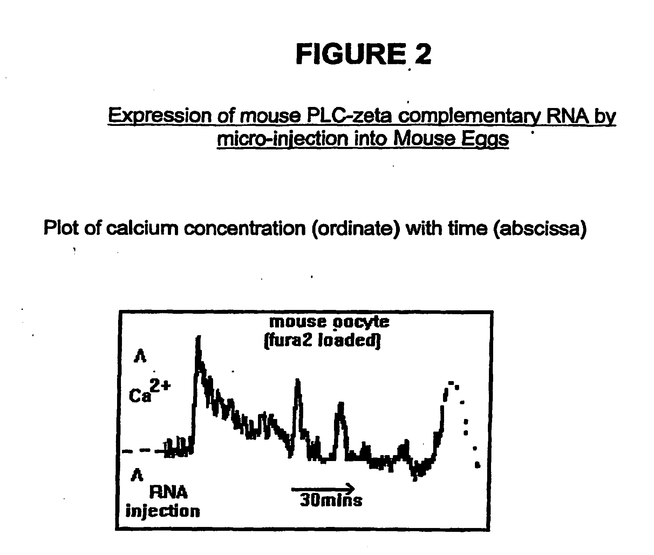

Figure 2 demonstrates that mouse PLCζ in mouse eggs causes calcium oscillations.
The data from the experiments of Examples 4 and 5 clearly show that PLCζ should have the effect of causing calcium oscillations in any cell type.

### EXAMPLE 6: Further Evidence that PLC-Zeta is Sperm Factor

### Complementary RNA synthesis and in vitro translation

The 1941bp open reading frame of mouse PLCζ was cloned into pCR-Blunt II-TOPO, sequenced, and subcloned (pTarget, Promega) to generate pTarget-mPLCζ. Complementary RNA (cRNA) was synthesized from linearised pTarget-mPLCζ (Ribomax RNA synthesis, Promega) in the presence of 3 mM m⁷G(5')ppp(5')G, isopropanol precipitated and resuspended in DEPC-treated water containing 4 U/ul RNasin (Promega). Mutagenesis of ²¹⁰Asp to ²¹⁰Arg in PLCζ to produce ^{D210R}PLCζ was achieved using the QuikChange Site-Directed Mutagenesis Kit (Stratagene). Constructs and cRNAs for rat PLCδ1 and ^{ΔPH}PLC δ1, which encoded the full-length (756 amino acids) and PH domain-deleted PLCδ1 (Δ1-132), respectively, and ^{D210R}PLCζ were produced in pTarget as above. cRNA (2 ug) was expressed *in vitro* (Reticulocyte lysate system, Promega) in the presence of [³⁵S]methionine (Amersham Pharmacia). Radiolabelled protein, analysed by SDS-PAGE and autoradiography, was displayed using QuantityOne software (BioRad).

### c-Myc-Epitope tagging, bacterial expression and PLCζ quantitation

The 1941 bp open reading frame of mouse PLCζ was subcloned into pGBK-T7 (Clontech) with an in-frame c-Myc epitope tag at the 5'-end (Lopez et al J Biol Chem 276 2758-2765 (2001)). The c-Myc-PLCζ was further subcloned into pcDNA3.1 and sequence-verified before cRNA synthesis from the T7 site (Ribomax) for egg micro-injection, as described above. For bacterial expression, c-Myc-PLCζ was subcloned into pBAD (Invitrogen) with an in-frame hexahistidine tag at the 3' end. The c-Myc-PLCζ-Histag protein was produced in 0.2% w/v arabinose-induced, BL21(DE3)pLysS *E.coli,* after extraction of the pelleted bacteria by five freeze-thaw and ultrasonication cycles, then purified by nickel affinity chromatography (ProBond, Invitrogen). Protein quantitation was performed using the BCA protein assay (Pierce) Densitometric analysis of the c-Myc-PLCζ band expressed in eggs micro-injected with different cRNA concentrations, c-Myc-PLCζ-Histag protein purified from *E.coli,* and calibrated sperm extract PLCζ derived from 10⁴-10⁶ mouse sperm, employed a c-Myc monoclonal antibody (1:2000, Santa Cruz Biotechnology) and rabbit anti-PLCζ antiserum (1:1000), respectively, using QuantityOne software (BioRad). A calibration standard plot, from analysis by immunoblot densitometry (Malek et al Biotechniques 6 1150-1153 (1997)) using the c-Myc antibody, was constructed using defined amounts of c-Myc-PLCζ-Histag protein, purified from *E.coli,* to enable the calculation of the relative c-Myc-PLCζ content in batches of 100 micro-injected eggs. For the quantitation analysis, expression of the c-Myc-PLCζ protein was assumed to be linear with time after cRNA micro-injection, as has been shown for micro-injected EGFP cRNA expressed in mouse eggs. This assumption was necessary because the c-Myc-PLCζ protein was below the detection limit within 3 hours of cRNA micro-injection. Hence, for a single mouse egg, the calculated 440-750 fg of c-Myc-PLCζ protein expressed 5 hours after micro-injection with 0.02 mg/ml cRNA, was equivalent to 44-75 fg expressed at 0.5 hours) (the time when the first Ca²⁺ transient is normally observed). A separate calibration plot using the anti-PLCζ antibody was constructed with different c-Myc-PLCζ-Histag protein concentrations to enable estimation of the relative PLCζ content in defined numbers of mouse sperm.

Results are given in sections (a) to (c) below.

### Immunodepletion of PLCζ from sperm extracts

Soluble extracts (Parrington et al Biochem J 341 1-4 (1999)) prepared from hamster sperm were incubated for 1 hour at 4°C with control IgG or anti-PLCζ antibody that had been covalently attached to Protein G beads (1 mg/ml, Seize X Kit, Pierce). The PLCζ content of the supernatant and precipitated beads was determined by immunoblot analysis with anti-PLCζ antibody. Antibody-treated sperm supernatants were also analysed for Ca²⁺ release activity by fluo-3 fluorometry with sea urchin egg homogenates, monitored using a Perkin-Elmer LS50B fluorimeter (as described by Jones et al in FEBS Letts 437 297-300 (1998)). They were also analysed for ability to generate CCOs by micro-injection into mouse eggs, as described below. Maximimal immunodepletion of the sperm PLCζ protein was achieved by using an optimised ratio of antibody beads to sperm extract for each experiment (n=4). The optimal ratio was empirically determined for each sperm extract preparation as the minimum concentration of sperm extract (0.3-0.8 mg/ml) that still retains Ca²⁺ release activity after treatment with the control IgG beads.

Results are given in section (d), below.

### Preparation and handling of gametes

Mouse egg procedures were carried out either in HEPES-buffered KSOM or amino acid supplemented KSOM (Summers et al Human Reprod 15 1791-1801 (2000)). Female MF1 mice were super-ovulated by injection with 5 IU of PMSG followed 48 hours later by HCG (Intervet). Eggs were collected 13.5-14.5 hours after HCG, maintained in 100 µl droplets of H-KSOM under mineral oil at 37°C and cRNA micro-injections performed within 1 hour. Expression of c-Myc-PLCζ in eggs was examined 5 hours after cRNA micro-injection, by adding SDS sample buffer to pelleted eggs and incubating at 95°C for 5 minutes prior to SDS-PAGE, immunoblot then densitometric analysis with the c-Myc monoclonal antibody, as described above. Calibrated mouse sperm pellets were re-suspended in 10 mM Tris-HCl pH 7.5, 15 mM dithiothreitol (Perry et al Biol Reprod 60 747-755 (1999)), then subjected to 5 freeze-thaw cycles in liquid N₂ and centrifuged at 20,000 x g at 4°C for 10 minutes, before densitometric analysis of the soluble extract with PLCζ antibody, as described above. For *in vitro* fertilization studies, sperm were capacitated for 2-3 hours before adding to eggs. Egg activation and development studies were in H-KSOM containing 2 µM cytochalasin D for 4 hours. Further development to 2-cell stage, morula and blastocyst stage was carried out in 50 µl droplets of KSOM under mineral oil at 37°C in a 5% CO₂ incubator.

### Measurement of intracellular Ca²⁺ in MII-arrested mouse eggs

Eggs loaded with 4 µM Fura red-AM (Molecular Probes) for 10 minutes were washed in H-KSOM and placed on a Nikon Diaphot stage. Loading media included sulfinpyrazone to prevent dye compartmentalisation and extrusion (Lawrence et al Development 124 223-241 (1997)). cRNA solutions in 120 mM KCl, 20 mM HEPES, pH 7.4, were micro-injected to 3-5% of egg volume as previously described (Swann 1990, *ibid*, Example 5). Protein synthesis was inhibited in control experiments where eggs were pre-incubated in solution containing 10 µM cycloheximide for 30 minutes prior to micro-injection with PLCζ cRNA (0.02 mg/ml; n=9). Injection volume was estimated from the displacement caused by bolus injection. Ca²⁺ measurements were performed on a CCD-based imaging system as previously described (Lawrence *et al*, 1997; *ibid*), or a Zeiss Axiovert 100 with illumination from a monochromator (Photonics) controlled by MetaFluor v4.0 (Universal Imaging Corp).

### Results

### (a) PLCζ triggers Ca²⁺ oscillations in eggs

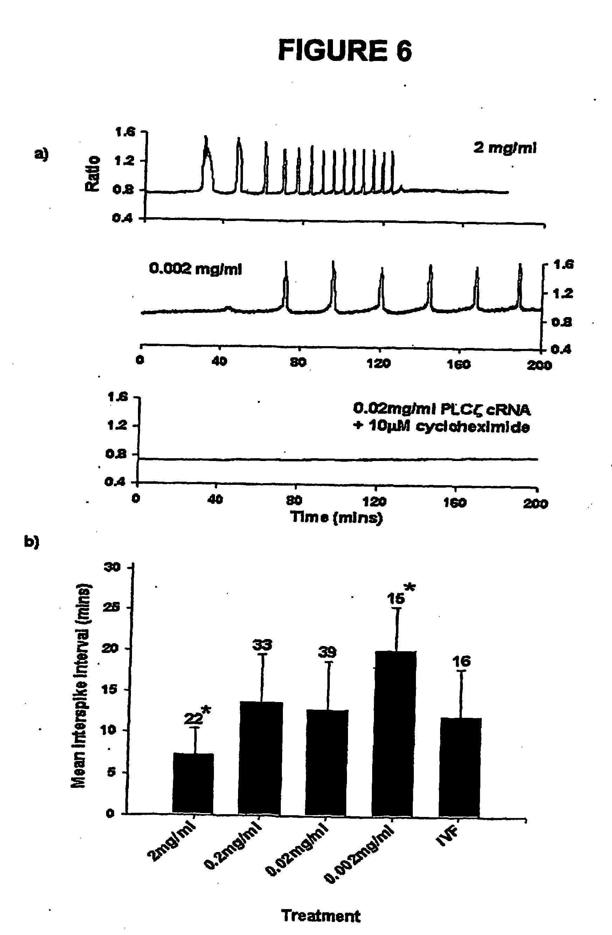

The defining character of the mammalian sperm factor is the ability to elicit CCOs that mimic the fertilization-associated transients displayed by mammalian eggs. To examine whether sperm PLCζ could trigger such CCOs, we introduced PLCζ complementary RNA (cRNA) by micro-injection into MII-arrested mouse eggs, as described previously for spermatogenic cell mRNA. Eggs micro-injected with a pipette concentration of 2 mg/ml PLCζ cRNA, corresponding to <0.1 mg/ml in the egg after a 3-5% injection volume, underwent a prolonged series of CCOs, similar to those shown in Figure 2, that commence within 15-20 minutes. The high oscillation frequency is similar to that observed upon micro-injection of concentrated sperm extracts into mouse eggs. CCOs of similar amplitude, but lower frequency, were obtained with a 1000-fold dilution to 0.002 mg/ml PLCζ cRNA (Figure 6a, middle trace; 0.0001 mg/ml in egg). None of the eggs treated with cycloheximide to block protein synthesis showed any Ca²⁺ transients after PLCζ cRNA-micro-injection (0.02 mg/ml, n=9; Fig. 6a, bottom trace). Robust CCOs were observed in 100% of the eggs micro-injected with the four different PLCζ cRNA concentrations tested, ranging from 0.002-2 mg/ml (Figure 6b). Importantly, the frequency, but not the amplitude, of CCOs varied with PLCζ cRNA concentration, directly matching the same phenomenon observed with different concentrations of sperm extract. The highest pipette concentration used, 2 mg/ml, produced CCOs with a mean interspike interval of 7.3 3.2 minutes (Figure 6b). The lowest pipette concentration of PLCζ cRNA that gives oscillations within 2 hours of injection (0.002 mg/ml), displayed a mean interspike interval of 20.1 ± 5.4 minutes (Figure 6b). Both of these values are significantly different to the mean interspike interval produced with in vitro fertilization (IVF) of mouse eggs (12.1 ± 5.8 minutes). However, the interspike intervals for 0.2 and 0.02 mg/ml PLCζ cRNA (13.6 ± 3.2 and 12.7 ± 60 minutes, respectively) are not significantly different from IVF.

### (b) Fertilization-like Ca²⁺ signals via PLCζ

The CCOs at fertilization display some unique features. The first Ca²⁺ transient invariably lasts longer than subsequent oscillations, and exhibits a set of intriguing, smaller sinusoidal increases on top of the main peak. Micro-injection of a pipette concentration of PLCζ cRNA that produces an interspike interval matching IVF (i.e. 0.02 mg/ml; Figure 6b), resulted not only in the same, longer initial Ca²⁺ transient, but also displayed a similar pattern of smaller sinusoidal increases. The first Ca²⁺ increase after 0.02 mg/ml PLCζ cRNA micro-injection matches the first IVF transient in both average duration (PLCζ 2.8 ± 0.6 minutes, n=39 versus IVF 3.0 ± 0.7 minutes, n=16), and also in reproducibly producing the cluster of smaller Ca²⁺ increases superimposed on the first transient. A concentration of 0.02 mg/ml PLCζ cRNA was used for subsequent micro-injection experiments, unless stated otherwise, to provide the precise Ca²⁺ signaling conditions that are stereotypical of fertilization.

### (c) Physiological level of PLCζ in a single sperm

In order to quantitate the PLCζ expressed in micro-injected eggs, a c-Myc epitope tag was introduced at the N-terminus of PLCζ, as described above. Micro-injected c-Myc-PLCζ cRNA at different concentrations was as effective at generating Ca²⁺ oscillations in eggs as the untagged PLCζ, indicating that the N-terminal attachment of the c-Myc tag is not deleterious to PLCζ activity, as was shown for c-Myc-PLC .
Furthermore, the c-Myc-PLCζ protein expressed in eggs was readily detected in immunoblots using an anti-c-Myc monoclonal antibody, as a single band with the predicted mass of 78 kDa, whereas uninjected eggs exhibited no immunoreactivity. Comparison of the relative mobility of native mouse sperm PLCζ (74 kDa) and recombinant c-Myc-PLCζ protein (78 kDa [74 kDa PLCζ + 4 kDa c-Myc tag]) indicated that the deduced ORF of the PLCζ cDNA clone ([SEQ ID NO: 2], 74 kDa) represents the complete sperm PLCζ sequence. Densitometric analysis of the immunoreactive 78 kDa c-Myc-PLCζ protein expressed in eggs, compared with calibrated amounts of purified recombinant c-Myc-PLCζ protein produced in bacteria, enabled the determination of 44-75 fg/egg (n=4) as the amount of PLCζ protein that triggers Ca²⁺ oscillations using 0.02 mg/ml cRNA. This cRNA concentration is the one that most closely mimics the IVF response, though ten-fold lower levels (i.e. 4-8 fg PLCζ protein/egg using 0.002 mg/ml cRNA) are also able to cause Ca²⁺ oscillations (Figure 6).

The PLCζ content of sperm was also determined by densitometry with a PLCζ polyclonal antibody using a defined number of mouse sperm and compared with calibrated amounts of recombinant PLCζ protein. Using densitometric values within the recombinant PLCζ protein calibration plot, obtained from samples comprising 10⁴-10⁶ mouse sperm, a single mouse sperm was calculated to contain 20-50 fg PLCζ protein (n=4). The level of PLCζ able to produce Ca²⁺ oscillations in a single egg similar to fertilization (4-75 fg, i.e. with 0.002-0.02 mg/ml cRNA) is therefore in the same range as the single sperm content of PLCζ (20-50 fg). The observed quantitative correlation indicates that the PLCζ from a single sperm is sufficient to produce the Ca²⁺ oscillations observed upon sperm-egg fusion.

### (d) Sperm PLCζ depletion abrogates Ca²⁺ oscillations

To address whether the PLCζ in sperm is uniquely responsible for Ca²⁺ mobilisation in eggs, the PLCζ content of sperm extracts was specifically depleted using an anti-PLCζ antibody, as described above. Immunoblot analysis indicated that sperm extract supernatant retains the PLCζ protein after control antibody treatment, in contrast to PLCζ antibody-treated supernatant where the PLCζ is absent. Analysis of the corresponding precipitated antibody samples revealed that the sperm PLCζ is effectively removed by PLCζ antibody, but not by the control antibody. Assessment of Ca²⁺ release activity in antibody-treated sperm extracts using sea urchin egg homogenate assays showed that PLCζ-depleted samples lack any Ca²⁺ mobilising activity, whereas a robust Ca²⁺ release was observed with the control antibody-treated sperm extract containing PLCζ protein. Moreover, micro-injection of antibody-treated sperm extracts into mouse eggs illustrated that the ability of untreated samples to generate IVF-like Ca²⁺ oscillations is fully preserved in control antibody-treated samples, while PLCζ-depletion effectively abrogates Ca²⁺ release activity.

These PLCζ antibody depletion experiments (n=4) suggest that PLCζ is the sole component of sperm extracts possessing the ability to cause Ca²⁺ release in mouse eggs. Taken together with evidence that the PLCζ level in a single mouse sperm is sufficient to trigger IVF-like Ca²⁺ oscillations in a single mouse egg, the immunodepletion data provides compelling evidence that PLCζ is synonymous with the previously described mammalian sperm factor.

### (e) PLCζ activates normal embryo development

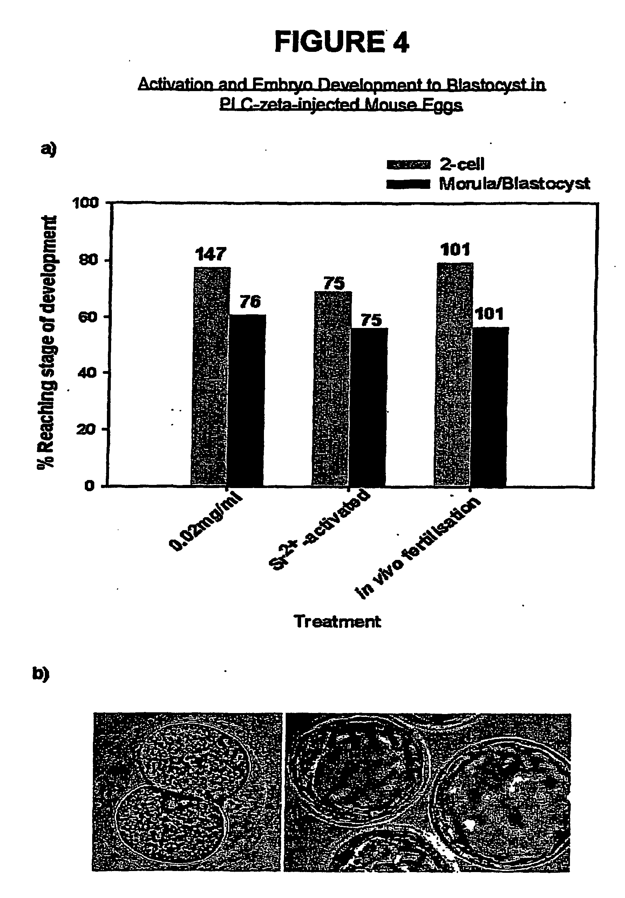

Since eggs that were micro-injected with PLCζ cRNA (0.02mg/ml) displayed all the properties of Ca²⁺ oscillations indistinguishable from those of IVF (Results (a) and (b) above) and is equivalent to the PLCζ content of a single sperm ((c) above), their ongoing development was monitored for several days after PLCζ-micro-injection.
PLCζ-micro-injected eggs underwent activation (Figure 4a) because normal development proceeded to the 2-cell stage within 24 hours (78%, n=147), and many reached the morula or blastocyst stages by 4-5 days (62%, n=76). None of the eggs micro-injected with buffer control reached the 2-cell stage, indicating activation as an artefact of micro-injection procedure did not occur. The proportion of PLCζ-induced embryos that developed to either the 2-cell, or morula and blastocyst stages, was the same as for eggs that are either parthenogenetically activated by strontium ions (n=75), or when embryos are collected at the 1-cell stage from female mice after in vivo fertilization (n=101) upon mating with males (Figure 4a).

Photomicrographs taken at 24 hours and 5 days after PLCζ-micro-injection into mouse eggs show the appearance of normal embryo development to the 2-cell stage and blastocyst stage (left and right panel, respectively, Figure 4b). There were no morphological differences to embryos obtained after fertilization with sperm. Thus, after inducing Ca²⁺ oscillations in the egg, sperm PLCζ-micro-injection also triggered the entire cascade of events required for activation and embryo development, in the same manner as sperm at fertilization.

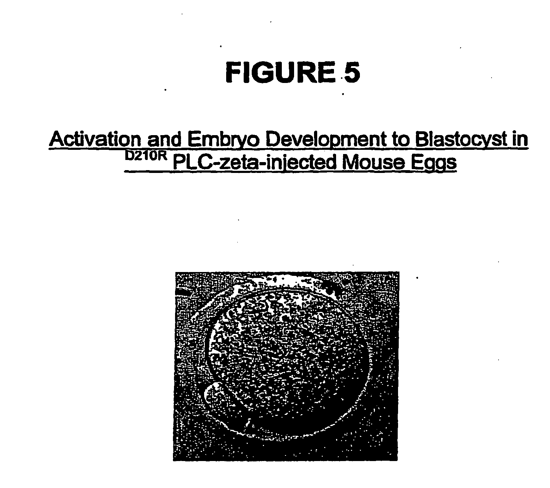

The possibility remained that a novel action of PLCζ other than PIP₂ hydrolysis is responsible for egg activation, such as a protein-protein interaction with a distinct egg molecule. To test whether an enzymatically active PLCζ is required for egg activation and embryo development, the ^{D210R}PLCζ cRNA (0.02mg/ml), which was shown to be defective in triggering Ca²⁺ oscillations, was micro-injected, and egg activation assessed after 24 hours. None of the ^{D210R}PLCζ cRNA-micro-injected eggs were found to proceed to the pronuclear or 2-cell stage (Figure 5, n=20), suggesting that the enzymatic function of sperm PLCζ is critical for egg activation.

### Human PLC-zeta triggers Ca oscillations in mouse oocytes

To examine the ability of hPLC-zeta to cause Ca2+ changes, cRNA for hPLC-zeta was microinjected into MII-arrested mouse oocytes with a pipette concentration of 20 µg/ml hPLC-zeta cRNA, which corresponds to 0.001 mg/ml in the oocyte after a 3-5% injection volume. Figure 8A shows a representative example Ca2+ recording for each of the four different concentrations ofhPLC-zeta cRNA that were microinjected. At 20 µg/ml hPLC-zeta cRNA triggered high frequency Ca2+ oscillations within 10-15 minutes of microinjection (mean interspike interval: 4.21 ± 1.79mins). As was observed with mouse PLC-zeta cRNA and hamster sperm extract microinjection, Ca2+ oscillations of lower frequency were obtained with lower concentrations of stimulus (Swann, 1990; Saunders et al, 2002). It was notable that even at pipette concentrations of 0.02 µg/ml, hPLC-zeta cRNA could still induce Ca2+ oscillations within two hours of microinjection. Although a wide range of cRNA concentrations from 20-0.02 µg/ml were used, the Ca2+ oscillations observed at each concentration lasted for a similar period of 3-4 hours (Fig. 8A). The mean interspike interval data showing the dose-response relationship with hPLC-zeta cRNA is summarised in the histogram in Figure 8B.

### Embryo development with hPLC-zeta

The microinjection of 20 µg/ml mPLC-zeta into mouse oocytes was previously demonstrated to induce Ca2+ oscillations and development to the blastocyst stage at rates comparable to that of *in vitro* fertilization. To examine if hPLC-zeta is also able to support development, and what effect the oscillation frequency might have on embryo development, MII-arrested oocytes were injected with 20, 2.0 and 0.2 µg/ml hPLC-zeta cRNA and monitored after 24h and 96h. All three concentrations were effective at activating the oocytes and enabling development to the 2-cell stage (Fig. 9). Using 2.0 and 0.2 µg/ml hPLC-zeta cRNA, mouse embryo development to morula/blastocyst was 33.3 and 38.9%, respectively (Fig. 9A). This compares with developmental rates with *in vivo* fertilization and parthenogenetic activation of 55-60% under our conditions using outbred mouse strains. It was conspicuous, however, that the high Ca2+ oscillation frequency (low mean interspike interval) produced with 20 µg/ml was ineffective at supporting development to morula/blastocyst stages (1.8% of oocytes reaching morula/blastocyst) and most of these embryos arrested at the 2-cell stage.
Micrographs of the mouse embryos produced by hPLC-zeta cRNA microinjection show they are morphologically similar to those following *in vitro* fertilization (Fig. 9B), analogous to the observations with mPLC-zeta , though blastocyst cell numbers have not been determined. These data suggest that microinjection of hPLC-zeta cRNA into unfertilized eggs alone can trigger early embryonic development to blastocyst stages in mouse embryos, but it appears that the high frequency of Ca2+ oscillations caused by the higher doses of hPLC-zeta is detrimental to development beyond the 2-cell stage.

### Simian PLC-zeta triggers Ca2+ oscillations in mouse oocytes.

The observations described above (Figs. 8 and 9), show that the human and mouse PLC-zeta can cause fertilization-like Ca2+ oscillations that initiates activation and development of mouse oocytes. The identification of two related, testis-specific cDNA sequences of 2.3 kb from *M*.
*fascicularis*, and the high degree of similarity of their ORF with the human and mouse PLC-zeta, enabled the prediction that these were simian PLC-zeta homologues. We therefore compared the ability of cRNA prepared from the two forms of sPLC-zeta, designated s1PLC-zeta and s2PLC-zeta (AB070108 and AB070109, respectively), to generate Ca2+ oscillations in mouse oocytes. Both forms were able to trigger Ca2+ oscillations and no functional difference was detected upon microinjecting either s1PLC-zeta or s2PLC-zeta cRNA (data not snown). For all subsequent experiments s1PLC-zetawas used (AB070108). Figure 10A shows that s1PLC-zeta cRNA triggered dose-dependent Ca2+ oscillations in mouse oocytes comparable to those seen with human and mouse PLC-zeta, at each of the three doses tested (0.2, 0.02, 0.002 mg/ml). Similar to the data with human PLC-zeta, (Fig. 8A), the period over which Ca2+ oscillations occurred was 3-4 hours for each of the three s1PLC-zeta cRNA concentrations microinjected. However, the frequency of Ca2+ spikes was different for each cRNA concentration, with the mean interspike interval decreasing with higher level of the stimulus (Fig. 10B). This data suggests that PLC-zeta, derived from the sperm/testis of various mammals lacks any species-specificity and, once introduced by microinjection, is able to trigger Ca2+ oscillations in heterologous mammalian oocytes. This finding is fully consistent with earlier observations of sperm extracts derived from various sources, including non-mammalian species, each causing Ca2+ oscillations in different mammalian oocytes. Figure 11 compares the mean interspike intervals for the three different mammalian forms of PLC-zeta at various pipette cRNA concentrations. Microinjecting cRNA for mPLC-zeta, hPLC-zeta and sPLC-zeta all gave rise to Ca2+ oscillations over a range of concentrations from 200 to 2 µg/ml. However, hPLC-zeta was distinct in being able to cause Ca2+ oscillations at the lower concentrations of 0.2-0.02 µg/ml (Fig 11). This suggests that under the same experimental conditions, the human form of PLC-zeta is more effective at generating Ca2+ oscillations in mouse oocytes than the PLC-zeta from mouse and monkey.

In addition to demonstrating that hPLC-zeta and sPLC-zeta are able to cause Ca2+oscillations in mouse oocytes (Figs. 8 and 10), we obtained empirical evidence that hPLC-zeta is more effective at causing Ca2+ oscillations than sPLC-zeta and mPLC-zeta (Fig. 11). The minimal amount of hPLC-zeta cRNA required to trigger Ca2+ oscillations was 1-2 orders of magnitude lower (0.2-0.02 _g/ml ) than the minimally effective dose of mouse or simian PLC-zeta cRNA (2 _g/ml ). These differences were observed as a consistent feature with different batches of cRNA that were each tested for expression *in vitro* (data not shown). The superior potency of hPLC-zeta cRNA is therefore likely to represent a genuine feature of the hPLC-zeta protein. Thus, we could predict that there is at least an order of magnitude difference in the sensitivity of mouse oocytes to hPLC-zeta compared with mPLC-zeta. It is not clear why hPLC-zeta exhibits greater virility than mPLC-zeta or sPLC-zeta. Subsequent to stimulating Ca2+ oscillations in mouse oocytes, the human PLC-zeta was also able to trigger development of embryos to the blastocyst stage (Fig. 8). This suggests that hPLC-zeta is able to produce all of the normal events of oocyte activation. However, one feature of the greater efficacy of hPLC-zeta is that high cRNA levels caused very high frequency Ca2+ oscillations in mouse oocytes (Fig. 8A, top trace). At concentrations of cRNA that resulted in Ca2+ oscillations of ∼1 spike every 5 minutes, hPLC-zeta was able to effect oocyte activation, but the embryos arrested at the 2-cell stage (Fig. 9A). Previous studies have shown that high frequency Ca2+ oscillations may either lead to apoptosis of oocytes, or to developmental changes in postimplantation embryos. Our data provides the first indication that high frequency Ca2+ oscillations can also activate an oocyte, but this non-physiological stimulus leads to arrest during the early cleavage stages.

In conclusion, we have herein disclosed and characterized the zeta isoform of phospholipase C and so elucidated a key trigger in the fertilization process.

**Table 2.**

| Genomic organization of the human *plc-zeta* gene | | | |
|---|---|---|---|
| Sequence coordinates and length of exons and introns comprising the human *plc-zeta* gene localised to chromosome 12p12.3 | | | |
| **Chromosome 12 coordinates** | **Exon Number** | **Intron Number** | **Length (basepairs)** |
| | | | |
| 4443338 - 4443286 | 1 | | 53 |
| 4443285 - 4442862 | | 1 | 424 |
| 4442861 - 4442712 | 2 | | 150 |
| 4442711 - 4441697 | | 2 | 1015 |
| 4441696 - 4441564 | 3 | | 133 |
| 4441563 - 4428887 | | 3 | 12677 |
| 4428886 - 4428667 | 4 | | 220 |
| 4428666 - 4424987 | | 4 | 3680 |
| 4424986 - 4424784 | 5 | | 203 |
| 4424783 - 4418341 | | 5 | 6443 |
| 4418340 - 4418195 | 6 | | 146 |
| 4418194 - 4410669 | | 6 | 7526 |
| 4410668 - 4410519 | 7 | | 150 |
| 4410518 - 4407131 | | 7 | 3388 |
| 4407130 - 4407045 | 8 | | 86 |
| 4407044 - 4406922 | | 8 | 123 |
| 4406921 - 4406853 | 9 | | 69 |
| 4406852 - 4405305 | | 9 | 1548 |
| 4405304 - 4405151 | 10 | | 154 |
| 4405150 - 4401621 | | 10 | 3530 |
| 4401620 - 4401503 | 11 | | 118 |
| 4401502 - 4400434 | | 11 | 1069 |
| 4400433 - 4400263 | 12 | | 171 |
| 4400262 - 4393572 | | 12 | 6691 |
| 4393571 - 4393443 | 13 | | 129 |
| 4393442 - 4389634 | | 13 | 3809 |
| 4389633 - 4389484 | 14 | | 150 |
| 4389483 - 4388679 | | 14 | 805 |
| 4388678 - 4388535 | 15 | | 144 |

SEQ ID NO:1 - Human PLC-zeta; PLCζ amino acid sequence, 608 residues

MEMRWFLSKIQDDFRGGKINLEKTQRLLEKLDIRCSYIHVKQIFKDNDRLKQGRITIEEFRA
IYRIITHREEIIEIFNTYSENRKILLASNLAQFLTQEQYAAEMSKAIAFEIIQKYEPIEEVRKAH
QMSLEGFTRYMDSRECLLFKNECRKVYQDMTHPLNDYFISSSHNTYLVSDQLLGPSDLWG
YVSALVKGCRCLEIDCWDGAQNEPVVYHGYTLTSKLLFKTVIQAIHKYAFMTSDYPVVLS
LENHCSTAQQEVMADNLQATFGESLLSDMLDDFPDTLPSPEALKFKILVKNKKIGTLKETH
ERKGSDKRGDNQDKETGVKKLPGVMLFKKKKTRKLKIALALSDLVIYTKAEKFKSFQHSR
LYQQFNENNSIGETQARKLSKLRVHEFIFHTRKFITRIYPKATRADSSNFNPQEFWNIGCQM
VALNFQTPGLPMDLQNGKFLDNGGSGYILKPHFLRESKSYFNPSNIKEGMPITLTIRLISGIQ
LPLTHSSSNKGDSLVIIEVFGVPNDQMKQQTRVIKKNAFSPRWNETFTFIIHVPELALIRFVV
EGQGLIAGNEFLGQYTLPLLCMNKGYRRIPLFSRMGESLEPASLFVYVWYVR

SEQ ID NO:2 - Mouse PLC-zeta; PLCζ amino acid sequence, total 647 residues

MESQLHELAEARWFLSKVQDDFRGGKINVEITHKLLEKLDFPCHFAHVKHIFKENDRQNQ
GRITIEEFRAIYRCIVHREEITEIFNTYTENRKILSENSLIEFLTQEQYEMEIDHSDSVEIINKYE
PIEEVKGERQMSIEGFARYMFSSECLLFKENCKTVYQDMNHPLSDYFISSSHNTYLISDQIL
GPSDIWGYVSALVKGCRCLEIDCWDGSQNEPIVYHGYTFTSKLLFKTVVQAINKYAFVTSD
YPVVLSLENHCSPGQQEVMASILQSTFGDFLLSDMLEEFPDTLPSPEALKFKILVKNRKVGT
LSETHERIGTDKSGQVLEWKEVIYEDGDEDSGMDPETWDVFLSRIKEEREADPSTLSGIAG
VKKRKRKMKIAMALSDLVIYTKAEKFRNFQYSRVYQQFNETNSIGESRARKLSKLRVHEFI
FHTAAFITRVYPKMMRADSSNFNPQEFWNVGCQMVALNFQTPGLPMDLQNGKFLDNGGS
GYILKPDILRDTTLGFNPNEPEYDDHPVTLTIRIISGIQLPVSSSSNTPDIVVIIEVYGVPNDHV
KQQTRVVKNNAFSPKWNETFTFLIQVPELALIRFVVETQQGLLSGNELLGQYTLPVLCMNK
GYRRVPLFSKSGANLEPSSLFIYVWYFRE

SEQ ID NO:3 - Human PLC-zeta; PLCζ nucleotide sequence, 1827 nucleotides

ATGGAAATGAGATGGTTTTTGTCAAAGATTCAGGATGACTTCAGAGGTGGAAAAATTA
ACCTAGAAAAAACTCAGAGGTTACTTGAAAAATTAGATATTCGGTGCAGTTATATTCA
TGTGAAACAGATTTTTAAGGACAATGACAGGCTGAAACAAGGAAGAATCACCATAGA
AGAATTTAGAGCAATTTATCGAATTATCACGCACAGAGAAGAAATTATTGAGATTTTC
AACACATATTCTGAAAACCGGAAAATTCTTTTAGCAAGTAATCTGGCTCAATTTCTGAC
ACAAGAACAATATGCAGCTGAGATGAGTAAAGCTATTGCTTTTGAGATCATTCAGAAA
TACGAGCCTATCGAAGAAGTTAGGAAAGCACACCAAATGTCATTAGAAGGTTTTACAA
GATACATGGATTCACGTGAATGTCTACTGTTTAAAAATGAATGTAGAAAAGTTTATCA
AGATATGACTCATCCATTAAATGATTATTTTATTTCATCTTCACATAACACATATTTGGT
ATCTGATCAATTATTGGGACCAAGTGACCTTTGGGGATATGTAAGTGCCCTTGTGAAA
GGATGCCGTTGTTTGGAGATTGACTGCTGGGATGGAGCACAAAATGAACCTGTTGT[?]T
ATCATGGCTACACACTCACAAGCAAACTTCTGTTTAAAACTGTTATCCAAGCTATACAC
AAGTATGCATTCATGACATCTGACTACCCAGTGGTGCTCTCTTTAGAAAATCACTGCTC
CACTGCCCAACAAGAAGTAATGGCAGACAATTTGCAGGCTACTTTTGGAGAGTCCTTG
CTTTCTGATATGCTTGATGATTTTCCTGATACTCTACCATCACCAGAGGCACTAAAATT
CAAAATATTAGTTAAAAATAAGAAAATAGGAACCTTAAAGGAAACCCATGAAAGAAA
AGGTTCTGATAAGCGTGGAGACAATCAAGACAAGGAAACAGGGGTAAAAAAGTTACC
TGGAGTAATGCTTTTCAAGAAAAAGAAGACCAGGAAGCTAAAAATTGCTCTGGCCTTA
TCTGATCTTGTCATTTATACGAAAGCTGAGAAATTCAAAAGCTTTCAACATTCAAGATT
ATATCAGCAATTTAATGAAAATAATTCTATTGGGGAGACACAAGCCCGAAAACTTTCA
AAATTGCGAGTCCATGAGTTTATTTTTCACACCAGGAAGTTCATTACCAGAATATATCC
CAAAGCAACAAGAGCAGACTCTTCTAATTTTAATCCCCAAGAATTTTGGAATATAGGT
TGTCAAATGGTGGCTTTAAATTTCCAGACCCCTGGTCTGCCCATGGATCTGCAAAATGG
GAAATTTTTGGATAATGGTGGTTCTGGATATATTTTGAAACCACATTTCTTAAGAGAGA
GTAAATCATACTTTAACCCAAGTAACATAAAAGAGGGTATGCCAATTACACTTACAAT
AAGGCTCATCAGTGGTATCCAGTTGCCTCTTACTCATTCATCATCTAACAAAGGTGATT
CATTAGTAATTATAGAAGTTTTTGGTGTTCCAAATGATCAAATGAAGCAGCAGACTCG
TGTAATTAAAAAAAATGCTTTTAGTCCAAGATGGAATGAAACATTCACATTTATTATTC
ATGTCCCAGAATTGGCATTGATACGTTTTGTTGTTGAAGGTCAAGGTTTAATAGCAGGA
AATGAATTTCTTGGGCAATATACTTTGCCACTTCTATGCATGAACAAAGGTTATCGTCG
TATTCCTCTGTTTTCCAGAATGGGTGAGAGCCTTGAGCCTGCTTCACTGTTTGTTTATGT
TTGGTACGTCAGATAA

SEQ ID NO: 4 - Mouse - PLC-zeta; PLCζ nucleotide sequence, 1944 nucleotides

ATGGAAAGCCAACTTCATGAGCTCGCAGAAGCAAGATGGTTTTTGTCAAAGGTTCAGG
ATGATTTTAGAGGTGGAAAAATCAACGTTGAAATTACTCACAAACTGCTTGAGAAACT
TGATTTCCCATGCCACTTTGCTCATGTGAAACATATTTTTAAGGAAAATGACAGACAGA
ACCAAGGAAGAATCACCATTGAAGAGTTTAGAGCCATTTACCGGTGTATTGTACATAG
AGAAGAAATCACGGAGATTTTCAACACGTATACTGAAAATAGGAAAATTCTTTCTGAG
AACAGTCTGATTGAGTTTCTAACCCAAGAGCAGTATGAAATGGAGATCGATCACTCTG
ATTCAGTAGAGATCATCAATAAGTATGAGCCTATTGAAGAAGTAAAGGGTGAGCGAC
AGATGTCAATTGAAGGTTTCGCAAGATACATGTTTTCATCAGAATGTCTACTGTTTAAA
GAGAACTGTAAAACCGTGTACCAAGATATGAATCATCCATTAAGTGATTATTTTATTTC
ATCATCTCACAACACATATTTGATATCCGATCAAATATTGGGACCGAGTGACATTTGG
GGATATGTAAGTGCTCTTGTGAAAGGCTGCCGCTGTCTGGAAATTGACTGCTGGATG
GATCCCAAAATGAGCCCATTGTGTACCATGGTTACACATTCACCAGCAAGCTTCTCTTC
AAAACTGTGGTCCAAGCAATAAACAAGTATGCCTTTGTGACATCTGATTACCCAGTAG
TGCTGTCCTTAGAAAATCACTGCTCCCCTGGTCAGCAGGAAGTGATGGCTAGCATTCTG
CAGAGCACCTTTGGAGACTTCCTGCTTTCGGACATGCTTGAGGAGTTTCCAGATACACT
ACCGTCTCCAGAGGCACTAAAATTCAAAATATTAGTGAAAAACAGGAAAGTGGGAAC
CTTATCTGAAACCCACGAGAGGATAGGAACCGACAAAAGTGGCCAAGTGCTAGAATG
GAAAGAAGTCATCTATGAAGATGGTGATGAAGACTCAGGAATGGATCCAGAAACATG
GGATGTCTTCCTATCACGGATCAAGGAGGAGAGGGAAGCAGATCCCTCGACATTGAGT
GGAATAGCAGGCGTCAAGAAAAGGAAGAGGAAGATGAAAATAGCCATGGCCTTATCT
GATCTTGTCATTTATACTAAGGCTGAGAAGTTCCGAAACTTCCAATATTCAAGAGTCTA
TCAGCAATTTAATGAGACCAATTCGATTGGAGAGTCTCGAGCTCGAAAACTTTCCAAA
TTGAGAGTCCATGAGTTTATTTTCCACACCGCGGCATTCATCACCAGAGTATACCCCAA
AATGATGAGAGCAGACTCTTCTAACTTTAACCCTCAAGAGTTTTGGAATGTAGGATGT
CAGATGGTGGCCTTGAACTTTCAAACCCCTGGACTGCCTATGGATTTGCAAAACGGGA
AATTTTTGGATAATGGAGGCTCTGGATATATTTTGAAGCCAGACATCCTTAGAGATAC
AACCCTGGGCTTTAACCCAAATGAACCAGAATATGACGACCATCCAGTTACCCTCACA
ATCCGAATCATCAGTGGGATCCAGTTGCCTGTTAGCTCATCCTCTAACACGCCTGACAT
AGTAGTGATCATAGAAGTCTACGGTGTTCCAAACGACCACGTGAAGCAGCAGACTCGT
GTTGTTAAGAATAATGCTTTTAGTCCAAAGTGGAATGAAACATTTACATTTCTTATTCA
AGTGCCAGAACTGGCATTGATACGTTTTGTTGTTGAAACTCAACAAGGCTTATTATCAG
GAAATGAATTACTCGGGCAGTACACTTTACCAGTTCTTTGCATGAACAAAGGTTATCGT

CGTGTTCCTCTGTTTTCCAAATCCGGTGCGAACCTTGAACCTTCCTCACTGTTTATTTAT
GTTTGGTACTTCAGAGAGTGA

SEQ ID NO: 5 - Mouse PLC-zeta nucleotide sequence, including untranslated region
(Genbank Accession No AF 35950) (2187 basepairs)

CCCCCCCGGCAAGCCATGCCAACTGTGAAGGTTTTAAGAACTTAGCTTCTGGACAAGT
TTTCTTCGAAAGTGAAAAGCAGTAGCAGCGAGAACAGCTGATGACGGTCACAAAAAG
ACAGTGTTACTTCTAAGACAAGTGACACCTTAGACGAAGAGCCCTCTATGGGAGGACA
AGCGGCCCAGATCATGAAATCATGGAAAGCCAACTTCATGAGCTCGCAGAAGCAAGA
TGGTTTTTGTCAAAGGTTCAGGATGATTTTAGAGGTGGAAAAATCAACGTTGAAATTA
CTCACAAACTGCTTGAGAAACTTGATTTCCCATGCCACTTTGCTCATGTGAAACATATT
TTTAAGGAAAATGACAGACAGAACCAAGGAAGAATCACCATTGAAGAGTTTAGAGCC
ATTTACCGGTGTATTGTACATAGAGAAGAAATCACGGAGATTTTCAACACGTATACTG
AAAATAGGAAAATTCTTTCTGAGAACAGTCTGATTGAGTTTCTAACCCAAGAGCAGTA
TGAAATGGAGATCGATCACTCTGATTCAGTAGAGATCATCAATAAGTATGAGCCTATT
GAAGAAGTAAAGGGTGAGCGACAGATGTCAATTGAAGGTTTCGCAAGATACATGTTTT
CATCAGAATGTCTACTGTTTAAAGAGAACTGTAAAACCGTGTACCAAGATATGAATCA
TCCATTAAGTGATTATTTTATTTCATCATCTCACAACACATATTTGATATCCGATCAAAT
ATTGGGACCGAGTGACATTTGGGGATATGTAAGTGCTCTTGTGAAAGGCTGCCGCTGT
CTGGAAATTGACTGCTGGGATGGATCCCAAAATGAGCCCATTGTGTACCATGGTTACA
CATTCACCAGCAAGCTTCTCTTCAAAACTGTGGTCCAAGCAATAAACAAGTATGCCTTT
GTGACATCTGATTACCCAGTAGTGCTGTCCTTAGAAAATCACTGCTCCCCTGGTCAGCA
GGAAGTGATGGCTAGCATTCTGCAGAGCACCTTTGGAGACTTCCTGCTTTCGGACATG
CTTGAGGAGTTTCCAGATACACTACCGTCTCCAGAGGCACTAAAATTCAAAATATTAG
TGAAAAACAGGAAAGTGGGAACCTTATCTGAAACCCACGAGAGGATAGGAACCGACA
AAAGTGGCCAAGTGCTAGAATGGAAAGAAGTCATCTATGAAGATGGTGATGAAGACT
CAGGAATGGATCCAGAAACATGGGATGTCTTCCTATCACGGATCAAGGAGGAGAGGG
AAGCAGATCCCTCGACATTGAGTGGAATAGCAGGCGTCAAGAAAAGGAAGAGGAAGA
TGAAAATAGCCATGGCCTTATCTGATCTTGTCATTTATACTAAGGCTGAGAAGTTCCGA
AACTTCCAATATTCAAGAGTCTATCAGCAATTTAATGAGACCAATTCGATTGGAGAGT
CTCGAGCTCGAAAACTTTCCAAATTGAGAGTCCATGAGTTTATTTTCCACACCGCGGCA
TTCATCACCAGAGTATACCCCAAAATGATGAGAGCAGACTCTTCTAACTTTAACCCTCA
AGAGTTTTGGAATGTAGGATGTCAGATGGTGGCCTTGAACTTTCAAACCCCTGGACTG
CCTATGGATTTGCAAAACGGGAAATTTTTGGATAATGGAGGCTCTGGATATATTTTGA
AGCCAGACATCCTTAGAGATACAACCCTGGGCTTTAACCCAAATGAACCAGAATATGA
CGACCATCCAGTTACCCTCACAATCCGAATCATCAGTGGGATCCAGTTGCCTGTTAGCT
CATCCTCTAACACGCCTGACATAGTAGTGATCATAGAAGTCTACGGTGTTCCAAACGA
CCACGTGAAGCAGCAGACTCGTGTTGTTAAGAATAATGCTTTTAGTCCAAAGTGGAAT
GAAACATTTACATTTCTTATTCAAGTGCCAGAACTGGCATTGATACGTTTTGTTGTTGA
AACTCAACAAGGCTTATTATCAGGAAATGAATTACTCGGGCAGTACACTTTACCAGTT
CTTTGCATGAACAAAGGTTATCGTCGTGTTCCTCTGTTTTCCAAATCCGGTGCGAACCT
TGAACCTTCCTCACTGTTTATTTATGTTTGGTACTTCAGAGAGTGACGCATGTAACTGA
CGCGTTAGCTACACATCACAGTAAACAGTCCAAAG

SEQ ID NO: 6 - Monkey PLC-zeta Accession number AB070108 Nucleotide sequence of 1923 bases encoding 641 amino acids

ATGGAAATGAAATGGTTTTTGTCGAAGATTCAGGATGACTTTAGAGGTGGAAAAATTA
ATCTAGAAAAAACTCAGAGGTTACTTGAAAAATTAGATATTCGGTGCAGTTATATTCA
TGTGAAACGGATATTTAAGGACAATGACAGGCTGAAACAAGGAAGAATCACCATAGA
AGAATTTAGAGCAATTTATCGAATTCTCACGCACAGAGAAGAAATTGTTGAGATTTTC
AACGCATATTCTGAAAACCGGAAAATTCTTTTAGAAAATAATCTGGTTCAATTTCTGAC
ACAAGAACAATATACAACTGAGATGAGTAAAACTATTGCTTTTGAGATCATTCAGAAA
TATGAACCTATCGAAGAAGTTAGGAAAGCACGCCAAATGTCATTAGAAGGTTTTACAA
GATATATGGATTCACGTGAATGTCAACTATTTAAAAATGAATGTAGAAAAGTTTATCA
AGATATGACTCATCCATTAAATGATTATTTTATTTCATCTTCACATAACACATATTTGGT
ATCTGATCAATTAGTGGGACCAAGTGACCTTTGGGGATATGTAAGTGCCCTTGTGAAA
GGATGCCGTTGTTTGGAGATTGATTGCTGGGATGGAGCACAAAATGAACCTGTTGTAT
ATCATGGCTACACACTCACCAGCAAACTTCTGTTTAAAACTGTTATCCAAGCTATACAC
AAGTACGCATTCATGACATCTGACTACCCAGTGGTGCTCTCTTTGGAAAATCACTGCTC
CCCTGCCCAGCAAGAAATAATGGCAGACAATTTGCAGACGACTTTTGGAGAGTCCTTG
CTTTCTGATATGCTTGCTGATTTTCCTGATACTCTACCATCACCAGAGGCACTAAAATT
CAAAGT[?]ATTAGTTAAAAATAAGAAAATAGGAA[?]CTTAAAGGAAACCCATGAAAGAA[?]
AGGTTCTGATAAGCGTGGTAAGGTGGAGGAATGGGAAGAAGAAGTGGCAGATCTGGA
GGAGGAGGAGGAGGAGGAGAGATTCAAAGAATCAGAAATATTCGAATCTGTTTTAGG
AGAAAATCAAGACAAGGAAACAGGGGTAAAAAAGTTATCTGGAGTAACGCTTTTCAA
GAAAAAGAAGACCAGGAAGCTAAAAATTGCTCTGGCCTTATCTGATCTTGTCATTTAT
ACTAAAGCTGAGAAGTTCAAAAGCTTTCAACATTCAAGATTATATCAGCAATTTAATG
AAAATAATTCTATTGGGGAGACACAAGCCCGAAAACTTTCAAAATTGAGAGCCCATGA
GTTTATTTTTCACACCAGGAAGTTCATTACCAGAATATATCCCAAAGCAACAAGAGCA
GACTCTTCTAATTTTAATCCCCAAGAATTTTGGAATATAGGTTGTCAAATGGTGGCTTT
AAATTTCCAGACCCCTGGTCTGCCTATGGATCTGCAAAATGGGAAATTTTTGGATAATG
GTGGTTCTGGATATATTTTGAAACCACATTTCTTAAGAGAGAGTGAATCATACTTTAAC
CCAAGTGACATAAAAGACAGTATGCCAATTACACTTACAATAAGGCTCATCAGTGGTA
TCCAGTTGCCTCTTACTCATTCATCATCTAACAAAGGTGATACATTAGTAATTATAGAA
GTTTTTGGTGTTCCAAATGATCAAATGAAGCAGCAGACTCGTGTAATTAAAAAAAATG
CTTTTAGTCCAAGATGGAATGAAACATTTACATTTATTATTCATGTCCCAGAATTGGCA
TTGATACGTTTTGTTGTTGAAAGTCAAGGTTTAATAGCAGGAAATGAATTTCTTGGGCA
ATATACTTTGCCACTTCTATGCATGAACAAAGGCTATCGTCGTGTTCCTCTGTTTTCCCG
AATGGGTGAGAGCCTTGAGCCTGCTTCACTGTTTGTTTATGTTTGGTACGTCAGA

SEQ ID NO: 7 - Monkey PLC-zeta Accession number AB070108 Protein sequence of 641
amino acids

MEMKWFLSKIQDDFRGGKINLEKTQRLLEKLDIRCSYIHVKRIFKDNDRLKQGRITIEEFRA
IYRILTHREEIVEIFNAYSENRKILLENNLVQFLTQEQYTTEMSKTIAFEIIQKYEPIEEVRKAR
QMSLEGFTRYMDSRECQLFKNECRKVYQDMTHPLNDYFISSSHNTYLVSDQLVGPSDLW
GYVSALVKGCRCLEIDCWDGAQNEPVVYHGYTLTSKLLFKTVIQAIHKYAFMTSDYPVVL
SLENHCSPAQQEIMADNLQTTFGESLLSDMLADFPDTLPSPEALKFKVLVKNKKIGTLKET
HERKGSDKRGKVEEWEEEVADLEEEEEEEEKFKESEIFESVLGENQDKETGVKKLSGVTLF
KKKKTRKLKIALALSDLVIYTKAEKFKSFQHSRLYQQFNENNSIGETQARKLSKLRAHEFIF
HTRKFITRIYPKATRADSSNFNPQEFWNIGCQMVALNFQTPGLPMDLQNGKFLDNGGSGYI
LKPHFLRESESYFNPSDIKDSMPITLTIRLISGIQLPLTHSSSNKGDTLVIIEVFGVPNDQMKQ
QTRVIKKNAFSPRWNETFTFIIHVPELALIRFVVESQGLIAGNEFLGQYTLPLLCMNKGYRR
VPLFSRMGESLEPASLFVYVWYVR

SEQ ID NO: 8 - Monkey PLC-zeta Accession number AB070109 Nucleotide sequence of 1917 bases encoding 640 amino acids

ATGGAAATGAAATGGTTTTTGTCGAAGATTCAGGATGACTTTAGAGGTGGAAAAATTA
ATCTAGAAAAAACTCAGAGGTTACTTGAAAAATTAGATATTCGGTGCAGTTATATTCA
TGTGAAACGGATATTTAAGGACAATGACAGGCTGAAACAAGGAAGAATCACCATAGA
AGAATTTAGAGCAATTTATCGAATTCTCACGCACAGAGAAGAAATTGTTGAGATTTTC
AACGCATATTCTGAAAACCGGAAAATTCTTTTAGAAAATAATCTGGTTCAATTTCTGAC
ACAAGAACAATATACAACTGAGATGAGTAAAACTATTGCTTTTGAGATCATTCAGAAA
TATGAACCTATCGAAGAAGTTAGGAAAGCACGCCAAATGTCATTAGAAGGTTTTACAA
GATATATGGATTCACGTGAATGTCAACTATTTAAAAATGAATGTAGAAAAGTTTATCA
AGATATGACTCATCCATTAAATGATTATTTTATTTCATCTTCACATAACACATATTTGGT
ATCTGATCAATTAGTGGGACCAAGTGACCTTTGGGGATATGTAAGTGCCCTTGTGAAA
GGATGCCGTTGTTTGGAGATTGATTGCTGGGATGGAGCACAAAATGAACCTGTTGTAT
ATCATGGCTACACACTCACCAGCAAACTTCTGTTTAAAACTGTTATCCAAGCTATACAC
AAGTACGCATTCATGACATCTGACTACCCAGTGGTGCTCTCTTTAGAAAATCACTGCTC
CCCTGCCCAGCAAGAAATAATGGCAGACAATTTGCAGACGACTTTTGGAGAGTCCTTG
CTTTCTGATATGCTTGCTGATTTTCCTGATACTCTACCATCACCAGAGGCACTAAAATT
CAAAGTATTAGTTAAAAATAAGAAAATAGGAACCTTAAAGGAAACCCATGAAAGAAA
AGGTTCTGATAAGCGTGGTAAGGTGGAGGAATGGGAAGAAGAAGTGGCAGATCTGGA
GGAGGAGGAGGAGGAGGAGGAGAAATTCAAAGAATCAGAAATATTCGAATCTGTTTT
AGGAGAAAATCAAGACAAGGAAACAGGGGTAAAAAAGTTATCTGGAGTAACGCTTTT
CAAGAAAAAGAAGACCAGGAAGCTAAAAATTGCTCTGGCCTTATCTGATCTTGTCATT
TATACTAAAGCTGAGAAGTTCAAAAGCTTTCAACATTCAAGATTATATCAGCAATTTA
ATGAAAATAATTCTATTGGGGAGACACAAGCCCGAAAACTTTCAAAATTGAGAGCCCA
TGAGTTTATTTTTCACACCAGGAAGTTCATTACCAGAATATATCCCAAAGCAACAAGA
GCAGACTCTTCTAATTTTAATCCCCAAGAATTTTGGAATATAGGTTGTCAAATGGTGGC
TTTAAATTTCCAGACCCCTGGTCTGCCTATGGATCTGCAAAATGGGAAATTTTTGGATA
ATGGTGGTTCTGGATATATTTTGAAACCACATTTCTTAAGAGAGAGTGAATCATACTTT
AACCCAAGTGACATAAAAGACAGTATGCCAATTACACTTACAATAAGGCTCATCAGTG
GTATCCAGTTGCCTCTTACTCATTCATCATCTAACAAAGGTGATACATTAGTAATTATA
GAAGTTTTTGGTGTTCCAAATGATCAAATGAAGCAGCAGACTCGTGTAATTAAAAAAA
ATGCTTTTAGTCCAAGATGGAATGAAACATTTACATTTATTATTCATGTCCCAGAATTG
GCATTGATACGTTTTGTTGTTGAAAGTCAAGGTTTAATAGCAGGAAATGAATTTCTTGG
GCAATATACTTTGCCACTTCTATGCATGAACAAAGGCTATCGTCGTGTTCCTCTGTTTT
CCCGAATGGGTGAGAGCCTTGAGCCTGCTTCACTGTTTGTTTATGTTTGGTACGTCAGA

SEQ ID NO: 9 - Monkey PLC-zeta Accession number AB070109 Protein sequence of 640 amino acids

MEMKWFLSKIQDDFRGGKINLEKTQRLLEKLDIRCSYIHVKRIFKDNDRLKQGRITIEEFRA
IYRILTHREEIVEIFNAYSENRKILLENNLVQFLTQEQYTTEMSKTIAFEIIQKYEPIEEVRKAR
QMSLEGFTRYMDSRECQLFKNECRKVYQDMTHPLNDYFISSSHNTYLVSDQLVGPSDLW
GYVSALVKGCRCLEIDCWDGAQNEPVVYHGYTLTSKLLFKTVIQAIHKYAFMTSDYPVVL
SLENHCSPAQQEIMADNLQTTFGESLLSDMLADFPDTLPSPEALKFKVLVKNKKIGTLKET
HERKGSDKRGKVEEWEEEVADLEEEEEEEERFKESEIFESVLGENQDKETGVKKLSGVTLFK
KKKTRKLKIALALSDLVIYTKAEKFKSFQHSRLYQQFNENNSIGETQARKLSKLRAHEFIFH
TRKFITRIYPKATRADSSNFNPQEFWNIGCQMVALNFQTPGLPMDLQNGKFLDNGGSGYIL
KPHFLRESESYFNPSDIKDSMPITLTIRLISGIQLPLTHSSSNKGDTLVIIEVFGVPNDQMKQQ
TRVIKKNAFSPRWNETFTFIIHVPELALIRFVVESQGLIAGNEFLGQYTLPLLCMNKGYRRV
PLFSRMGESLEPASLFVYVWYVR

SEQ ID NO: 10 - Rat PLC-zeta Nucleotide sequence of 1938 bases encoding 646 amino acids

ATGCATGCTCGAGCGGCCGCCAGTGTGATGGATATCTGCAGAATTCGCCCTTTTCAGG
ATGATTTCAGAGGTGGAAAAATCAGCGCTGGAATTACTCACAAACTGCTCGAGAAACT
TGATTTCCCATGCCATTTTGCTCATGTGAAACGTATTTTAAGGAAAATGACAGACATA
ACCAAGGAAGAATCACCACCGAAGATTTAGAACCATCTATCGGTGTATTGTACATAG
AGAAGAGATCGTTGAGATTTTCAACACGTATACTGAAAACAGGAAAATTCTCCCCGAG
GACAGTCTGATTGAATTTCTAACCCAAGAGCAGTATGAAATGGAGATGGATGAGTCCA
GTTCAGTGGAGATCATCCAGAAGTACGAGCCCATTGCAGAAGTAAAGAACGAGCGGC
AGATGTCAATTGAAGGTTTTGCAAGATACATGTTTTCTTCAGAATGTCTACTGTTTAAA
GAGACGTGTAACACAGTGTACCAAGATATGAATAAGCCACTAAATGATTACTATATTT
CATCGTCTCACAACACATATTTGATATCTGATCAAATATTGGGACCAAGTGACATTTGG
GGATATATAAGTGCCCTCGTGAAAGGTTGCCGCTGTCTGGAAATTGACTGCTGGGATG
GAGCACAAAATGAACCCATTGTGTACCATGGCTACACTCTCACCAGCAAGCTTCTCTTC
AAAACCGTTATCCAAGCAATAAACAAGTACGCCTTCGTGACGTCTGATTACCCAGTGG
TGCTGTCCTTAGAGAATCACTGCTCCCCTGGTCAACAGGAAGTGATGACCGACATTCT
GCAGAGTACCTTTGGAGACTTTCTGCTCTCAGACATACTTGACGAGTTTCCAGACAGTT
TGCCATCTCCAGAGGCACTGAAATTTAAAATATTAGTGAAAAATAAGAAAGTTGGAAC
CTTATCTGAAACCCGCGAGAGGCTGGGGACTGACAAAAGGGGCATAGCGCTAGACTT
GGAAGAAGAAATCTATGAAAATGAAGACGAAGACTCAGGAAAGGAGCCAGAAACGT
GGGATGATTTCCTGTCACGGGTTAAGGAGGAGCAGGAGGCAGACCCCTCAACGTTGAG
CGGAATAGCAGATGCCAAGAAAAAGATCAGGAAGCTAAGAGTAGCTCTGGCCTTATC
TGATCTTGTCATTTATACCAAAGCTGAGAAGTTCCGAAACTTCCAATATTCAAGAGTCT
ATCAGCAGTTTAATGAGACCACTTCTATGGGAGAGTCTCGAGCTCGAAAACTTTCAAA
ATTGAGAGCCCATGAGTTTATTTTCCACACTGCAGCGTTCATCACCAGAGTGTACCCCA
AGTTCACGAGAGCAGACTCTTCTAATTTTAATCCTCAAGAGTTTTGGAATGTGGGCTGT
CAGATGGTGGCCTTGAATTTTCAAACCCCTGGACTGCCTATGGATTTGCAAAACGGGA
AATTTTTGGATAATGGAGGCTCTGGATATGTTTTGAAGCCAGACTTTCTTAGAGACACA
ACTTTGGGCTTTAACCCAAATGAACCAGAAGGAGATGGCCATCCGGTTACCCTCACGA
TCCGACTCATCAGTGGGATCCAGTTGCCTGTTAACGTGCCCTCAAATACATCTGACATA
ATAGTGATCATAGAAGTCTACGGTGTCCCAAACGACCACATGAAGCAGCAGAGTCGTG
CCGTTAAGAACAATGCTTTTAGTCCAAGGTGGAATGAAACATTCACATTTCTTATTCAA
GTGCCAGAATTGGCACTGATACGTTTCGTTGTTGAAACTCAAGGCTTCCTATCGGGAA
ATGAATTACTTGGGCAGTACACTTTACCCGTTCTTTGCATGAACAAAGGTTATCGTCGT
GTTCCTCTGTTTTCCAAATCCGGTGCGAACCTTGAACCTTCCTCTCTGTTTATTTACGTT
TGGTACTACAGAGAG

SEQ ID NO: 11 - Rat PLC-zeta Protein sequence of 646 amino acids

MHARAAASVMDICRIRPFQDDFRGGKISAGITHKLLEKLDFPCHFAHVKRIFKENDRHNQG
RITTEDFRTIYRCIVHREEIVEIFNTYTENRKILPEDSLIEFLTQEQYEMEMDESSSVEIIQKYE
PIAEVKNERQMSIEGFARYMFSSECLLFKETCNTVYQDMNKPLNDYYISSSHNTYLISDQIL
GPSDIWGYISALVKGCRCLEIDCWDGAQNEPIVYHGYTLTSKLLFKTVIQAINKYAFVTSD
YPVVLSLENHCSPGQQEVMTDILQSTFGDFLLSDILDEFPDSLPSPEALKFKILVKNKKVGT
LSETRERLGTDKRGIALDLEEEIYENEDEDSGKEPETWDDFLSRVKEEQEADPSTLSGIADA
KKKIRKLRVALALSDLVIYTKAEKFRNFQYSRVYQQFNETTSMGESRARKLSKLRAHEFIF
HTAAFITRVYPKFTRADSSNFNPQEFWNVGCQMVALNFQTPGLPMDLQNGKFLDNGGSG
YVLKPDFLRDTTLGFNPNEPEGDGHPVTLTIRLISGIQLPVNVPSNTSDIIVIIEVYGVPNDHM
KQQSRAVKNNAFSPRWNETFTFLIQVPELALIRFVVETQGFLSGNELLGQYTLPVLCMNKG
YRRVPLFSKSGANLEPSSLFIYVWYYRE

SEQ ID NO: 12 – PLC Sequences Alignment

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 1 MTSEEMTASVLIPVTQRKVVSAQSAADESSEKVSDINISKAHTVRRSGET
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 51 SHTISQLNKLKEEPSGSNLPKILSIAREKIVSDENSNEKCWEKIMPDSAK
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 101 NLNINCNNILRNHQHGLPQRQFYEMYNSVAEEDLCLETGIPSPLERKVFP
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 151 GIQLELDRPSMGISPLGNQSVIIETGRAHPDSRRAVFHFHYEVDRRMSDT
delta1-human 1 --------------------------------------------------
delta3-human 1 --------------------------------------------------
delta2-bovine 1 --------------------------------------------------

delta4-rat 1 --------------
gamma1-human 1 --------------------------------------------------
gamma2-human 1 --------------------------------------------------

zeta-monkeyA 1 --------------------------------------------------
zeta-human I --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 201 FCTLSENLILDDCGNCVPLPGGEEKQKKNYVAYTCKLMELAKNCDNKNEQ
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 251 LQCDHCDTLNDKYFCFEGSCEKVDMVYSGDSFCRKDFTDSQAAKTFLSHF
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 301 EDFPDNCDDVEEDAFKSKKERSTLLVRRFCKNDREVKKSVYTGTRAIVRT
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 351 LPSGHIGLTAWSYIDQKRNGPLLPCGRVMEPPSTVEIRQDGSQRLSEAQW
delta1-human 1 --------------------------------------------------
delta3-human 1 --------------------------------------------------
delta2-bovine 1 --------------------------------------------------

delta4-rat 1 ----------------------
gamma1-human 1 ------------------------------------------
gamma2-human 1 ------------------------------------------

zeta-monkeyA 1 ------------------------------------------
zeta-human 1 ------------------------------------------
zeta-mouse 1 ------------------------------------------
zeta-rat 1 ------------------------------------------
beta1-human 1 ------------------------------------------
beta3-mouse 1 ------------------------------------------
beta2-human 1 ------------------------------------------
beta4-mouse 1 ------------------------------------------
epsilon-human 401 .YPIYNAVRREETENTVGSLLHFLTKLPASETAHGRISVGPCLKQCVRDTV
delta1-human 1 ------------------------------------------
delta3-human 1 ------------------------------------------
delta2-bovine 1 ------------------------------------------
delta4-rat 1 ------------------------------------------
gamma1-human 1 ------------------------------------------
gamma2-human 1 ------------------------------------------

zeta-monkeyA 1 ------------------------------------------
zeta-human 1 ------------------------------------------
zeta-mouse 1 ------------------------------------------
zeta-rat 1 ------------------------------------------
beta1-human 1 ------------------------------------------
beta3-mouse 1 ------------------------------------------
beta2-human 1 ------------------------------------------
beta4-mouse 1 ------------------------------------------
epsilon-human 451 CEYRATLQRTSISQYITGSLLEATTSLGARSGLLSTFGGSTGRMMLKERQ
delta1-human 1 ------------------------------------------
delta3-human 1 ------------------------------------------
delta2-bovine 1 ------------------------------------------
delta4-rat 1 ------------------------------------------
gamma1-human 1 ------------------------------------------
gamma2-human 1 ------------------------------------------

zeta-monkeyA 1 ------------------------------------------
zeta-human 1 ------------------------------------------
zeta-mouse 1 ------------------------------------------
zeta-rat 1 ------------------------------------------
beta1-human 1 ------------------------------------------
beta3-mouse 1 ------------------------------------------
beta2-human 1 ------------------------------------------
beta4-mouse 1 ------------------------------------------
epsilon-human 501 PGPSVANSNALPSSSAGISKELIDLQPLIQFPEEVASILMEQEQTIYRRV
delta1-human 1 ------------------------------------------
delta3-human 1 ------------------------------------------
delta2-bovine 1 ------------------------------------------
delta4-rat 1 ------------------------------------------
gamma1-human 1 ------------------------------------------
gamma2-human 1 ------------------------------------------

zeta-monkeyA 1 ------------------------------------------
zeta-human 1 ------------------------------------------
zeta-mouse 1 ------------------------------------------
zeta-rat 1 ------------------------------------------
beta1-human 1 ------------------------------------------
beta3-mouse 1 ------------------------------------------
beta2-human 1 ------------------------------------------
beta4-mouse 1 ------------------------------------------
epsilon-human 551 LPVDYLCFLTRDLGTPECQSSLPCLKASISASILTTQNGEHNALEDLVMR
delta1-human 1 ------------------------------------------
delta3-human 1 ------------------------------------------

delta2-bovine 1 ---------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 601 FNEVSSWVTWLILTAGSMEEKREVFSYLVHVAKCCWNMGNYNAVMEFLAG
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 651 LRSRKVLKMWQFMDQSDIETMRSLKDAMAQHESSCEYRKVVTRALHIPGC
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 701 KVVPFCGVFLKELCEVLDGASGLMKLCPRYNSQEETLEFVADYSGQDNFL
delta1-human 1 --------------------------------------------------
delta3-human 1 --------------------------------------------------
delta2-bovine 1 --------------------------------------------------
delta4-rat 1 --------------------------------------------------
gamma1-human 1 --------------------------------------------------
gamma2-human 1 --------------------------------------------------

zeta-monkeyA 1 --------------------------------------------------
zeta-human 1 --------------------------------------------------
zeta-mouse 1 --------------------------------------------------
zeta-rat 1 --------------------------------------------------
beta1-human 1 --------------------------------------------------
beta3-mouse 1 --------------------------------------------------
beta2-human 1 --------------------------------------------------
beta4-mouse 1 --------------------------------------------------
epsilon-human 751 QRVGQNGLKNSEKESTVNSIFQVIRSCNRSLETDEEDSPSEGNSSRKSSL
delta1-human 1 --------------------------------------------------
delta3-human 1 --------------------------------------------------

delta2-bovine 1 -------------
delta4-rat 1 ----------------------------------------------
gamma1-human 1 ----------------------------------------------
gamma2-human 1 ----------------------------------------------

zeta-monkeyA 1 ----------------------------------------------
zeta-human 1 ----------------------------------------------
zeta-mouse 1 ----------------------------------------------
zeta-rat 1 ----------------------------------------------
beta1-human 1 -------------------------------------MGSLQGIATK
beta3-mouse 1 ------------------------------------MAGARPGVHAL
beta2-human 1 ----------------------------------------MSLLNP
beta4-mouse 1 ----------------------------------------MAKPYE
epsilon-human 801 KDKSRWQFIIGDLLDSDNDIFEQSKEYDSHGSEDSQKAEDHGTELIPWYV
delta1-human 1 -----------------------------------MDSGRDFLTLH
delta3-human 1 ---------------------------------------------M
delta2-bovine 1 ---------------------------------------MAYLLQG
delta4-rat 1 ---------------------------------------MASQIQK
gamma1-human 1 ----------------------------MAGAASPCANGCGPGAPS
gamma2-human 1 -----------------------------------MSTTVNVDSLA

zeta-monkeyA 1 ----------------------------------------------
zeta-human 1 ----------------------------------------------
zeta-mouse 1 ----------------------------------------------
zeta-rat 1 ----------------------------------------------
beta1-human 11 ILIRILSDALERKEE----DLKSSTIVTP-IILRTEPQGFEFYWTDQNKE
beta3-mouse 12 QLEPSTVVKTERRSSKFIKWDEEASSRNL-VTLRVEPNGFELYWTGPNME
beta2-human 7 VLLPPKVKAYLSQEERFIKWDDETTVASP-VILRVDPKGYYLYWTYQSKE
beta4-mouse 7 FNWQKEVPSFLQESAVFDRYEEESFVFEPNCLFKVDEFGFFLTWKSEGKE
epsilon-human 851 LSIQADVHQFLLQSATVIHYDQDTHLSAR-CFLQLQPDNSTLTWVKPTTA
delta1-human 12 GLQDDEDLQALLKGSQLLKVKSSSWRRERFYKLQEDCKTIWQ--------
delta3-human 2 GLTEDEDVRAMLRGSRLRKIRSRTWHKERLYRLQEDGLSVWF--------
delta2-bovine 8 RLPINQDLLLMQKGTMMRKVRSKSWKKLRFFRLQDDGMTVWH--------
delta4-rat 8 LLTTNQDLLLMQKGTMMRKVRTKSWKKLRYFRLQDDGMTVWH--------
gamma1-human 19 DAEVLHLCRSLEVGTVMTLFYSKKSQRPERKTFQVKLETRQITWS-----
gamma2-human 12 EYEKSQIKRALELGTVMTVFSFRKS-TPERRTVQVIMETRQVAWS-----

zeta-monkeyA 1 ----------------------------------------------
zeta-human 1 ----------------------------------------------
zeta-mouse 1 ----------------------------------------------
zeta-rat 1 ----------------------------------------------
beta1-human 56 ----------------------------------------------
beta3-mouse 61 ----------------------------------------------
beta2-human 56 ----------------------------------------------
beta4-mouse 57 ----------------------------------------------
epsilon-human 900 SPASSKAKLGVLNNTAEPGKFPLLGNAGLSSLTEGVLDLFAVKAVYMGHP
delta1-human 54 ----------------------------------------------
delta3-human 44 ----------------------------------------------
delta2-bovine 50 ----------------------------------------------
delta4-rat 50 ----------------------------------------------
gamma1-human 64 ----------------------------------------------
gamma2-human 56 ----------------------------------------------

zeta-monkeyA 1 ----------------------------------------------
zeta-human 1 ----------------------------------------------
zeta-mouse 1 ----------------------------------------------
zeta-rat 1 ----------------------------------------------
beta1-human 56 ----------------------------------------------
beta3-mouse 61 ----------------------------------------------
beta2-human 56 ----------------------------------------------
beta4-mouse 57 ----------------------------------------------
epsilon-human 950 GIDIHTVCVQNKLGSMFLSETGVTLLYGLQTTDNRLLHFVAPKHTAKMLF
delta1-human 54 ----------------------------------------------
delta3-human 44 ----------------------------------------------

delta2-bovine 50 ----------------------------
delta4-rat 50 ------------------------------------------------
gamma1-human 64 ------------------------------------------------
gamma2-human 56 ------------------------------------------------

zeta-monkeyA 1 ------------------------------------------------
zeta-human 1 ------------------------------------------------
zeta-mouse 1 ------------------------------------------------
zeta-rat 1 ------------------------------------------------
beta1-human 56 ------------------------------------------------
beta3-mouse 61 ------------------------------------------------
beta2-human 56 ------------------------------------------------
beta4-mouse 57 ------------------------------------------------
epsilon-human 1000 SGLLELTRAVRKMRKFPDQRQQWLRKQYVSLYQEDGRYEGPTLAHAVELF
delta1-human 54 ------------------------------------------------
delta3-human 44 ------------------------------------------------
delta2-bovine 50 ------------------------------------------------
delta4-rat 50 ------------------------------------------------
gamma1-human 64 ------------------------------------------------
gamma2-human 56 ------------------------------------------------

zeta-monkeyA 1 ------------------------------------------------
zeta-human 1 ------------------------------------------------
zeta-mouse 1 ------------------------------------------------
zeta-rat 1 ------------------------------------------------
beta1-human 56 ------------------------------------------TELLDLSL
beta3-mouse 61 ------------------------------------------VDTLDISS
beta2-human 56 ------------------------------------------MEFLDITS
beta4-mouse 57 ------------------------------------------GQVLECSL
epsilon-human 1050 GGRRWSARNPSPGTSAKNAEKPNMQRNNTLGISTTKKKKKILMRGESGEV
delta1-human 54 ------------------------------------------ESRKVMRTP
delta3-human 44 ------------------------------------------QRR-IPRAP
delta2-bovine 50 ------------------------------------------ARQAGGRAK
delta4-rat 50 ------------------------------------------GRHLESISK
gamma1-human 64 ------------------------------------------RGADKIEG
gamma2-human 56 ------------------------------------------KTADKIEG

zeta-monkeyA 1 ------------------------------------------------
zeta-human 1 ------------------------------------------------
zeta-mouse 1 ------------------------------------------------
zeta-rat 1 ------------------------------------------------
beta1-human 64 VKDARCGRHAKAPRDPKLRELLDVGN-IGRLEQRMITVVYRPDLVN----
beta3-mouse 69 IRDTRTGRYARLPKDPKIREVLGFGGPDTRLEEKLMTVVAGPDPVN----
beta2-human 64 IRDTRFGKFAKMPKSQKLRDVFNMDFPDNSFLLKTLTVVSGPDMVD----
beta4-mouse 65 INSIRQAAIPKDPR--ILAALEAVGKSENDLEGRILCVCSGTDLVN----
epsilon-human 1100 TDDEMATRKAKMHKECRSRSGSDPQDINEQEESEVNAIANPPNPLPSRRA
delta1-human 63 ESQLFSIEDIQEVRMGHRTEGLEKFARDVPEDRCFSIVFKDQ--------
delta3-human 52 SQHIFFVQHIEAVKEGHQSEGLRRFGGAFAPARCLTIAFKGR--------
delta2-bovine 59 PS--FSISDVDTVREGHESELLRNLAEEFPLEQGFTIVFHGR--------
delta4-rat 59 PT--FSISDVERIRKGQDSELLRYLVEEFPLEQGFTIVFNGR--------
gamma1-human 72 AIDIREIKEIRPGKTSRDFDRYQEDPAFRPDQSHCFVILYGMEFRLK---
gamma2-human 64 FLDIMEIKEIRPGKNSKDFERAK---AVRQKEDCCFTILYGTQFVLS---

zeta-monkeyA 1 ------------------------------------------------
zeta-human 1 ------------------------------------------------
zeta-mouse 1 ------------------------------------------------
zeta-rat 1 ------------------------------------------------
beta1-human 109 ---------------------------ISHLNELVAFQEEVAKEWTNEVFSL
beta3-mouse 115 ---------------------------TTELNFMAVQDDTVKVWSEELFKL
beta2-human 110 ---------------------------LTFHNFVSYKENVGKAWAEDVLAL
beta4-mouse 109 ---------------------------IGFTYMVEENPEVTKQWVEGLRSI
epsilon-human 1150 HSLTTAGSPNLAAGTSSPIRPVSSPVLSSSNKSPSSAWSSSSWHGRIKGG
delta1-human 105 ---------------------------RNTLDLIAPSPADAQHWVLGLHKI
delta3-human 94 ---------------------------RKNLDLAAPTAEEAQRWVRGLTKL

delta2-bovine 99 ---------------
delta4-rat 99 ---------------------------RPNLDLVANSVEEAQTWMRGLQLL
gamma1-human 119 ----------------------------TLSLQPTSEDEVNMWIKGLTWL
gamma2-human 108 ----------------------------TLSLAPDSKEDAVNWLSGLKIL

zeta-monkeyA 1 -------------MEMKWFLSKIQDDFRGGKINLEKTQRLLEKLD----I
zeta-human 1 -------------MEMRWFLSKIQDDFRGGKINLEKTQRLLEKLD----I
zeta-mouse 1 -----MESQLHELAEARWFLSKVQDDFRGGKINVEITHKLLEKLD----F
zeta-rat 1 -----MHARAAASVMDICRIRPFQDDFRGGKISAGITHKLLEKLD----F
beta1-human 133 ATNLLAQNMSRDAFLEKAYTKLKLQVTPEGRIPLKNIYRLFSADR--KRV
beta3-mouse 139 AMNILAQNASRNTFLRKAYTKLKLQVNQDGRIPVKNILKMFSADK--KRV
beta2-human 134 VKHPLTANASRSTFLDKILVKLKMQLNSEGKIPVKNFFQMFPADR--KRV
beta4-mouse 133 IHNFRANNVSPMTCLKKHWMKLAFLTNTTGKIPVRSITRTFASGKTEKVI
epsilon-human 1200 MKGFQSFMVSDSNMSFVEFVELFKSFSVRSRKDLKDLFDVYAVPCNRSGS
delta1-human 129 IHHSGSMDQRQKLQHWIHSCLRKADKNKDNKMSFKELQNFLKELN----I
delta3-human 118 AARLDAMSQRERLDHWIHSYLHRADSNQDSKMSFKEIKSLLRMVN----V
delta2-bovine 123 VGFVTNMDQQERLDQWLSDWFQRGDKNQDGRMSFGEVQRLLHLMN----V
delta4-rat 123 VDLVARMNYQEQLDQMLREWFQQADRNQDSRMSFREAQRLLLLMN----V
gamma1-human 141 MEDTLQAPTPLQIERWLRKQFYSVDRNREDRISAKDLKNMLSQVN----Y
gamma2-human 130 HQEAMNASTPTIIESWLRKQIYSVDQTRRNSISLRELKTILPLIN----F

zeta-monkeyA 34 RCSYIHVKRDFKDNDRLKQGRITIEEFRAIYRIITAREE-----------
zeta-human 34 RCSYIHVKQIFKDNDRLKQGRITIEEFRAIYRIITAREE-----------
zeta-mouse 42 PCHFAHVKHIFKENDRQNQGRITIEEFRAIYRCIVAREE-----------
zeta-rat 42 PCHFAHVKRIFKENDRHNQGRITTEDFRTIYRCIVAREE-----------
beta1-human 181 ETAMEACSLPSSRNDSILQEDFTQPEVYRVFENNLCLRPE----------
beta3-mouse 187 ETALESCGLNFNRSESIRPDEFPLEIFERFLNKLCLRPD----------
beta2-human 182 EAALSACHLPKGKNDAINPEDFPEPVYKSFLMSLCPRPE----------
beta4-mouse 183 FQALKELGLPSGKNDEIEPAAFTYEKFYELTQKICPRTD----------
epsilon-human 1250 ESAPLYTNLTIDEETSDLQPDLDLLTRNVSDLGLFIKSKQQLSDNQRQIS
delta1-human 175 QVDDSYARKIFRECDHSQTDSLEDEEIEAFYKMLTQRVE----------
delta3-human 164 DMNDMYAYLLFKECDHSNNDRLEGAEIEEFLRRLLKRPE----------
delta2-bovine 169 EMDQEYAFQLFQTADTSQSGTLEGEEFVEFYKSLTQRPE----------
delta4-rat 169 EMDEEYAFSLFQEADVSQSNTLDSEEFVQFYKALTKRTE----------
gamma1-human 187 RVPNMRFLRERLTDLEQRSGDITYGQFAQLYRSLMYSAQKTMDLPFLEAS
gamma2-human 176 KVSSAKFLKDKFVEIGAHKDELSFEQFHLFYKKLMFEQQKSILDEFKKDS

zeta-monkeyA 73 -IVEIFNAYSENRKILLENNLVQFLTQEQYTTEMSKTIA---------FE
zeta-human 73 -IIEIFNTYSENRKILLASNLAQFLTQEQYAAEMSKAIA---------FE
zeta-mouse 81 -ITEIFNTYTENRKILSENSLIEFLTQEQYEMEIDHSDS---------VE
zeta-rat 81 -IVEIFNTYTENRKILPEDSLIEFLTQEQYEMEMDESSS---------VE
beta1-human 220 IDNIFSEFGAKSKPYLTVDQMMDFINLKQRDPRLNEILYPPPKQEQVQVL
beta3-mouse 226 IDKILLEIGAKGKPYLTLEQLMDFINQKQRDPRLNEVLYPPLRSSQARLL
beta2-human 221 IDEIFTSYHAKAKPYMTKEHLTKFINQKQRDSRLNSLLFPPARPDQVQGL
beta4-mouse 222 IEDLFKKINGDKTDYLTVDQLVSFLNEHQRDPRLNEILFPFYDAKRAMQI
epsilon-human 1300 DAIAAASIVTNGTGIESTSLGIFGVGILQLNDFLVNCQGEHCTYDEILSI
delta1-human 214 -IDRTFAEAAGPGETLSVDQLVTFLQHQQREEAAGPALA---------LSL
delta3-human 203 -LEEIFHQYSGEDRVLSAPELLEFLEDQG-EEGATLARA---------QQL
delta2-bovine 208 -VQELFEKFSSDGQKLTLLEFVDFLQEEQKEGERASDLA---------LEL
delta4-rat 208 -IEELFENFSSDKQKLTLLEFVDFLREEQKESDHSSDLA---------LKL
gamma1-human 237 TLRAG---ERPELCRVSLPEFQQFLLDYQGELWAVDRLQVQ-------EFM
gamma2-human 226 SVFILGNTDRPDASAVYLHDFQRFLIHEQQEHWAQDLNKVR-------ERM

zeta-monkeyA 114 IQKYEEIEEVRKARQMSLEGFTRYMDSRE-CQLFKNECRKVYQDMTHPLN
zeta-human 114 IQKYEEIEEVRKAHQMSLEGFTRYMDSRE-CLLFKNECRKVYQDMTHPLN
zeta-mouse 122 INKYEEIEEVKGERQMSIEGFARYMFSSE-CLLFKENCKTVYQDMNHPLS
zeta-rat 122 IQKYEEIAEVKNERQMSIEGFARYMFSSE-CLLFKETCNTVYQDMNKPLN
beta1-human 270 IEKYEENNSLARKGQISVDGFMRYLSGEE-NGVVSTEKLDLNEDMSRPLS
beta3-mouse 276 IEKYETNKQFLERDQMSMEGFSRYLGGEE-NGILPLEALDLSMDMTQPLS
beta2-human 271 IDKYEESGINAQRGQLSPEGMVWFLCGPE-NSVLAQDKLLLHHDMTQPLN
beta4-mouse 272 IEMYEPDEELKKKGLISSDGFCKYLMSDE-NAPVFLDRLELYQEMDHPLA
epsilon-human 1350 IQKFEPSISMCHQGLMSFEGFARFLMDKENFASKNDESQENIKELQLPLS
delta1-human 255 IERYEPSETTKAQRQMTKDGFLMYLLSAD-GSAFSLAHRRVYQDMGQPLS
delta3-human 243 IQTYELNETAKQHELMTLDGFMMYLLSPE-GAALDNTHTCVFQDMNQPLA

delta2-bovine 249 IDRYEESESGKLRHV
delta4-rat 249 IDRYEESENGRLLRVLSKDGFLSYLCSAD-GNIFNPDCLPIYQDMTQPLS
gamma1-human 278 LSFLRDPLREIEEPYFFLDEFVTFLFSKENSVWNSQLDAVCPDTMNNPLS
gamma2-human 270 TKFILDDTMRETAEPFLFVDEFLTYLFSRENSIWDEKYDAVDMQDMNNPLS

zeta-monkeyA 163 DYFISSSHNTYLVSDQLVGPSDLW-YVSALVKGCRCLEIDCWDG--AQNE
zeta-human 163 DYFISSSHNTYLVSDQLLGPSDLW-YVSALVKGCRCLEIDCWDG--AQNE
zeta-mouse 171 DYFISSSHNTYLISDQILGPSDIW-YVSALVKGCRCLEIDCWDG--SQNE
zeta-rat 171 DYYISSSHNTYLISDQILGPSDIW-YISALVKGCRCLEIDCWDG--AQNE
beta1-human 319 HYFINSSHNTYLTAGQIAGNSSVEMYRQALISGCRCVELDCWRGRTAEEE
beta3-mouse 325 AYFINSSHNTYLTAGQLAGPSSVEMYRQALIWGCRCVELDVWKGRPPEEE
beta2-human 320 HYFINSSHNTYLTAGQFSGLSSAEMYRQVLLSGCRCVELDCWKGKPPDEE
beta4-mouse 321 HYFISSSHNTYLTGRQFGGKSSVEMYRQVLLAGCRCVELDCWDGKGEDQE
epsilon-human 1400 YYYIESSHNTYLTGHQLKGESSVELYSQVLLQGCRSVELDCWDG--DDGM
delta1-human 304 HYLVSSSHNTYLLEDQLAGPSSTEAYIRALCKGCRCLELDCWDG--PNQE
delta3-human 292 HYFISSSHNTYLTDSQIGGPSSTEAYVRAFAQGCRCVELDCWEG--PGGE
delta2-bovine 298 HYYINSSHNTYLVGDQLCGQSSVEGYIRALKRGCRCVEVDIWDG--PSGE
delta4-rat 298 HYYINSSHNTYLLGDQFCGQSSVEGYIRALKRGCRCVEVDTWDG--PDGE
gamma1-human 328 HYWISSSHNTYLTGDQFSSESSLEAYARCLRMGCRCIELDCWDG--PDGM
gamma2-human 320 HYWISSSHNTYLTGDQLRSESSPEAYIRCLRMGCRCIELDCWDG--PDGK

zeta-monkeyA 211 PV YHG TLTSK LFKTVIQAIHKYAFMTSDYPVVLSLENHCS-PAQQEI
zeta-human 211 PV YHG TLTSK LFKTVIQAIHKYAFMTSDYPVVLSLENHCS-TAQQEV
zeta-mouse 219 PI YHG TFTSK LFKTVVQAINKYAFVTSDYPVVLSLENHCS-PGQQEV
zeta-rat 219 PI YHG TLTSK LFKTVIQAINKYAFVTSDYPVVLSLENHCS-PGQQEV
beta1-human 369 PVITHGFTMTTEISFKEVIKAIAECAFKTSPFPILLSFENHVDSPKQQAK
beta3-mouse 375 PFITHGFTMTTEVPLRDVLEAIAEAAFKTSPYPVILSFENHVDSAKQQAK
beta2-human 370 PIITHGFTMTTDIFFKEAIEAIAESAFKTSPYPIILSFENHVDSPRQQAK
beta4-mouse 371 PIITHGKAMCTDILFKDVIQAIKETAFVTSEYPVILSFENHCS-KYQQYK
epsilon-human 1448 PIIYHGHTPTTKIPFKEVVEAIDRSAFINSDLPIIISIENHCS-LPQQRK
delta1-human 352 PIIYHGYTFTSKILFCDVLRAIRDYAFKASPYPVILSLENHCT-LEQQRV
delta3-human 340 PVIYHGHTLTSKILFRDVVQAVRDHAFTLSPYPVILSLENHCG-LEQQAA
delta2-bovine 346 PIVYHGHTLTSRIPFKDVVAAIGQYAFQTSDYPVILSLENHCS-WEQQEI
delta4-rat 346 PVVYHGRTLTSRILFKDVIATLAQYAFQSSDYPLILSLDNHCT-WEQQKT
gamma1-human 376 PVIYHGHTLTTKIKFSDVLHTIKEHAFVASEYPVILSIEDHCS-IAQQRN
gamma2-human 368 PVIYHGWTRTTKIKFDDVVQAIKDHAFVTSSFPVILSIEEHCS-VEQQRH

zeta-monkeyA 260 MADNLQTTFGESLL DMLADFPDT-----LPSPEALKFKVLVKNKKIGTLK
zeta-human 260 MADNLQATFGESLL DMLEDFPDT-----LPSPEALKFKILVKNKKIGTLK
zeta-mouse 268 MASILQSTFGDFLL DMLEEFPDT-----LPSPEALKFKILVKNRKVGTLS
zeta-rat 268 MTDILQSTFGDFLL DILDEFPDS-----LPSPEALKFKILVKNKKVGTLS
beta1-human 419 MAEYCRLIFGDALIMEPLEKYPLESGVPLPSPMDLMYKILVKNKKKSHKS
beta3-mouse 425 MAEYCRSIFGDALLIDPLDKYPLSAGIPLPSPQDLMGRILVKNKKRHRPS
beta2-human 420 MAEYCRTIFGDMLLTEPLEKFPLKPGVPLPSPEDLRGKILIKNKKNQFSG
beta4-mouse 420 MSKYCEDLFGDLLLKQALESHPLEPGRPLPSPNDLKRKILIKNKRLKPEV
epsilon-human 1497 MAEIFKTVFGEKLVTKFLFETDFSDDPMLPSPEQLRKKVLLKNKKLKAHQ
delta1-human 401 MARHLHAILGPMLLNRPLDG-VTNS---LPSPEQLKGKILLKGKKLGGLL
delta3-human 389 MARHLCTILGDMLVTQALDSPNPEE---LPSPEQLKGRVLVKGKKLPAAR
delta2-bovine 395 IVRHLTEILGDQLLTTALDGQPPTQ---LPSPEDLRGKILVKGKKLMLEE
delta4-rat 395 MAHHLIAIFGEQLLSTTLEEQLIDI---MPSPEQLRGKILVKGKKLRTIE
gamma1-human 425 MAQYFKKVLGDTLLTKPVEISADG----LPSPNQLKRKILIKHKKLAEGS
gamma2-human 417 MAKAFKEVFGDLLLTKPTEASADQ----LPSPSQLREKIIIKHKKLGP--

zeta-monkeyA 306 ETHERKGSDKRGKVEEWEEEVADLEEEEEEEEKFKESEI-----------
zeta-human 306 ETHERKGSDKRG--------------------------------------
zeta-mouse 314 ETHERIGTDKSGQVLEWKEVIYE----DGDEDSGMDPET-----------
zeta-rat 314 ETRERLGTDKRGIALDLEEEIYE----NEDEDSGKEPET-----------
beta1-human 469 S---EGSGKKKLSEQASNTYSDSSSMFEPSSP---GAG------------
beta3-mouse 475 TGVPDSSVRKRPLEQSNSALSESSAATEPSSP---QLGSPSSDSCPGLSN
beta2-human 470 PTSSSKDTGGEAEGSSPPSAPAVWAGEEGTELEEE---------------
beta4-mouse 470 EKKQLEALKSMMEAGESAAPASILEDDNEEEIESADQEEEAHPEYKFG--
epsilon-human 1547 TPVDILKQKAHQLASMQVQAYNGGNANPRPANNEEEEDEEDEYDYDYESL
delta1-human 447 PPGGEGGPEATVVSDEDEAAEMEDEA------------------------
delta3-human 436 SEDGRALSDREEEEEEDDEEEEEEVEA-----------------------

delta2-bovine 442 EEEEPEAELEAEQEAI
delta4-rat 442 VVESDKEEEELEKDEGSDLDPASAEL------------------------
gamma1-human 471 AYEEVPTSMMYSENDISNSIKNGILYLEDPVNHEWYPHYFVLTSSKIYYS
gamma2-human 461 ----RGDVEVNMEDKKDEHKQQGELYMWDSIDQKWTRHYCAIADAKLSFS

zeta-monkeyA 345 -------------------------------------------FESVLGE
zeta-human 318 -------------------------------------------------D
zeta-mouse 349 -------------------------------------------WDVFLSR
zeta-rat 349 -------------------------------------------WDDFLSR
beta1-human 501 ----------------------------------------------EADT
beta3-mouse 522 GEEVGL-----------EKTSLEPQKSLGEESLSREPNVPMPDRDREDEE
beta2-human 505 -------------------------------------------EVEEEEE
beta4-mouse 518 ---------------------------------NELSADDYSHKEAVANS
epsilon-human 1597 SDDNIL-----------EDRPENKSCN-DKLQFEYNEEIPKRIKKADNSA
delta1-human 473 ---------------------------------------------VRSRV
delta3-human 462 ---------------------------------------------AAQRR
delta2-bovine 468 ---------------------------------------------PQDLS
delta4-rat 468 ---------------------------------------------DMQSQ
gamma1-human 521 EETSSDQGNEDEEEPKEVSSSTELHSNEKWFHGKLGAGRDGRHIAERLLT
gamma2-human 507 DDIEQT----MEEEVPQDIPPTELHFGEKWFHKKVEKRTS----AEKLLQ

zeta-monkeyA 352 NQDKETGVKKLSGVTLFKKKKTRKLKEALALSDLVVYTKA----------
zeta-human 319 NQDKETGVKKLPGVMLFKKKKTRKLKEALALSDLVVYTKA----------
zeta-mouse 356 IKEEREADPSTLSGIAGVKKRKRKMKIAMALSDLVVYTKA----------
zeta-rat 356 VKEEQEADPSTLSGIADAKKKIRKLRVALALSDLVVYTKA----------
beta1-human 505 ESDDEDDDDDCKKSSMDEGTAGSEAMATEEMSNLVNYIQP----------
beta3-mouse 561 EDEEEEETTDPKKPTTDEGTASSEVNATEEMSTLVNYVEP----------
beta2-human 512 EESGNLDEEEIKKMQSDEGTAGLEVTAYEEMSSLVNYIQP----------
beta4-mouse 535 VKKGLVTVEDEQAWMASYKYVGATTNIHPYLSTMINYAQP----------
epsilon-human 1635 CNKGKVYDMELGEEFYLDQNKKESRQIAPELSDLVIYRQA----------
delta1-human 478 QHKPKED----------------KLRLAQELSDMVIYCKS----------
delta3-human 467 LAK----------------------QISPELSAIAVYCHA----------
delta2-bovine 473 PRSEDKKKKPK-------------AILCPALSALVVYLKA----------
delta4-rat 473 PESQEQASGNKSKNKKKFLLQSSTTILCPDLSALVVYLRT----------
gamma1-human 571 EYCIETGAPDGSFLVRESETFVGDYTLSFWRNGKVQHCRIHSRQDAGTPK
gamma2-human 549 EYCMETGGKDGTFLVRESETFPNDYTLSFWRSGRVQHCRIRSTMEGGTLK

zeta-monkeyA 392 ------EKFKSFQHS-----------------------------------
zeta-human 359 ------EKFKSFQHS-----------------------------------
zeta-mouse 396 ------EKFRNLQYS-----------------------------------
zeta-rat 396 ------EKFRNLQYS-----------------------------------
beta1-human 545 ------VKFESFEIS-----------------------------------
beta3-mouse 601 ------VKFKSFEAS-----------------------------------
beta2-human 552 ------TKFVSFEFS-----------------------------------
beta4-mouse 575 ------VKFQGFHVA-----------------------------------
epsilon-human 1675 ------VKFPGLSTLNASGSSRGKERKSRKSIFGNNPGRMSPGETASFNK
delta1-human 502 ------VHFGGFSSPG----------------------------------
delta3-human 485 ------TELR-TLHPA----------------------------------
delta2-bovine 500 ------VTFYSFTHS-----------------------------------
delta4-rat 513 ------APFCSFTHS-----------------------------------
gamma1-human 621 FFLTDN--------------------------------------------
gamma2-human 599 YYLTDN--------------------------------------------

zeta-monkeyA 401 ------------------------------RLYCQFNENNEIGETQAVKL
zeta-human 368 ------------------------------RLYCQFNENNEIGETQAVKL
zeta-mouse 405 ------------------------------RVYCQFNETNSIGESRAVKL
zeta-rat 405 ------------------------------RVYCQFNETTSMGESRAVKL
beta1-human 554 ------------------------------KKRNKSFEMSSFVETKGLEQ
beta3-mouse 610 ------------------------------RKRNKCFEMSSFVETKAMEQ
beta2-human 561 ------------------------------AQKNRSHVISSFTELKAYDL
beta4-mouse 584 ------------------------------EERNIHYNMSSFNESVGLGY
epsilon-human 1719 TSGKSSCEGIRQTWEESSSPLNPTTSLSAIIRTPKCHHTSSLNFNAAKRL
delta1-human 512 ------------------------------TPGCAFYEMASFSENRALRL
delta3-human 494 ------------------------------PNAPQPCQVSSLSERKAKKL

delta2-bovine 509 ---------------------------- [?]ENYHI[?]YDISSFSESK[?]NI
delta4-rat 522 ----------------------------KENYHIYDISSFSESKANNI
gamma1-human 627 ----------------------------LVFDSLYDLITHYQQVPLRCNEF
gamma2-human 605 ----------------------------LRFERMYALICHYRETHLPCAEF

zeta-monkeyA 421 SILRAHEF.FHTRKF.[?]RIYPKATRADSSN[?]NPQEFWNIGCQMVALNFQT
zeta-human 388 SILRVHEF.FHTRKF.[?]RIYPKATRADSSN[?]NPQEFWNIGCQMVALNFQT
zeta-mouse 425 SILRVHEF.FHTAAF.[?]RVYPKMMRADSSN[?]NPQEFWNVGCQMVALNFQT
zeta-rat 425 SILRAHEF.FHTAAF.[?]RVYPKFTRADSSN[?]NPQEFWNVGCQMVALNFQT
beta1-human 574 LTKSPVEFVEYNKMQLSRIYPKGTRVDSSNYMPQLFWNAGCQMVALNFQT
beta3-mouse 630 LTKSPMEFVEYNKQQLSRIYPKGTRVDSSNYMPQLFWNVGCQLVALNFQT
beta2-human 581 LSKASVQFVDYNKRQMSRIYPKGTRMDSSNYMPQMFWNAGCQMVALNFQT
beta4-mouse 604 LKTHAIEFVNYNKRQMSRIYPKGGRVDSSNYMPQIFWNAGCQMVSLNYQT
epsilon-human 1769 CRRYSQKLIQHTACQLLRTYPAATRIDSSNPNPLMFWLHGIQLVALNYQT
delta1-human 532 LQESGNGFVRHNVGHLSRIYPAGWRTDSSNYSPVEMWNGGCQIVALNFQT
delta3-human 514 IREAGNSFVRHNAPQLTRVYPLGLRMNSANYSPQEMWNSGCQLVALNFQT
delta2-bovine 529 IKEAGDEFVQHNAWQLSRVYPSGLRTDSSNYNPQEFWNAGCQMVAMNMQT
delta4-rat 542 IREAGNEFVQHNARQLCRVYPSGLRTDSSNYNPQEHWNVGCQMVAMNMQT
gamma1-human 650 EMRLSEPVPQTNAHESKEWYHASLTRAQAEHMLMRVPRDGAFLVRKRNEP
gamma2-human 628 ELRLTDPVPNPNPHESKPWYYDSLSRGEAEDMLMRIPRDGAFLIRKREGS

zeta-monkeyA 471 PGLPMDL.NGKFLDNGGSGYILKPHFLRESESYFNPSN-IKGSMPITLTI
zeta-human 438 PGLPMDL.NGKFLDNGGSGYILKPHFLRESKSYFNPSN-IKEGMPITLTI
zeta-mouse 475 PGLPMDL.NGKFLDNGGSGYILKPDILRDTTLGFNPNEPEYDDHPVTLTI
zeta-rat 475 PGLPMDL.NGKFLDNGGSGYVLKPDFLRDTTLGFNPNEPEGDGHPVTLTI
beta1-human 624 MDLAMQINMGMYEYNGKSGYRLKPEFMRR---PDKHFDPFTEGIVDGIVA
beta3-mouse 680 LDLPMQLNAGVFEYNGRSGYLLKPEFMRR---PDKSFDPFTEVIVDGIVA
beta2-human 631 MDLPMQQNMAVFEFNGQSGYLLKHEFMRR---PDKQFNPFSVDRIDVVVA
beta4-mouse 654 PDLAMQLNQGKFEYNGSCGYLLKPDFMRR---PDRTFDPFSETPVDGVIA
epsilon-human 1819 DDLPLHLNAAMFEANGGCGYVLKPPVLWDKNCPMYQKFSPLERDLDSMDP
delta1-human 582 PGPEMDVYQDRFQDNGACGYVLKPAFLRDPNGTFNPR---ALAQGPWWAR
delta3-human 564 PGYEMDLNAGRFLVNGQCGYVLKPACLRQPDSTFDP--------EYPGPPR
delta2-bovine 579 AGLEMDLCDGLFRQNAGCGYVLKPDFLRDAQSSFHPERPISPFKAQTLII
delta4-rat 592 AGSAMDICDGLFRQNGGSGYVLKPEFLRDTQSSFNPMKPVSLYKAQILVV
gamma1-human 700 NSYAISFRAEGKIKHCRVQQEGQTVMLGN-SEFDSLVDLISYYEKHPLYR
gamma2-human 678 DSYAITFRARGKVKHCRINRDGRHFVLGTSAYFESLVELVSYYEKHSLYR

zeta-monkeyA 520 R[?][?]GIQ[?]PLT------HSSSNKGDTLV[?][?][?]VEG[?][?]--NLQM[?]QQT[?]VIKK
zeta-human 487 R[?][?]GIQ[?]PLT------HSSSNKGDSLV[?][?][?]VEG[?][?]--NLQM[?]QQT[?]VIKK
zeta-mouse 525 R[?][?]GIQ[?]PVS------SSS-NTPDIVV[?][?][?]VYG[?][?]--NLHV[?]QQT[?]VVKN
zeta-rat 525 R[?][?]GIQ[?]PVH------VPS-NTSDIIV[?][?][?]VYG[?][?]--NLHM[?]QQS[?]AVKN
beta1-human 671 NTLSVKIISGQ------FLSDKKVGTYVEVDMFGLPVDTRRKAFKTKTSQG
beta3-mouse 727 NALRVKVISGQ------FLSDKKVGIYVEVDMFGLPVDTR-RKYRTRTSQG
beta2-human 678 TTLSITVISGQ------FLSERSVRTYVEVELFGLPGDPKRRYRTKLSPST
beta4-mouse 701 ATCSVQVISGQ------FLSDKKIGTYVEVDMYGLPTDTIRKEFRTRMVMN
epsilon-human 1869 AVYSLTIVSGQ------NVCPSNSMGSPCIEVDVLGMPLDSCHFRTRPIHR
delta1-human 629 KRLNIRVISGQQLPKVNKNK-NSIVDPKVTVEIHGVSRDVASRQTAVITN
delta3-human 607 TTLSIQVLTAQQLPKLNAEKPHSIVDPLVRIEIHGVPADCARQETDYVLN
delta2-bovine 629 QEPWLQVISGQQLPKVDNTKEQSIVDPLVRVEIFGVRPDTTRQETSYVEN
delta4-rat 642 QV-----ISGQRLPKVDKTKETTVVDPLVRVELYGVPEDTKQQETSYVEN
gamma1-human 749 KMKLRYPINEEALEK--IGTAEPDYGALYEGRNPGFYVEANPMPTFKCAV
gamma2-human 728 KMRLRYPVTPELLER--YNTER-DINSLYDVSRMYVDPSEINPSMPQRTV

zeta-monkeyA 563 NAFSPRWNETFTFIIHVPELAL[?]RF[?]VESQG-LIAGNEFLGQYTLPLLCM
zeta-human 530 NAFSPRWNETFTFIIHVPELAL[?]RF[?]VEGQG-LIAGNEFLGQYTLPLLCM
zeta-mouse 567 NAFSPKWNETFTFLLQVPELAL[?]RF[?]VETQQGLLSGNELLGQYTLPVLCM
zeta-rat 567 NAFSPRWNETFTFLLQVPELAL[?]RF[?]VETQG-FLSGNELLGQYTLPVLCM
beta1-human 716 NAVNPVWEEEPIVFKKVVLPTLACLRIAVYE---EGGKFIGHRILPVQAI
beta3-mouse 771 NSFNPVWDEEPFDFPKVVLPTLASLRIAAFE---EGGKFVGHRILPVSAI
beta2-human 723 NSINPVWKEEPFVFEKILMPELASLRVAVME---EGNKFLGHRIIPINAL
beta4-mouse 746 NGLNPVYNEESFVFRKVILPDLAVLRIAVYD---DNNKLIGQRILPLDGL
epsilon-human 1914 NTLNPMWN-EQFLFRVHFEDLVFLRFAVVEN---NSSAVTAQRIIPLKAL
delta1-human 678 NGFNPWWDTEFAFEVVVPDLALIRFLVEDYD-ASSKNDFIGQSTIPLNSL
delta3-human 657 NGFNPRWGQTLQFQLRAPELALVRFVVEDYD-ATSPNDFVGQFTLPLSSL

delta2-bovine 679 NGFNLYWGGTLCERL-----------------------
delta4-rat 687 NGINFYWGETFYFQIQVPELAMLRFVVKDYS-RTSRNNFIGQYTLPWTCM
gamma1-human 797 KALFDYKAQREDELTFIKSAIIQNVEKQEGGWWRGDYGGKKQLWFPSNYV
gamma2-human 775 KALYDYKAKRSDELSFCRGALIHNVSKEPGGWWKGDYGTRIQQYFPSNYV

zeta-monkeyA 612 NKGYR-VPLFSRMGESLEPASLFV-WYVR-----------------
zeta-human 579 NKGYR-IPLFSPMGESLEPASLFV-WYVR-----------------
zeta-mouse 617 NKGYR-VPLFSKSGANLEPSSLFI-WYFRE----------------
zeta-rat 616 NKGYR-VPLFSKSGANLEPSSLFI-WYYRE----------------
beta1-human 763 RPGYHYICLRNERNQPLTLPAVFVYIEVKDYVPDTYADVIEALSNPIRYV
beta3-mouse 818 RSGYHYVCLRNEANQPLCLPALLIYTEASDYIPDDHQDYAEALINPIKHV
beta2-human 770 NSGYHHICLHSESNMPLTMPALFIFLEMKDYIPGAWADLTVALANPIKFF
beta4-mouse 793 QAGYRHISLRNEGNKPLSLPTIFCNIVLKTYVPDGFGDIVDALSDPKKFL
epsilon-human 1960 KRGYRHLQLRNLHNEVLEISSLFINSRRMEENSSGNTMSASSMFN-----
delta1-human 727 KQGYRHVHLMSKNGDQHPSATLFVKISLQD------------------
delta3-human 706 KQGYRHIHLLSKDGASLSPATLFIQIRIQRS-----------------
delta2-bovine 728 QQGYRHIHLLSKDGLSLHPASIFVHICTQEVSEEAES-----------
delta4-rat 736 KHGYRHVSLLSEDGTSLHPASIFVYTCMQEDLDMDEP-----------
gamma1-human 847 EEMVNPVALEPEREHLDENSPLGDLLRGVLDVPACQIAIRPEGKNNRLFV
gamma2-human 825 EDISTADFEELEKQIIEDN-PLGSLCRGILDLNTYNVVKAPQGKNQKSFV

zeta-monkeyA --------------------------------------------------
zeta-human --------------------------------------------------
zeta-mouse --------------------------------------------------
zeta-rat --------------------------------------------------
beta1-human 813 NLMEQRAKQLAALTLEDEEEVKKEADPGETPSEAPSEARTTPAENGVNHT
beta3-mouse 868 SLMDQRAKQLAALIGESEAQASTETYQETPCQQPGSQLPSNPTPNPLDAS
beta2-human 820 SAHDTKSVKLKEAMG-------------------GLPEKPFPLASPVASQ
beta4-mouse 843 SITEKRADQMRAMGIETSDIADVPSDTS----KNDKKGKANPAKANVTPQ
epsilon-human 2005 -----------------------------------TEERKCLQTHRVTVH
delta1-human --------------------------------------------------
delta3-human --------------------------------------------------
delta2-bovine --------------------------------------------------
delta4-rat --------------------------------------------------
gamma1-human 897 FSISMASVAHWSLDVAADSQEELQDWVKKIREVAQTADARLTEGKIMERR
gamma2-human 874 FILEPKEQGDPPVEFATDRVEELFEWFQSIREITWKIDSKENNMKYWEKN

zeta-monkeyA --------------------------------------------------
zeta-human --------------------------------------------------
zeta-mouse --------------------------------------------------
zeta-rat --------------------------------------------------
beta1-human 863 TTLTPKPPSQALHSQPAPGSVKAPAKTEDLIQSVLTEVEAQTIEELKQQK
beta3-mouse 918 PRWPPGPTTSSTSSSLSS-----PGQRDDLIASILSEVTPTPLEELRSHK
beta2-human 851 VNGALAPTSNGSPAARAG--------AREEAMKEAAEPRTASLEELRELK
beta4-mouse 889 SSSELRPTTTAALGSGQE------------AKKGIELIPQVRIEDLKQMK
epsilon-human 2020 GVPGPEPFTVFTINGGTK----------------AKQLLQQILTNEQDIK
delta1-human --------------------------------------------------
delta3-human --------------------------------------------------
delta2-bovine --------------------------------------------------
delta4-rat --------------------------------------------------
gamma1-human 947 KKIALELSELVVYCRPVPFDEEKIGTERACYRDMSSFPETKAEKYVNKAK
gamma2-human 924 QSIAIELSDLVVYCKPT--SKTKDNLENPDFREIRSFVETKADSIIRQKP

zeta-monkeyA --------------------------------------------------
zeta-human --------------------------------------------------
zeta-mouse --------------------------------------------------
zeta-rat --------------------------------------------------
beta1-human 913 SFVKLQKKHYKEMKDLVKRHHKKTTDLIKEHTTKYNEIQNDYLRRRAALE
beta3-mouse 963 AMVKLRSRQDRDLRELHKKHQRKAVALTRRLLDGLAQARAEGKCRPSPSA
beta2-human 893 GVVKLQRRHEKELRELERRGARRWEELLQRGAAQLAELGPPGVGGVGACK
beta4-mouse 927 AYLKHLKKQQKELNSLKKKHAKEHSTMQKLHCTQVDKIVAQYDKEKSTHE
epsilon-human 2054 PVTTDYFLMEEKYFISKEKNECRKQPFQRAIGPEEEIMQILSSWFPEEGY
delta1-human --------------------------------------------------
delta3-human --------------------------------------------------

delta2-bovine -------------
delta4-rat --------------------------------------------------
gamma1-human 997 GKKFLQYNRLQLSRIYPKGQRLDSSNYDPLPMWICGSQLVALNFQTPDKP
gamma2-human 972 -VDLLKYNQKGLTRVYPKGQRVDSSNYDPFRLWLCGSQMVALNFQTADKY

zeta-monkeyA --------------------------------------------------
zeta-human --------------------------------------------------
zeta-mouse --------------------------------------------------
zeta-rat --------------------------------------------------
beta1-human 963 KSAKKDSKKKSEPSSPDHGSSTIEQDLAALDAEMTQKLIDLKDKQQQQLL
beta3-mouse 1013 LGKATNSEDVKEE-------------------EEAKQYREFQNRQVQSLL
beta2-human 943 LGPGKGSRKKRSLPREESAG----AAPGEGPEGVDGRVRELKDRLELELL
beta4-mouse 977 KILEKAMKKKGGS---------------------------NCLEIKKET
epsilon-human 2104 MG-------------------------------------------RIVLK
delta1-human --------------------------------------------------
delta3-human --------------------------------------------------
delta2-bovine --------------------------------------------------
delta4-rat --------------------------------------------------
gamma1-human 1047 MQMNQALFMTGRHCGYVLQPSTMRDEAFDPFDKSSLRGLEPCAISIEVLG
gamma2-human 1021 MQMNHALFSLNGRTGYVLQPESMRTEKYDPMPPESQR-KILMTLTVKVLG

zeta-monkeyA --------------------------------------------------
zeta-human. --------------------------------------------------
zeta-mouse --------------------------------------------------
zeta-rat --------------------------------------------------
beta1-human 1013 NLRQEQYYSEKYQKREHIKLLIQKLTDVAEECQNNQLKKLKEICEKEKKE
beta3-mouse 1044 ELREAQADVETKRKLEHLRQAHQRLKEVVLDTHTTQFKRLKELNEREKKE
beta2-human 989 RQGEEQYECVLKRKEQHVAEQISKMMELAREKQAAELKALKETSENDTKE
beta4-mouse 999 EIKIQTLTTDHKSKVKEIVAQHTKEWSEMINTHSAEEQEIRDLHLSQQCE
epsilon-human 2111 TQQENLEEKNIVQDDKEVILSSEEESFFVQVHDVSPEQPRTVIKAPRVST
delta1-human --------------------------------------------------
delta3-human --------------------------------------------------
delta2-bovine --------------------------------------------------
delta4-rat --------------------------------------------------
gamma1-human 1097 ARHLPKNGRGIVCPFVEIEVAGAEYDSTKQKTEFVVDNGLNPVWP--AKP
gamma2-human 1070 ARHLPKLGRSIACPFVEVEICGAEYGNNKFKTTVVNDNGLSPIWAPTQEK

zeta-monkeyA --------------------------------------------------
zeta-human --------------------------------------------------
zeta-mouse --------------------------------------------------
zeta-rat --------------------------------------------------
beta1-human 1063 LKKKMDKKRQEKIT--EAKSKDKSQMEEEKTEMIRSYIQEVVQYIKRLEE
beta3-mouse 1094 LQKILDRKRNNSIS--EAKTREKHKKEVELTEINRRHITESVNSIRRLEE
beta2-human 1039 MKKKLETKRLERIQGMTKVTTDKMAQERLKREINNSHIQEVVQVIKQMTE
beta4-mouse 1049 LLRKLLINAHEQQTQQLKLSHDRESKEMRAHQAKISMENSKAISQDKSIK
epsilon-human 2161 AQDVIQQTLCKAKYSYSILSNPNPSDYVLLEEVVKDTTNKKTTTPKSSQR
delta1-human --------------------------------------------------
delta3-human --------------------------------------------------
delta2-bovine --------------------------------------------------
delta4-rat --------------------------------------------------
gamma1-human 1145 FHFQISNPEFAFLRFVVYEEDMFSDQNFLAQATFPVKGLKTGYRAVPLKN
gamma2-human 1120 VTFEIYDPNLAFLRFVVYEEDMFSDPNFLAHATYPIKAVKSGFRSVPLKN

zeta-monkeyA --------------------------------------------------
zeta-human --------------------------------------------------
zeta-mouse --------------------------------------------------
zeta-rat --------------------------------------------------
beta1-human 1111 AQSKRQEKLVEKHKEIRQQILDEKPKLQVELEQEYQDKFKRLPLEILEFV
beta3-mouse 1142 AQKQRHERLVAGQQQVLQQLEEEEPKLLAQLTQECQEQRERLPQEIRRCL
beta2-human 1089 NLERHQEKLEEKQAACLEQIREMEKQFQKEALAEYEARMKGLEAEVKESV
beta4-mouse 1099 NKAERERRVRELNSSNTKKFLEERKRLAMKQSKEMDQLKKVQLEHLEFLE
epsilon-human 2211 VLLDQECVFQAQSKWKGAGKFILKLKEQVQASREDKKKGISFASELKKLT
delta1-human --------------------------------------------------
delta3-human --------------------------------------------------

delta2-bovine ----------------------------------------
delta4-rat --------------------------------------------
gamma1-human 1195 NYSEDLELASLLIKIDIFPAKENGDLSPFSGTSLRERGSDASGQLFHGRA
gamma2-human 1170 GYSEDIELASLLVFCEMRPVLESEEELYSSCRQLRRRQEELNNQLFLYDT

zeta-monkeyA --------------------------------------------------
zeta-human --------------------------------------------------
zeta-mouse --------------------------------------------------
zeta-rat --------------------------------------------------
beta1-human 1161 QEAMKGKISEDSNHGSAPLSLSSDPGKVNHKTPSSEELGGDIPGKEFDTP
beta3-mouse 1192 LGETAEGLGDGPLVACASNGHAPGSGGHLSSADSESQEENTQL-------
beta2-human 1139 RACLRTCFPSEAKDKPERACECPPELCEQDPLIAKADAQESRL-------
beta4-mouse 1149 KQNEQAKEMQQMVKLEAEMDRRPATVV-----------------------
epsilon-human 2261 KSTKQPRGLTSPSQLLTSESIQTKEEKPVGGLSPVTQWITDSD-------
delta1-human --------------------------------------------------
delta3-human --------------------------------------------------
delta2-bovine --------------------------------------------------
delta4-rat --------------------------------------------------
gamma1-human 1245 REGSFESRYQQPFEDFRISQEHLADHFDSRERRAPRRTRVNGDNRL----
gamma2-human 1220 HQNLRNANRDALVKEFSVNENHSSCTRRNATRG-----------------

zeta-monkeyA -
zeta-human -
zeta-mouse -
zeta-rat -
beta1-human 1211 L
beta3-mouse -
beta2-human -
beta4-mouse -
epsilon-human -
delta1-human -
delta3-human -
delta2-bovine -
delta4-rat -
gamma1-human -
gamma2-human -

## Claims

1. An isolated, purified or recombinant nucleic acid molecule comprising:
a nucleic acid molecule encoding a PLC-zeta; PLCζ polypeptide capable of
triggering calcium oscillations in oocytes and **characterized by** SEQ ID NO: 4, which
sequence is the mouse PLC-zeta; PLCζ, or a sequence homologous thereto, or a
sequence which hybridises thereto under stringent conditions, or SEQ ID NO: 10,
which sequence is the rat PLC-zeta; PLCζ, or a sequence homologous thereto, or a
sequence which hybridises thereto under stringent conditions.

2. A nucleic acid molecule according to claim 1, wherein the sequence is a DNA or RNA, including a cDNA or MRNA, sequence.

3. An isolated, purified or recombinant polypeptide, encoded by a nucleic acid molecule according to any preceding claim.

4. An isolated, purified or recombinant polypeptide, comprising SEQ ID NO: 2, being the mouse PLC-zeta; PLCζ, or a sequence homologous thereto.

5. An isolated, purified or recombinant polypeptide, comprising SEQ ID NO: 11, being the rat PLC-zeta; PLCζ, or a sequence homologous thereto.

6. A PLC-zeta protein, other than human PLC-zeta protein, **characterised by** exhibiting the following properties:
(a) An amino acid sequence comprising in the range of from 600 to 720, preferably 600 to 699, more preferably 600 to 650, amino acid residues;
(b) A domain sequence comprising the EF hand, X, Y, and C2 domains but absent the PH domain; and
(c) At least five consecutive amino acid residues from a conserved region, which region is selected from:
(i)QDDFRGGKI (11-19);
(ii) LLEKLD (27-32); and
(iii) QGRIT (52-56) in the EF1 domain;
(iv) ENRKIL (82-87); and
(v) FLTQEQY (95-101) in the EF2 domain;
(vi) YQQFNE (403-408) in the Y domain; and
(vii) TLTIR (516-520);
(viii) ISGIQLP (522-528); and
(ix) LCMNKGYRR (609-617) in the C2 domain,
wherein the residues are denoted by their conventional single letter codes and the numbers in parentheses refer to the AB070108 (monkey A) reference sequence.

7. A polypeptide or protein according to any of claims 3 to 6, having a molecular weight in the range of from 70 to 75 kD, as determined by mass spectrometry.

8. A recombinant non-human PLC-zeta protein, capable of generating cytoplasmic calcium oscillations (CCOs) when introduced into a mammalian cell.

9. A recombinant non-human mRNA encoding PLC-zeta protein, capable of generating cytoplasmic calcium oscillations (CCOs) when introduced into a mammalian egg.

10. A recombinant non-mammalian PLC-zeta protein, capable of generating cytoplasmic calcium oscillations (CCOS) when introduced into a non-mammalian cell.

11. A recombinant non-mammalian mRNA encoding PLC-zeta protein, capable of generating cytoplasmic calcium oscillations (CCOs) when introduced into a non-mammalian egg.

12. A method for the preparation of a polypeptide or protein according to any of claims 3 to 8 and 10, which method comprises:
(a) isolation and/or purification thereof from mammalian sperm; or
(b) expression of a nucleic acid molecule encoding the polypeptide and, optionally, isolation and/or purification of the resulting polypeptide.

13. A method according to claim 12 wherein expression of the nucleic acid molecule encoding said polypeptide or protein involves the use of one or more of the following oligonucleotides:
Reverse TriplEx primer: 5' CTC GGG AAG CGC GCC ATT GTG TTG GT 3' (26mer).
Forward mouse primer: 5' GCT AAC GCG TCA GTT ACA TGC GTC ACT C 3' (28mer)
Reverse T7 primer: 5' GTA ATA CGA CTC ACT ATA GGG C 3' (22mer)
Forward mouse primer: 5' GCT AAC GCG TCA GTT ACA TGC GTC ACT C 3' (28mer)
Reverse mouse primer: 5' ATC ATG GAA AGC CAA CTT C 3' (19mer)

14. A vector comprising a recombinant construct comprising a nucleic acid molecule according to any of claims 1 or 2.

15. A cultured host cell transformed or transfected by a vector according to claim 14.

16. A cell, plasmid, virus, non human live organism or other vehicle that has been genetically-or protein-engineered to produce a polypeptide or protein according to any of claims 3 to 8 and 10, said cell, plasmid, virus, live organism or other vehicle having incorporated expressibly therein a nucleic acid molecule according to any of claims 1 or 2.

17. The use of a polypeptide encoded by a nucleic acid molecule according to any of claims 1 or 2, or the nucleic acid molecule itself, in the manufacture of a medicament for use in medicine, including gene therapy.

18. A pharmaceutical formulation comprising a polypeptide or protein according to any of claims 3 to 8 and 10, and a pharmaceutically acceptable carrier therefor.

19. A pharaceutical formulation comprising a nucleic acid molecule according to claims 1 or 2, and a pharmaceutically acceptable carrier therefore.

20. A diagnostic method for determining the fertility status of a mammal, which method comprises determining the amount of a protein according to SEQ ID NO: 1 human PLC-zeta or a sequence 70% homologous thereto, or nucleic acid sequence according to SEQ ID NO: 3 human PLC-zeta or a sequence 70% homologous thereto, present or absent in a test sample obtained from the mammal, which amount is indicative of the level of fertility of the mammal.

21. A diagnostic or screening method comprising:
(a) determining the genetic code of a test sample comprising a nucleic acid molecule of the mammalian PLC-zeta (PLCζ gene obtained from an individual; and
(b) comparing a region of the code obtained from the test sample with the corresponding region of a wild type mammalian PLCζ nucleic acid sequence, such as [SEQ ID NO: 3 or 4] to determine if there are any variations;
whereby a variation in the sample code relative to the predetermined sequence is indicative of a condition, such as lowered fertility or infertility, associated with disruption in calcium oscillation patterns that are a prerequisite to normal biological function absent in the condition.

22. A screening method for screening an individual suspected of a fertility problem, which screening method comprises the steps of:
(a) analysing a test sample comprising a nucleic acid molecule of the human PLC-zeta (PLCζ gene or an amino acid sequence encoded thereby obtained from the individual; said analysis involving
(b) analysing the test sample for the presence of a variant of the human PLCζ gene or an amino acid sequence encoded thereby or for the presence of one or more surrogate markers that are indicative of or correlated to the presence of a variant of the human PLCζ gene or an amino acid sequence encoded thereby,
wherein the variant of the human PLCζ gene or an amino acid sequence encoded thereby exhibits at least one variation when compared to the wild type PLCζ sequence.

23. A method according to any of claims 20 to 22, wherein the test sample comprises genomic DNA.

24. An antibody raised to a polypeptide according to any of claims 3 to 8 and 10.

25. An antibody according to claim 24, which is a monoclonal antibody raised to a polypeptide according to any of claims 3 to 8 and 10.

26. A diagnostic or screening kit for determining the fertility status of an individual, which kit comprises:
(a) an oligonucleotide having a nucleic acid sequence corresponding to a region of a PLC-zeta (PLCζ) variant, which region incorporates at least one variation from the corresponding wild-type PLCζ gene sequence; and/or
(b) an oligonucleotide having a nucleic acid sequence corresponding to the wild-type PLCζ gene sequence in the region specified in (a); and/or
(c) an oligonucleotide having a nucleic acid sequence corresponding to a specific region of the wild-type PLCζ gene sequence, which specific region comprises a sequence not otherwise present in the genomic DNA of the mammal; and/or
(d) antibodies, such as monoclonal antibodies, raised to any oligonucleotide specific to any one of (a) to (c) above; and, optionally,
(e) one or more reagents suitable for carrying out PCR for amplifying desired regions of the individual's DNA.

27. A kit according to claim 26, wherein any of kit components (a) to (c) comprise(s) a plurality of said oligonucleotides immobilised on a solid support.

## Patentansprüche

1. Isoliertes, gereinigtes oder rekombinantes Nukleinsäuremolekül, umfassend:
ein Nukleinsäuremolekül, das ein PLC-zeta; PLCζ-Polypeptid, das Calciumoszillationen in Oozyten auslösen kann, kodiert und durch SEQ ID NO:4 charakterisiert ist, wobei die Sequenz das Maus-PLC-zeta; PLCζ oder eine dazu homologe Sequenz oder eine Sequenz ist, die unter stringenten Bedingungen daran hybridisiert, oder durch SEQ ID NO:10 charakterisiert ist, wobei die Sequenz das Ratte-PLC-zeta; PLCζ oder eine dazu homologe Sequenz oder eine Sequenz ist, die unter stringenten Bedingungen daran hybridisiert.

2. Nukleinsäuremolekül nach Anspruch 1, wobei die Sequenz eine DNA- oder RNA-, einschließlich eine cDNA- oder MRNA-, Sequenz ist.

3. Isoliertes, gereinigtes oder rekombinantes Polypeptid, das durch ein Nukleinsäuremolekül nach einem der vorhergehenden Ansprüche kodiert wird.

4. Isoliertes, gereinigtes oder rekombinantes Polypeptid, umfassend SEQ ID NO:2, wobei es sich um das Maus-PLC-zeta; PLCζ oder eine dazu homologe Sequenz handelt.

5. Isoliertes, gereinigtes oder rekombinantes Polypeptid, umfassend SEQ ID NO:11, wobei es sich um das Ratte-PLC-zeta; PLCζ oder eine dazu homologe Sequenz handelt.

6. PLC-zeta-Protein, das von menschlichem PLC-zeta-Protein verschieden ist, **dadurch gekennzeichnet, dass** es die folgenden Eigenschaften aufweist:
(a) Eine Aminosäuresequenz, die im Bereich von 600 bis 720, vorzugsweise 600 bis 699, mehr bevorzugt 600 bis 650 Aminosäurereste umfasst,
(b) eine Domänensequenz, welche die EF-Hand-, X-, Y- und C2-Domänen, jedoch nicht die PH-Domäne, umfasst, und
(c) mindestens fünf aufeinander folgende Aminosäurereste von einer konservierten Region, wobei die Region ausgewählt ist aus:
(i) QDDFRGGKI (11-19),
(ii) LLEKLD (27-32) und
(iii) QGRIT (52-56) in der EF1-Domäne,
(iv) ENRKIL (82-87) und
(v) FLTQEQY (95-101) in der EF2-Domäne,
(vi)YQQFNE (403-408) in der Y-Domäne und
(vii) TLTIR (516-520),
(viii) ISGIQLP (522-528) und
(ix) LCMNKGYRR (609-617) in der C2-Domäne,
wobei die Reste mit ihren herkömmlichen Einbuchstabencodes bezeichnet sind und sich die Zahlen in Klammern auf die AB070108 (Affe A)-Referenzsequenz beziehen.

7. Polypeptid oder Protein nach einem der Ansprüche 3 bis 6, das ein Molekulargewicht im Bereich von 70 bis 75 kD, bestimmt mittels Massenspektrometrie, aufweist.

8. Rekombinantes, nicht-menschliches PLC-zeta-Protein, das zytoplasmatische Calciumoszillationen (CCO's) erzeugen kann, wenn es in eine Säugerzelle eingebracht wird.

9. Rekombinante, nicht-menschliche mRNA, die PLC-zeta-Protein kodiert, das zytoplasmatische Calciumoszillationen (CCO's) erzeugen kann, wenn es in ein Säugerei eingebracht wird.

10. Rekombinantes, nicht-Säuger-PLC-zeta-Protein, das zytoplasmatische Calciumoszillationen (CCO's) erzeugen kann, wenn es in eine nicht-Säugerzelle eingebracht wird.

11. Rekombinante, nicht-Säuger-mRNA, die PLC-zeta-Protein kodiert, das zytoplasmatische Calciumoszillationen (CCO's) erzeugen kann, wenn es in ein nicht-Säugerei eingebracht wird.

12. Verfahren zur Herstellung eines Polypeptids oder Proteins nach einem der Ansprüche 3 bis 8 und 10, wobei das Verfahren umfasst:
(a) Isolieren und/oder Reinigen desselben ausgehend von Säugersperma oder
(b) Expression eines Nukleinsäuremoleküls, welches das Polypeptid kodiert, und gegebenenfalls Isolieren und/oder Reinigen des resultierenden Polypeptids.

13. Verfahren nach Anspruch 12, bei dem die Expression des Nukleinsäuremoleküls, welches das Polypeptid oder Protein kodiert, die Verwendung von einem oder mehreren der folgenden Oligonukleotide umfasst:
TriplEx-Rückwärtsprimer: 5' CTC GGG AAG CGC GCC ATT GTG TTG GT 3' (26mer)
Maus-Vorwärtsprimer: 5' GCT AAC GCG TCA GTT ACA TGC GTC ACT C 3' (28mer)
T7-Rückwärtsprimer: 5' GTA ATA CGA CTC ACT ATA GGG C 3' (22mer)
Maus-Vorwärtsprimer: 5' GCT AAC GCG TCA GTT ACA TGC GTC ACT C 3' (28mer)
Maus-Rückwärtsprimer: 5' ATC ATG GAA AGC CAA CTT C 3' (19mer)

14. Vektor, der ein rekombinantes Konstrukt umfasst, das ein Nukleinsäuremolekül nach Anspruch 1 oder 2 umfasst.

15. Kultivierte Wirtszelle, die durch einen Vektor nach Anspruch 14 transformiert oder transfiziert worden ist.

16. Zelle, Plasmid, Virus, nicht-menschlicher lebender Organismus oder anderes Vehikel, die bzw. das bzw. der genetisch oder mittels Proteinen so konstruiert worden ist, dass sie bzw. es bzw. er ein Polypeptid oder Protein nach einem der Ansprüche 3 bis 8 und 10 erzeugt, wobei die Zelle, das Plasmid, das Virus, der lebende Organismus oder das andere Vehikel ein exprimierbar darin eingebrachtes Nukleinsäuremolekül nach Anspruch 1 oder 2 aufweist.

17. Verwendung eines durch ein Nukleinsäuremolekül nach Anspruch 1 oder 2 kodierten Polypeptids oder des Nukleinsäuremoleküls selbst bei der Herstellung eines Medikaments zur Verwendung in der Medizin, einschließlich einer Gentherapie.

18. Pharmazeutische Formulierung, die ein Polypeptid oder Protein nach einem der Ansprüche 3 bis 8 und 10 und einen pharmazeutisch verträglichen Träger dafür umfasst.

19. Pharmazeutische Formulierung, die ein Nukleinsäuremolekül nach Anspruch 1 oder 2 und einen pharmazeutisch verträglichen Träger dafür umfasst.

20. Diagnoseverfahren zur Bestimmung des Fruchtbarkeitsstatus eines Säugers,
wobei das Verfahren das Bestimmen der Menge eines Proteins gemäß SEQ ID NO:1, menschliches PLC-zeta, oder einer Sequenz mit 70 % Homologie dazu, oder einer Nukleinsäuresequenz gemäß SEQ ID NO:3, menschliches PLC-zeta, oder einer Sequenz mit 70 % Homologie dazu, die in einer Testprobe, die von dem Säuger erhalten worden ist, vorliegt oder abwesend ist, umfasst, wobei die Menge das Fruchtbarkeitsniveau des Säugers anzeigt.

21. Diagnose- oder Screeningverfahren, umfassend:
(a) Bestimmen des genetischen Codes einer von einem Lebewesen erhaltenen Testprobe, die ein Nukleinsäuremolekül des Säuger-PLC-zeta (PLCζ)-Gens umfasst
und
(b) Vergleichen einer Region des von der Testprobe erhaltenen Codes mit der entsprechenden Region einer Wildtyp-Säuger-PLCζ-Nukleinsäuresequenz, wie z.B. [SEQ ID NO:3 oder 4], um zu bestimmen, ob irgendwelche Variationen vorliegen, wodurch eine Variation in dem Code der Probe bezogen auf die vorgegebene Sequenz einen Zustand anzeigt, wie z.B. eine verminderte Fruchtbarkeit oder Unfruchtbarkeit, der mit einer Unterbrechung der Calciumoszillationsstrukturen zusammenhängt, die Voraussetzung für eine normale biologische Funktion sind, welche in dem Zustand nicht vorliegt.

22. Screeningverfahren zum Screenen eines Lebewesens, bei dem vermutet wird, dass ein Fruchtbarkeitsproblem vorliegt, wobei das Screeningverfahren die Schritte umfasst:
(a) Analysieren einer von dem Lebewesen erhaltenen Testprobe, die ein Nukleinsäuremolekül des menschlichen PLC-zeta (PLCζ)-Gens oder eine dadurch kodierte Aminosäuresequenz umfasst, wobei die Analyse umfasst:
(b) Analysieren der Testprobe bezüglich der Gegenwart einer Variante des menschlichen PLCζ-Gens oder einer dadurch kodierten Aminosäuresequenz oder bezüglich der Gegenwart eines oder mehrerer Surrogatmarker(s), welche(r) die Gegenwart einer Variante des menschlichen PLCζ-Gens oder einer dadurch kodierten Aminosäuresequenz anzeigt bzw. anzeigen oder mit dieser korreliert ist bzw. sind,
wobei die Variante des menschlichen PLCζ-Gens oder einer **dadurch** kodierten Aminosäuresequenz mindestens eine Variation zeigt, wenn sie mit der Wildtyp-PLCζ-Sequenz verglichen wird.

23. Verfahren nach einem der Ansprüche 20 bis 22, bei dem die Testprobe genomische DNA umfasst.

24. Antikörper, der gegen ein Polypeptid nach einem der Ansprüche 3 bis 8 und 10 gerichtet ist.

25. Antikörper nach Anspruch 24, bei dem es sich um einen monoklonalen Antikörper handelt, der gegen ein Polypeptid nach einem der Ansprüche 3 bis 8 und 10 gerichtet ist.

26. Diagnose- oder Screeningkit zum Bestimmen des Fruchtbarkeitsstatus eines Lebewesens, wobei das Kit umfasst:
(a) Ein Oligonukleotid mit einer Nukleinsäuresequenz, die einer Region einer PLC-zeta (PLCζ)-Variante entspricht, wobei die Region mindestens eine Variation von der entsprechenden Wildtyp-PLCζ-Gensequenz umfasst, und/oder
(b) ein Oligonukleotid mit einer Nukleinsäuresequenz, die der Wildtyp-PLCζ-Gensequenz in der in (a) spezifizierten Region entspricht, und/oder
(c) ein Oligonukleotid mit einer Nukleinsäuresequenz, die einer spezifischen Region der Wildtyp-PLCζ-Gensequenz entspricht, wobei die spezifische Region eine Sequenz umfasst, die sonst nicht in der genomischen DNA des Säugers vorliegt, und/oder
(d) Antikörper, wie z.B. monoklonale Antikörper, die gegen jedwedes Oligonukleotid gerichtet sind, spezifisch für jedwedes der vorstehenden (a) bis (c), und gegebenenfalls
(e) ein oder mehrere Reagenz(ien), das bzw. die zur Durchführung einer PCR zur Amplifizierung gewünschter Regionen der DNA des Lebewesens geeignet ist bzw. sind.

27. Kit nach Anspruch 26, wobei jedwede der Kitkomponenten (a) bis (c) eine Mehrzahl der Oligonukleotide umfasst, die auf einem festen Träger immobilisiert sind.

## Revendications

1. - Molécule d'acide nucléique isolée, purifiée ou recombinante, comprenant :
une molécule d'acide nucléique codant pour un polypeptide PLC-zeta; PLCζ capable de déclencher des oscillations de calcium dans des ovocytes et **caractérisée par** SEQ ID NO :4, laquelle séquence est la PLC-zeta; PLCζ de souris, ou une séquence homologue à celle-ci, ou une séquence qui s'hybride avec celle-ci dans des conditions stringentes, ou SEQ ID NO :10, laquelle séquence est la PLC-zeta; PLCζ de rat, ou une séquence homologue à celle-ci, ou une séquence qui s'hybride avec celle-ci dans des conditions stringentes.

2. - Molécule d'acide nucléique selon la revendication 1, dans laquelle la séquence est une séquence d'ADN ou d'ARN, comprenant un ADNc ou un ARNM.

3. - Polypeptide isolé, purifié ou recombinant, codé par une molécule d'acide nucléique selon l'une quelconque des revendications précédentes.

4. - Polypeptide isolé, purifié ou recombinant, comprenant SEQ ID NO: 2, qui est la PLC-zeta; PLCζ de souris, ou une séquence homologue à celle-ci.

5. - Polypeptide isolé, purifié ou recombinant, comprenant SEQ ID NO: 11, qui est la PLC-zeta; PLCζ de rat, ou une séquence homologue à celle-ci.

6. - Protéine PLC-zeta, autre que la protéine PCL-zeta humaine, **caractérisée par le fait qu'**elle présente les propriétés suivantes :
(a) une séquence d'acides aminés comprenant dans la plage de 600 à 720, de préférence 600 à 699, de façon davantage préférée, 600 à 650, résidus d'acide aminé ;
(b) une séquence de domaine comprenant les domaines main EF (EF hand), X, Y et C2, mais pas le domaine PH ; et
(c) au moins cinq résidus d'acide aminé consécutifs à partir d'une région conservée, laquelle région est choisie parmi :
(i) QDDFRGGKI (11-19) ;
(ii) LLEKLD (27-32) ; et
(iii) QGRIT (52-56) dans le domaine EF1 ;
(iv) ENRKIL (82-87) ; et
(v) FLTQEQY (95-101) dans le domaine EF2 ;
(vi) YQQFNE (403-408) dans le domaine Y ; et
(vii) TLTIR (516-520) ;
(viii) ISGIQLP (522-528) ; et
(ix) LCMNKGYRR (609-617) dans le domaine C2,
les résidus étant désignés par leurs codes lettres uniques classiques et les nombres entre parenthèses se référant à la séquence de référence AB070108 (singe A).

7. - Polypeptide ou protéine selon l'une quelconque des revendications 3 à 6, ayant une masse moléculaire dans la plage de 70 à 75 kD, telle que déterminée par spectrométrie de masse.

8. - Protéine PLC-zeta non-humaine recombinante, capable de générer des oscillations de calcium cytoplasmique (CCO) lorsqu'elle est introduite dans une cellule de mammifère.

9. - Protéine PLC-zeta codant pour un ARNm non-humain, recombinante, capable de générer des oscillations de calcium cytoplasmique (CCO) lorsqu'elle est introduite dans un oeuf de mammifère.

10. - Protéine PLC-zeta non-mammifère, recombinante, capable de générer des oscillations de calcium cytoplasmique (CCO) lorsqu'elle est introduite dans une cellule non-mammifère.

11. - Protéine PLC-zeta codant pour un ARNm non-mammifère, recombinante, capable de générer des oscillations de calcium cytoplasmique (CCO) lorsqu'elle est introduite dans un oeuf non-mammifère.

12. - Procédé de préparation d'un polypeptide ou d'une protéine selon l'une quelconque des revendications 3 à 8 et 10, lequel procédé comprend :
(a) l'isolement et/ou la purification de celui-ci/celle-ci à partir de sperme de mammifère ; ou
(b) l'expression d'une molécule d'acide nucléique codant pour le polypeptide et, facultativement, l'isolement et/ou la purification du polypeptide résultant.

13. - Procédé selon la revendication 12, dans lequel l'expression de la molécule d'acide nucléique codant pour ledit polypeptide ou ladite protéine met en oeuvre l'utilisation d'un ou de plusieurs des oligonucléotides suivants :
Amorce TriplEx antisens : 5'CTC GGG AAG CGC GCC ATT GTG TTG GT 3' (26mer).
Amorce sens de souris : 5'GCT AAC GCG TCA GTT ACA TGC GTC ACT C 3' (28mer)
Amorce antisens T7 : 5'GTA ATA CGA CTC ACT ATA GGG C 3' (22mer)
Amorce sens de souris : 5'GCT AAC GCG TCA GTT ACA TGC GTC ACT C 3' (28mer)
Amorce antisens de souris : 5'ATC ATG GAA AGC CAA CTT C 3' (19muer).

14. - Vecteur comprenant un produit de construction recombinant comprenant une molécule d'acide nucléique selon l'une ou l'autre des revendications 1 ou 2.

15. - Cellule hôte mise en culture transformée ou transfectée par un vecteur tel que défini à la revendication 14.

16. - Cellule, plasmide, virus, organisme vivant non-humain ou autre véhicule qui a été génétiquement modifié ou à protéines génétiquement modifiées pour produire un polypeptide ou une protéine selon l'une quelconque des revendications 3 à 8 et 10, ladite cellule, ledit plasmide, ledit virus, ledit organisme vivant ou ledit autre véhicule ayant incorporé dans celle-ci/celui-ci de manière exprimable une molécule d'acide nucléique selon l'une ou l'autre des revendications 1 ou 2.

17. - Utilisation d'un polypeptide codé par une molécule d'acide nucléique selon l'une ou l'autre des revendications 1 ou 2, ou de la molécule d'acide nucléique elle-même, dans la fabrication d'un médicament pour une utilisation en médecine, comprenant la thérapie génique.

18. - Formulation pharmaceutique comprenant un polypeptide ou une protéine selon l'une quelconque des revendications 3 à 8 et 10, et un véhicule pharmaceutiquement acceptable pour celle-ci.

19. - Formulation pharmaceutique une molécule d'acide nucléique selon l'une ou l'autre des revendications 1 ou 2, et un véhicule pharmaceutiquement acceptable pour celle-ci.

20. - Procédé de diagnostic pour déterminer l'état de fertilité d'un mammifère, lequel procédé comprend la détermination de la quantité d'une protéine conforme à PLC-zeta humaine de SEQ ID NO: 1 ou à une séquence homologue à 70% à celle-ci, ou d'une séquence d'acide nucléique conforme à PLC-zeta humaine de SEQ ID NO: 3 ou à une séquence homologue à 70% à celle-ci, présente ou absente dans un échantillon de test obtenu à partir du mammifère, laquelle quantité est indicative du niveau de fertilité du mammifère.

21. - Procédé de diagnostic ou de criblage, comprenant :
(a) la détermination du code génétique d'un échantillon de test comprenant une molécule d'acide nucléique du gène de PLC-zeta(PLCζ) de mammifère obtenu d'un individu ;
et
(b) la comparaison d'une région du code obtenu à partir de l'échantillon de test avec la région correspondante d'une séquence d'acide nucléique PLCζ de mammifère de type sauvage, telle que [SEQ ID NO: 3 ou 4] pour déterminer s'il y a de quelconques variations ;
ce par quoi une variation dans le code d'échantillon par rapport à la séquence prédéterminée est indicative d'un état, tel qu'une fertilité diminuée ou une infertilité, associé à une rupture des motifs d'oscillation de calcium qui sont un pré-requis pour une fonction biologique normale absente dans l'état.

22. - Procédé de criblage pour cribler un individu suspecté d'avoir un problème de fertilité, lequel procédé de criblage comprend les étapes :
(a) d'analyse d'un échantillon de test comprenant une molécule d'acide nucléique du gène de PLC-zeta(PLCζ) humain ou une séquence d'acides aminés codée par celui-ci obtenu à partir de l'individu ;
ladite analyse mettant en jeu :
(b) l'analyse de l'échantillon de test pour déterminer la présence d'un variant du gène PLCζ humain ou d'une séquence d'acides aminés codée par celui-ci ou la présence d'un ou plusieurs marqueurs auxiliaires qui sont indicatifs de ou corrélés à la présence d'un variant du gène PLCζ humain ou d'une séquence d'acides
aminés codée par celui-ci,
ledit variant du gène PLCζ humain ou d'une séquence d'acides aminés codée par celui-ci présentant au moins une variation par comparaison avec la séquence PLCζ de type sauvage.

23. - Procédé selon l'une quelconque des revendications 20 à 22, dans lequel l'échantillon de test comprend de l'ADN génomique.

24. - Anticorps dirigé contre un polypeptide tel que défini à l'une quelconque des revendications 3 à 8 et 10.

25. - Anticorps selon la revendication 24, qui est un anticorps monoclonal dirigé contre un polypeptide tel que défini à l'une quelconque des revendications 3 à 8 et 10.

26. - Coffret de diagnostic ou de criblage pour déterminer l'état de fertilité d'un individu, lequel coffret comprend :
(a) un oligonucléotide ayant une séquence d'acides nucléiques correspondant à une région d'un variant de PLC-zeta(PLCζ), laquelle région comprend au moins une variation par rapport à la séquence génique PLC-zeta(PLCζ) de type sauvage ; et/ou
(b) un oligonucléotide ayant une séquence d'acides nucléiques correspondant à la séquence génique PLCζ de type sauvage dans la région spécifiée dans (a) ; et/ou
(c) un oligonucléotide ayant une séquence d'acides nucléiques correspondant à une région spécifique de la séquence génique PLCζ de type sauvage, laquelle région spécifique comprend une séquence non autrement présente dans l'ADN génomique du mammifère ; et/ou
(d) des anticorps, tels que des anticorps monoclonaux, dirigés contre tout oligonucléotide spécifique à l'un quelconque de (a) à (c) ci-dessus ; et, facultativement,
(e) un ou plusieurs réactifs appropriés pour réaliser une PCR pour amplifier des régions désirées de l'ADN de l'individu.

27. - Coffret selon la revendication 26, dans lequel l'un quelconque des composants du coffret (a) à (c) comprend une pluralité desdits oligonucléotides immobilisés sur un support solide.
